# EUROPEAN PATENT SPECIFICATION

(11) **EP 2 074 083 B1**
(45) Date of publication and mention of the grant of the patent: **19.06.2013**
(21) Application number: 07824996.8
(22) Date of filing: 27.10.2007
(51) Int. Cl.: C07C 311/21, A61K 31/18, A61P 29/00, C07D 207/26, C07D 211/18, C07D 211/56, C07D 211/62, C07D 211/76, C07D 213/38, C07D 213/72, C07D 233/54, C07D 239/04, C07D 239/42, C07D 241/20, C07D 243/08, C07D 295/12, C07D 295/20, C07D 401/04, C07D 401/06, C07D 401/12

(54) **NEW SULFONAMIDE DERIVATIVES AS BRADYKININ ANTAGONISTS**
NEUE SULFONAMIDDERIVATE ALS BRADYKININANTAGONISTEN
NOUVEAUX DÉRIVÉS SULFONAMIDES UTILISÉS COMME ANTAGONISTES DE LA BRADYKININE

(30) Priority: 27.10.2006 HU 0600810
(43) Date of publication of application: 01.07.2009
(73) Proprietor: Richter Gedeon Nyrt., 1103 Budapest (HU)
(72) Inventor: BOZÓ, Éva, 1102 Budapest (HU); BEKE, Gyula, 2092 Budakeszi (HU); ÉLES, János, 1121 Budapest (HU); FARKAS, Sándor, 1103 Budapest (HU); HORNOK, Katalin, 1141 Budapest (HU); KESERÜ, György, 2089 Telki (HU); SCHMIDT, Éva, 1021 Budapest (HU); SZENTIRMAY, Éva, 2030 Érd (HU); VÁGÓ, István, H-1121 Budapest (HU); VASTAG, Mónika, 1025 Budapest (HU)
(74) Representative: HOFFMANN EITLE
(86) International application number: PCT/HU2007/000104
(87) International publication number: WO 2008/050168

(56) References cited:
- WO-A-2005/004810
- WO-A-2007/072092
- GB-A- 1 279 843
- DATABASE CHEMCATS CHEMICAL ABSTRACTS SERVICE, COLUMBUS, OHIO, US; XP002473691 Database accession no. 2021940186 & "Enamine Screening Library" 17 January 2008 (2008-01-17), ENAMINE , KIEV, UKRAINE
- DATABASE REGISTRY [Online] CHEMICAL ABSTRACTS SERVICE, COLUMBUS, OHIO, US; 22 June 2005 (2005-06-22), XP002473692 retrieved from STN Database accession no. 852687-99-1
- R. PERRONE, ET AL.: "N-2-4-(4-Chlorophenyl)piperazin-1-ylethyl -3-methoxybenzamide: a potent and selective dopamine D4 ligand" JOURNAL OF MEDICINAL CHEMISTRY, vol. 41, no. 24, 4 November 1998 (1998-11-04), pages 4903-4909, XP000907280 AMERICAN CHEMICAL SOCIETY, WASHINGTON, DC, US ISSN: 0022-2623

## Description

### FIELD OF THE INVENTION

The present invention relates to new sulfonamide derivatives of formula (I) and optical antipodes or racemates and/or salts and/or hydrates and/or solvates thereof which are useful in the treatment or prevention of painful and inflammatory processes. The present invention also relates to the processes for producing compounds of formula (I) and to pharmacological compositions containing the same.

### BACKGROUND OF THE INVENTION

Kinins are endogenous peptides formed in plasma and peripheral tissues in response to tissue injury or infection following catalytic cleavage of kininogens by kallikrein enzymes. Kinins play an important role in the pathophysiological processes accompanying pain and inflammation. Their biological actions are mediated by two G-protein coupled membrane receptors, denoted B1 and B2. Broth B1 and B2 receptors have been cloned [Biochem. Biophys. Res. Commun., 184 (1992) 260-268 and J.Biol.Chem., 269 (1994) 21583-21586] and the mechanisms regulating their expression, self-maintenance and signalling function is under intensive investigations [Pharmacol.Rev., 57 (2005) 27-77].

The first set of kinins, bradykinin (BK) and kallidin (LysBK) preferentially act through stimulation of constitutively expressed and rapidly desensitising B2 receptors, which are widely distributed in many tissues. On the other hand, their active carboxypeptidase metabolites, the second set of kinins, desArg⁹BK (DABK) and LysdesArg⁹BK (LysDABK) activate inducible and non-desensitising B1 receptors, which are rarely expressed under non-pathological conditions. Generally B1 receptors rapidly appear after injuries of various natures (tissue trauma, infections, *etc*.). Thus the B1 receptor up-regulation appears to be part of a generalized response that includes the local co-expression (eventually up-regulation) of enzymes, receptors, autacoids, cytokines and chemokines that notoriously play key roles in the early and late responses of tissues to various types of injury.

In animal models it has been demonstrated that there is a switch in dominance of function from B2 to B1 in chronic inflammatory states. While the B2 receptor is implicated in the acute phase of the inflammatory and pain response, the B1 receptor is involved in the chronic phase of this response. The involvement of kinin receptors in inflammation and pain transduction has been supported by the results of studies on mice lacking bradykinin B1 receptors. B1 receptor deficient mice are different from wild-type mice in sensory functions, exhibiting increased analgesic thresholds to noxious chemical and heat stimuli, and drastic reduction in the accumulation of polymorphonuclear leukocytes at sites of inflammation [PNAS, 97 (2000) 8140-8145 and Neuropharmacology 41 (2001) 1006-1012]. Furthermore the most original finding in B1 receptor deficient mice was the direct evidence for a role of central kinin receptors in nociception suggesting that the hypoalgesia seen in B1-receptor knockout mice is partly due to reduced central sensitisation in the spinal cord. However, apart from the above changes B1 knockout mice were apparently normal without any apparent pathological changes.

Apart from the evidence of basal expression of B1 receptors on the periphery recently more and more evidence shows that B1 receptors are constitutively expressed 'centrally' in some neuronal elements, including the spinal cord and some higher structures as well. The function of these receptors is unclear but they have been implicated in pain transmission and hyperalgesia. Therefore, B1 receptor antagonists are believed to be useful in alleviating pain not only via peripheral sites but also to have possibly broader spectrum of analgesic effects if they block central B1 receptors as well [NeuroReport 11 (2000) 4003-4005; NeuroReport, 12 (2001) 2311-2313; Neuroscience 107 (2001) 665-673 and Neuroscience Letters 294 (2000) 175-178].

On the basis of scientific data bradykinin receptors are involved in mediation of pain and hyperalgesia in several ways. B1 receptor antagonists may have diverse modes of action. They have (1) indirect ('peripheral') effects on the nociceptors via inhibition of release of other algogenic mediators. N.B. B1 receptors appear upon inflammatory induction on cells adjacent to sensory neurones (macrophages, fibroblasts or endothelial cells) are involved in releasing mediators (prostaglandins, cytokines and nitric oxide) that sensitize or activate the nociceptors. (2) direct ('peripheral') effects on nociceptors expressing B1 receptors (constitutively) or upon induction and (3) 'central' effects on pain processing in the superficial dorsal horn of spinal cord.

Therefore, an orally active non-peptide bradykinin B1 receptor antagonist could be a potential therapeutic agent in the treatment of chronic inflammatory pain.

Several patents and patent applications describe bradykinin B1 receptor antagonists which have different chemical structures. Such documents are for instance the following international patent applications: WO200075107, WO02076964, WO04054584, WO02099388, WO05004810.
3-[([1,1'-biphenyl]-2-ylamino sulphonyl] -N-[2-(2-pyridinyl)-ethyl]benzamide (CAS Registry No. 852687-99-1) is comprised in the Enamine Screening Library (www.enamine.net).

### SUMMARY OF THE INVENTION

We have found a class of benzamide derivatives which have high affinity for bradykinin B1 receptors and selectivity over bradykinin B2 receptors. The selectivity is particularly important as the undesired side effects of the compounds are much less pronounced.

The present invention relates to new sulfonamide derivatives of formula (I) for use as a medicament 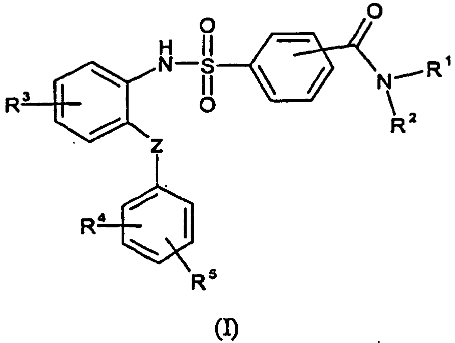 wherein
- R¹: is hydrogen atom or C₁-C₄ alkyl group;
- R²: is selected from (1) hydrogen atom; with the proviso that R¹ and R² can not be simultaneously hydrogen atom; (2) -(CH₂)ₙ-NR^{a}R^{b}, (3) -(CH₂)ₘ-X-Q, or
- R¹ and R²: together with the nitrogen atom to which they are attached form a 4-7 membered heterocyclic ring containing 0-3 heteroatom (in addition to the nitrogen atom to which R¹ and R² attached) selected from O, S and N; wherein said ring is optionally substituted with C₁-C₄ alkyl, -(CH₂)_{q}-Y-P, oxo, 4-(4,5-dihydro-1*H* imidazol-2-yl)-benzyl, 4-(1,4,5,6-tetrahydro-pyrimidin-2-yl)-benzyl or 4-ylmethyl-benzamidine group;
- R³, R⁴ and R⁵: are independently of each other hydrogen atom, halogen atom, cyano, amino, or amino substituted with one or more C₁-C₄ alkyl group, C₁-C₄ alkoxy, trifluoromethyl, trifluoromethoxy, C₁-C₄ alkyl, hydroxy, C₁-C₄ alkoxycarbonyl or -C(=O)-NH₂ group;
- Z: is selected from (1) single bond; (2) oxygen atom; (3) CH₂ group; (4) CO group; (5) NR^{c} group; (6) S atom; (7) SO₂ group ;
- n: is an integer from 1 to 4;
- m: is 0 or an integer from 2 to 6;
- q: is an integer from 0 to 4;
- R^{a} and R^{b}: are (1) hydrogen atom, (2) straight or branched C₁-C₆ alkyl group;
- R^{c}: is hydrogen atom or C₁-C₄ alkyl group;
- X: is a single bond, -CO- or -CO-NH- or NH-CO- group;
- Y: is a single bond, -CO- or -CONR^{a} group;
- P: is (1) -N(C1-C₄ alkyl)₂ group; (2) -NH-(CH₂)ₙ-Het group; (3) a saturated, partially unsaturated or aromatic 4-7 membered ring containing 1-3 heteroatom selected from O, S and N; wherein said ring is optionally substituted with oxo or C₁-C₄ alkyl group;
- Q: is (1) a saturated, partially unsaturated or aromatic 4-7 membered ring containing 1-3 heteroatom selected from O, S and N; wherein said ring is optionally substituted with oxo, -(CH₂)ₙ-Het, 1-piperidinyl, 1-(C₁-C₄-alkyl)-4-piperidinyl, 4-piperidinyl, 2-pyrimidinyl, 2-pyrazinyl, 2-pyridyl, 4-methyl-2-pyridyl, 6-methyl-2-pyridyl or 4-pyridyl group; (2) phenyl group, optionally substituted with -(CH₂)ₙ-Het, -(CH₂)ₙ-NH-C(=NH)-NH₂, 4,5-dihydro-1*H*-imidazol-2-yl or [1,4']bipiperidinyl-1'-yl group; (3) C₅-C₇ cycloalkyl group, optionally substituted with -(CH₂)ₙ-Het group; (4) benzyl group, optionally substituted with -(CH₂)ₙ-Het, -(CH₂)n-NH-C(=NH)-NH₂ or 4,5-dihydro-1*H*-imidazol-2-yl group; (5) -(CH₂)ₙ-Het group;
- Het: is a 4-7 membered heterocyclic ring containing 1-3 heteroatom selected from O, S, SO₂ and N, optionally substituted with (1) oxo; (2) one or more C₁-C₄ alkyl group;
and optical antipodes or racemates and/or salts and/or hydrates and/or solvates thereof.

The invention also relates to the pharmaceutical compositions containing the compounds of formula (I) or optical antipodes or racemates or salts or hydrates or solvates thereof as active ingredient.

In another aspect, the present invention relates to the compounds of formula (I) as defined above, wherein 3-[([1,1'-biphenyl]-2-ylamino sulphonyl]-N-[2-(2-pyridinyl)-ethyl]benzamide is excluded.

Furthermore objects of the present invention are the synthesis of compounds of formula (I), and the chemical and pharmaceutical manufacture of medicaments containing these compounds, as well as the compounds for use in methods of treatment, which means administering to a mammal to be treated - including human - effective amount/amounts of compounds, of formula (I) of the present invention as such or as medicament.

### DETAILED DESCRIPTION OF THE INVENTION

The present invention relates to new bradykinin B1 receptor antagonist sulfonamide derivatives of formula (I) 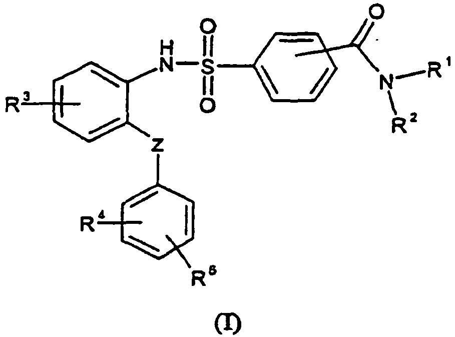 wherein
- R¹: is hydrogen atom or C₁-C₄ alkyl group;
- R²: is selected from (1) hydrogen atom; with the proviso that R¹ and R² can not be simultaneously hydrogen atom; (2) -(CH₂)ₙ-NR^{a}R^{b}, (3) -(CH₂)ₘ X-Q, or
- R¹ and R²: together with the nitrogen atom to which they are attached form a 4-7 membered heterocyclic ring containing 0-3 heteroatom (in addition to the nitrogen atom to which R¹ and R² attached) selected from O, S and N; wherein said ring is optionally substituted with C₁-C₄ alkyl, -(CH₂)_{q}-Y-P, oxo, 4-(4,5-dihydro-1*H*-imidazol-2-yl)-benzyl, 4-(1,4,5,6-tetrahydro-pyrimidin-2-yl)-benzyl or 4-ylmethyl-benzamidine group;
- R³, R⁴ and R⁵: are independently of each other hydrogen atom, halogen atom, cyano, amino, or amino substituted with one or more C₁-C₄ alkyl group, C₁-C₄ alkoxy, trifluoromethyl, trifluoromethoxy, C₁-C₄ alkyl, hydroxy, C₁-C₄ alkoxycarbonyl or -C(=O)-NH₂ group;
- Z: is selected from (1) single bond; (2) oxygen atom; (3) CH₂ group; (4) CO group; (5) NR^{c} group; (6) S atom; (7) SO₂ group ;
- n: is an integer from 1 to 4;
- m: is 0 or an integer from 2 to 6;
- q: is an integer from 0 to 4;
- R^{a} and R^{b}: are (1) hydrogen atom, (2) straight or branched C₁-C₆ alkyl group;
- R^{c}: is hydrogen atom or C₁-C₄ alkyl group;
- X: is a single bond, -CO- or -CO-NH- or NH-CO- group;
- Y: is a single bond, -CO- or -CONR^{a} group;
- P: is (1) -N(C₁-C₄ alkyl)₂ group; (2) -NH-(CH₂)ₙ-Het group; (3) a saturated, partially unsaturated or aromatic 4-7 membered ring containing 1-3 heteroatom selected from O, S and N; wherein said ring is optionally substituted with oxo or C₁-C₄ alkyl group;
- Q: is (1) a saturated, partially unsaturated or aromatic 4-7 membered ring containing 1-3 heteroatom selected from O, S and N; wherein said ring is optionally substituted with oxo, -(CH₂)ₙ-Het, 1-piperidinyl, 1-(C₁-C₄-alkyl)-4-piperidinyl, 4-piperidinyl, 2-pyrimidinyl, 2-pyrazinyl, 2-pyridyl, 4-methyl-2-pyridyl, 6-methyl-2-pyridyl or 4-pyridyl group; (2) phenyl group, optionally substituted with -(CH₂)ₙ-Het, -(CH₂)ₙ-NH-C(=NH)-NH₂, 4,5-dihydro-1*H*-imidazol-2-yl or [1,4']bipiperidinyl-1'-yl group; (3) C₅-C₇ cycloalkyl group, optionally substituted with -(CH₂)ₙ-Het group; (4) benzyl group, optionally substituted with -(CH₂)ₙ-Het, -(CH₂)ₙ-NH-C(=NH)-NH₂ or 4,5-dihydro-1*H*-imidazol-2-yl group; (5) -(CH₂)ₙ-Het group;
- Het: is a 4-7 membered heterocyclic ring containing 1-3 heteroatom selected from O, S, SO₂ and N, optionally substituted with (1) oxo; (2) one or more C₁-C₄ alkyl group;
and optical antipodes or racemates and/or salts and/or hydrates and/or solvates thereof.

The invention also relates to the pharmaceutical compositions containing the compounds of formula (I) or optical antipodes or racemates or salts or hydrates or solvates thereof as active ingredient.

Furthermore objects of the present invention are the synthesis of compounds of formula (I), and the chemical and pharmaceutical manufacture of medicaments containing these compounds, as well as the compounds for use in methods of treatment, which means administering to a mammal to be treated - including human - effective amount/amounts of compounds of formula (I) of the present invention as such or as medicament.

The term "halogen" substituent denotes fluorine, chlorine, bromine or iodine atoms. The term C₁-C₄ alkyl group used in the present description denotes methyl, ethyl, normal- and isopropyl and different butyl groups. These C₁-C₄ alkyl groups can be in the C₁-C₄ alkoxy groups and C₁-C₄ alkoxycarbonyl groups.

The 4-7 membered heterocyclic ring in the meaning of R¹ and R² can be e.g. piperidine, pyrrolidine, piperazine, homopiperazine, morpholine, thiomorpholine and the like.

The Het group can be *e.g*. piperidine, pyrrolidine, piperazine, homopiperazine, morpholine, thiomorpholine, pyridine, pyrimidine, pyrazine and the like.

The saturated, partially unsaturated or aromatic 4-7 membered ring in the meaning of P and Q can be *e.g*. piperidine, pyrrolidine, piperazine, homopiperazine, morpholine, thiomorpholine, imidazole, pyridine and the like.

The invention relates also to the salts of compounds of formula (I) formed with acids or bases.

Both organic and inorganic acids can be used for the formation of acid addition salts. Suitable inorganic acids can be *e.g*. hydrochloric acid, sulfuric acid and phosphoric acid. Representatives of monovalent organic acids can be *e.g*. formic acid, acetic acid, trifluoroacetic acid, propionic acid, and different butyric acids, valeric acids and capric acids. Representatives of bivalent organic acids can be *e.g*. oxalic acid, malonic acid, maleic acid, fumaric acid and succinic acid. Other organic acids can also be used, such as hydroxy acids *e.g*. citric acid, tartaric acid, or aromatic carboxylic acids *e.g*. benzoic acid or salicylic acid, as well as aliphatic and aromatic sulfonic acids *e.g*. methanesulfonic acid and p-toluenesulfonic acid. Especially valuable group of the acid addition salts is in which the acid component itself does not have therapeutical effect in the applied dose or it does not have unfavorable influence on the effect of the active ingredient. These acid addition salts are pharmaceutically acceptable acid addition salts. The reason why acid addition salts, which do not belong to the pharmaceutically acceptable acid addition salts belong to the present invention is, that in given case they can be advantageous in the purification and isolation of the desired compounds.

Among the salts formed with bases especially important are the salts formed with alkali metals, *e.g*. sodium, potassium, alkaline-earth metals, *e.g*. calcium and magnesium, as well as with ammonia or organic amines. The latter bases can have further substituents, *e.g*. hydroxy or amino groups, which can influence *e.g*. the solubility and the handling of the product. The salts formed with bases are pharmaceutically acceptable base addition salts.

According to the invention the compounds of formula (I) can be synthesized by reacting an amine derivative of formula (II) 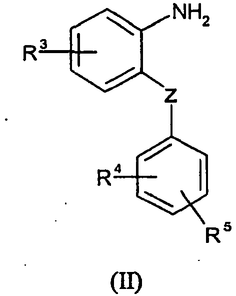 - wherein the meaning of R³, R⁴ and R⁵ are as described above for the formula of (I) - with sulfonyl chloride of formula (III) 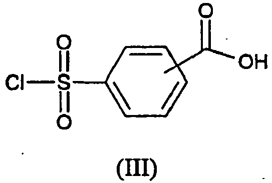 then the so obtained phenylsulfamoyl benzoic acid derivative of formula (IV) 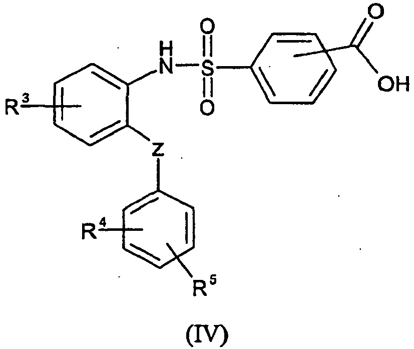 - wherein the meaning of R³, R⁴ and R⁵ are as defined above - is reacted with an amine derivative of formula (V) 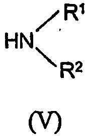 - wherein the meaning of R¹ and R² are as defined above - and the obtained phenylsulfamoyl benzamide derivative of formula (I) in given case can be transformed into an other compound of formula (I) by introducing new substituents and/or modifying or removing the existing ones, and/or salt formation and/or liberating the compound from salts.

The sulfonylation reaction is preferably carried out in a proper solvent, preferably in the presence of a base. The reactions are followed by thin layer chromatography. The necessary reaction time is 6-20 h. The work-up of the reaction mixture can be carried out by different methods.
a) The reaction mixture is concentrated and the product is isolated by crystallization or extraction. If the crude product is not pure enough, then column chromatography can be used for the purification of it. The column chromatography is carried out either on normal phase using Kieselgel 60 as adsorbent and different solvent systems, *e.g*. n-hexane/ethyl acetate, chloroform/methanol, dichloromethane/ethyl acetate or chloroform/acetone as eluents, or on reversed phase using YMC-Pack ODS-AQ type packings (produced by YMC) and acetonitrile/water/trifluoroacetic acid or acetonitrile/water/acetic acid as eluent.
b) The reaction mixture is poured into ice-water and the product is isolated by filtration or extraction. The crude product is crystallized or purified by column chromatography as described above. The structures of the products are determined by IR, NMR and mass spectrometry.

The amide bond formation is preferably carried out by preparing an active derivative from a carboxylic acid of formula (IV) which is reacted with an amine of formula (V) preferably in the presence of a base.

The transformation of a carboxylic acid into an active derivative can be carried out *in situ* during the amide bond formation in a proper solvent (*e.g*. dimethylformamide, acetonitrile, chlorinated hydrocarbons or hydrocarbons or the mixture thereof). The active derivatives can be acid chlorides (*e.g*. prepared from carboxylic acid with thionyl chloride), mixed anhydrides (*e.g*. prepared from carboxylic acid with isobutyl chloroformate in the presence of a base, *e.g*. triethylamine), active esters (*e.g*. prepared from carboxylic acid with hydroxybenztriazol (HOBt) and dicyclohexyl-carbodiimide (DCC) or O-benzotriazol-1-yl-N,N,N',N'-tetramethyluronium hexafluorophosphate (HBTU) in the presence of a base e.g. triethylamine). The active derivatives can be prepared at a temperature in the range of 0 °C to room temperature. A proper amine of formula (V) is added as a base or as a salt formed with inorganic acid to the so obtained solution or suspension in the presence of a base, *e.g*. triethylamine, needed for the liberation of the amine. The condensation reactions are followed by thin layer chromatography. The necessary reaction time is 6-20 h. The work-up of the reaction mixture can be carried out by different methods.

When the reaction mixture is a suspension, the precipitate is filtered off, washed with water and/or with an organic solvent to give the pure product. If the reaction mixture is a solution at the end of the amide bond formation reaction:
a) The reaction mixture is concentrated, and the residue is crystallized or extracted with a proper organic solvent and in given case purified by column chromatography. The column chromatography is carried out on normal phase using Kieselgel 60 as adsorbent and different solvent systems, *e.g*. toluene/methanol, chloroform/methanol or toluene/acetone, as eluents or on reversed phase using YMC-Pack ODS-AQ type packings (produced by YMC) and acetonitrile/water/trifluoroacetic acid or acetonitrile/water/acetic acid as eluent.
b) The reaction mixture is directly purified by column chromatography as described above to yield the pure product.

The structures of the products are determined by IR, NMR and mass spectrometry.

The obtained benzamide derivatives of formula (I) - independently from the method of preparation - in given case can be transformed into another compound of formula (I) by introducing further substituents and/or modifying and/or removing the existing ones, and/or formation of salts with acids and/or liberating the benzamide derivative of formula (I) from the obtained acid addition salts by treatment with a base and/or the free sulfonamide derivative of formula (I) can be transformed into a salt by treatment with a base.

For instance the compounds of formula (I) containing free hydroxy group can be transformed into acyloxy or sulfoxy derivatives with different acylating or sulfonylating agents. The reactions can be carried out for example in chlorinated hydrocarbons using acid chloride or acid anhydride as acylating agent in the presence of a base (*e.g*. triethylamine or sodium carbonate). The sulfonamide derivatives of formula (I) containing a nitro group can be transformed into amines by reduction and the amines can be further reacted to give acid amides as described for the acylation of hydroxy groups or carbamate derivatives can be synthesized. Ester groups can be hydrolyzed and the obtained free carboxylic acids can be transformed into amides by reacting with proper amine derivatives. *N*-(*tert*-Butoxycarbonyl) group can be cleaved by organic or inorganic acids (*e.g*. trifluoroacetic acid or hydrogen chloride). Cyano groups can be transformed into amide, *N*-hydroxy-amidine or different N-containing heterocyclic groups.

Most of the amines of formula (V) and the sulfonyl chloride of formula (III) are either commercially available or can be synthesized by different known methods. The syntheses of some new amines of formula (V) are described in the Examples. Following these procedures the other amines of formula (V) can also be prepared.

The compounds of the present invention and as well as their pharmaceutically acceptable salts or hydrates or solvates can be used as such or suitably in the form of pharmaceutical compositions. These compositions (drugs) can be in solid, liquid or semiliquid form and pharmaceutical adjuvant and auxiliary materials can be added, which are commonly used in practice, such as carriers, excipients, diluents, stabilizers, wetting or emulsifying agents, pH- and osmotic pressure-influencing, flavoring or aromatizing, as well as formulation-promoting or formulation-providing additives.

The dosage required to exert the therapeutical effect can vary within wide limits and will be fitted to the individual requirements in each of the particular case, depending on the stage of the disease, the condition and the bodyweight of the patient to be treated, as well as the sensitivity of the patient against the active ingredient, route of administration and number of daily treatments. The actual dose of the active ingredient to be used can safely be determined by the attending physician skilled in the art in the knowledge of the patient to be treated.

The pharmaceutical compositions containing the active ingredient according to the present invention usually contain 0.01 to 100 mg of active ingredient in a single dosage unit. It is, of course possible that the amount of the active ingredient in some compositions exceeds the upper or lower limits defined above.

The solid forms of the pharmaceutical compositions can be *e.g*. tablets, dragées, capsules, pills or lyophilized powder ampoules useful for the preparation of injections. Liquid compositions are the injectable and infusable compositions, fluid medicines, packing fluids and drops. Semiliquid compositions can be ointments, balsams; creams, shaking mixtures and suppositories.

For the sake of a simple administration it is suitable if the pharmaceutical compositions comprise dosage units containing the amount of the active ingredient to be administered once, or a few multiples or a half, third or fourth part thereof. Such dosage units are *e.g*. tablets, which can be powdered with grooves promoting the halving or quartering of the tablet in order to exactly administer the required amount of the active ingredient.

Tablets can be coated with an acid-soluble layer in order to assure the release of then active ingredient content after leaving the stomach. Such tablets are enteric-coated. A similar effect can be achieved also by encapsulating the active ingredient.

The pharmaceutical compositions for oral administration can contain *e.g*. lactose or starch as excipients, sodium carboxymethylcellulose, methylcellulose, polyvinyl pyrrolidine or starch paste as binders or granulating agents. Potato starch or microcrystalline cellulose is added as disintegration agents, but ultraamylopectin or formaldehyde casein can also be used. Talcum, colloidic silicic acid, stearin, calcium or magnesium stearate can be used as antiadhesive and lubricants.

The tablets can be manufactured *e.g*. by wet granulation, followed by pressing. The mixed active ingredients and excipients, as well as in given case part of the disintegrants are granulated with an aqueous, alcoholic or aqueous alcoholic solution of the binders in an appropriate equipment, then the granulate is dried. The other disintegrants, lubricants and antiadhesive agents are added to the dried granulate, and the mixture is pressed to a tablet. In given case the tablets are made with halving groove to ease the administration.

The tablets can be made directly from the mixture of the active ingredient and the proper auxiliaries by pressing. In given case, the tablets can be coated by using additives commonly used in the pharmaceutical practice, *e.g*. stabilizers, flavoring, coloring agents, such as sugar, cellulose derivatives (methyl- or ethylcellulose, sodium carboxymethylcellulose, etc), polyvinyl pyrrolidone, calcium phosphate, calcium carbonate, food coloring agents, food laces, aroma agents, iron oxide pigments, etc. In the case of capsules the mixture of the active ingredient and the auxiliaries is filled into capsules.

Liquid oral compositions, *e.g*. suspensions, syrups, elixirs can be made by using water, glycols, oils, alcohols, coloring and flavoring agents.

For rectal administration the composition is formulated in suppositories or clysters. The suppository can contain beside the active ingredient a carrier, so called adeps pro suppository. Carriers can be vegetable oils, such as hydrogenated vegetable oils, triglycerides of C₁₂-C₁₈ fatty acids (preferably the carriers under the trade name Witepsol). The active ingredient is homogeneously mixed with the melted adeps pro suppository and the suppositories are moulded.

For parenteral administration the composition is formulated as injection solution. For manufacturing the injection solution the active ingredients are dissolved in distilled water and/or in different organic solvents, such as glycolethers, in given case in the presence of solubilizers, *e.g*. polioxyethylensorbitane-monolaurate, -monooleate, or monostearate (Tween 20, Tween 60, Tween 80). The injection solution can also contain different auxiliaries, such as conserving agents, *e.g*. ethylendiamine tetraacetate, as well as pH adjusting agents and buffers and in given case local anaesthetic, *e.g*. lidocain. The injection solution containing the active ingredient of the invention is filtered before it is filled into ampoules, and it is sterilized after filling.

If the active ingredient is hygroscopic, then it can be stabilized by liophylization.

### Utilities

The compounds of the present invention are bradykinin receptor antagonists, in particular selective bradykinin B1 receptor antagonists, consequently are useful in the treatment or prevention of painful and inflammatory processes. The compounds would be effective in the treatment of pain including, *e.g*., chronic pain, particularly inflammatory pain, hyperalgesia, bone and joint pain (osteoarthritis), repetitive motion pain, myofascial pain (muscular injury, fibromyalgia), visceral pain (ulcerative colitis, pancreatitis, cystitis, uveitis), perioperative pain (general surgery, gynecological), postoperative pain (postsurgical pain syndrome), posttraumatic pain (*e.g*. sprains or fracture), neuropathic pain (postherpetic neuralgia, nerve injury, phantom limb pain, mononeuropthy, polyneuropathy) dental pain, and cancer pain. Furthermore for the treatment of pain associated with angina, menstruation, diabetic vasculopathy, post capillary resistance or diabetic symptoms associated with insulitis (*e.g*. hyperglycemia, diuresis, proteinurea and increased nitrite and kallikrein urinary excretion), diabethic hyperalgeisa. Moreover the compounds may be used for the treatment angioedema, atherosclerosis, septic shock *e.g*. as anti-hypovolemic and/or anti-hypotensive agents, and sepsis. They may be used as smooth muscle relaxants for the treatment of spasm of the gastrointestinal tract or uterus. Further, the compounds of this invention can additionally be used to treat inflammatory skin disorders, such as psoriasis and eczema, and skin injuries including burning and sunburning (UV-erythema and pain). The compounds may be used to treat inflammatory pain of varied origins (*e.g*. rheumatoid arthritis, rheumatic disease, tenosynovitis, liver disease, irritable bowel syndrome, inflammatory bowel disease, Crohn's disease, nephritis, allergic rhinitis, vasomotor rhinitis, uveitis, gingivitis), allergies. Such compounds may be used therapeutically to treat inflammatory airways disease *e.g.* chronic obstructive pulmonary disease, adult respiratory distress syndrome, bronchitis, pneumonia, asthma. They may be used to control, restrict or reverse airways hyperreactivity in asthma, to treat intrinsic and extrinsic asthma including allergic asthma (atopic or non-atopic), occupational asthma, viral or bacterial exacerbated asthma, other non-allergic asthmas, "wheezy-infant syndrome", as well as exercise-induced bronchoconstriction. They may be effective against pneumoconiosis, including aluminosis, antracosis, asbestosis, chalicosis, ptilosis, siderosis, silicosis, tabacosis and byssinosis. Additionally, they may be effective in some neurological disorders, *e.g*. against multiple sclerosis, Alzheimer's disease, epilepsy, cerebral edema, headache including cluster headache, migraine including prophylactic and acute use, as well as closed head trauma.

### Biological evaluation

### Functional assay:

### Assessment of antagonist potency at B1 and B2 receptors in vitro by measurement of cytosolic calcium ion concentration with a plate reader fluorimeter in cells expressing recombinant human B1 or B2 receptors

### Cell culture

Chinese hamster ovary (CHO) cells stably expressing recombinant human B1 (CHO-B1, Euroscreen) or B2 (CHO-B2, Perkin-Elmer) receptors were cultured in Dulbecco's Modified Eagle's Medium (DMEM) containing 10% Fetal Calf Serum (FCS), 100 U/ml penicillin, 0.1 mg/ml streptomycin, 0.25 µg/ml amphotericin B, 1% Minimum Essential Medium Eagle (MEM), non essential amino acid solution, 600 µg/ml G418, 1% pyruvate (for the B2 cell line). Cells were kept at 37°C in a humidified incubator in an atmosphere of 5% CO₂/95% air and were passaged 1:4 three times a week. Cells were plated at 1.5-2.5 ×10⁴ cell/well on standard 96-well microplates, measurements of cytosolic calcium ion concentration ([Ca²⁺]ᵢ) were carried out 1-2 days after cell plating.

### Fluorimetric measurement of cytosolic calcium concentration

Measurements of [Ca²⁺]_{¡} were carried out on CHO-B1 and CHO-B2 cells stably expressing human B1 and B2 receptors, respectively. Cells were grown in standard 96-well microplates and before the measurement were loaded with a fluorescent Ca²⁺-sensitive dye, fluo-4/AM (2 µM): after removing the culture medium the dye was added to the cells (dissolved in assay buffer: 145 mM NaCl, 5 mM KCl, 2 mM MgCl₂, 2 mM CaCl₂, 10 mM HEPES, 20 mM D-glucose, 2 mM probenecid, 100 µl/well) and cells were incubated at 37°C in a humidified incubator in an atmosphere of 5% CO₂/95% air for 40-120 min. To stop dye loading cells were washed twice with assay buffer. After washing, various concentrations of the test compounds (diluted in extracellular medium from a DMSO stock solution, final DMSO concentration was <0.1%) or buffer were added to each well depending on the experimental setup. After incubation at 37°C for 20-25 min. baseline and agonist-evoked changes of [Ca²⁺]ᵢ were measured column by column with a plate reader fluorimeter (Fluoroskan Ascent, Labsystems). Excitation and detection of emission was carried out from the bottom of the plate. Filters used for Fluo-4: excitation filter - 485 nm, emission filter - 538 nm. The whole measurement process was performed at 37°C and was controlled by custom software. Inhibitory potency of the test compounds was assessed by measuring the reduction in the agonist-evoked [Ca²⁺]ᵢ-elevation in the presence of different concentrations of the compounds. The agonists were LysDABK for CHO-B1, and bradykinin for CHO-B2 cells. Agonists were applied at an EC₈₀ concentration, the EC₈₀-values were derived from daily determined dose-response curves. Fluorescence data were expressed as ΔF/F (fluorescence change normalized to baseline). All treatments on a single plate were measured in multiple wells. Data from all wells with the same treatment were averaged and the average values were used for analysis. Inhibitory potency of a compound at a single concentration point was expressed as percent inhibition of the control agonist response. Sigmoidal concentration-inhibition curves were fitted to the data (derived from at least three independent experiments) and IC₅₀-Values were determined as the concentration that produces half of the maximal inhibition caused by the compound.

The examined reference compounds measured in functional and binding tests are the following:
1) 4-{2-[(2,2-diphenyl-ethyl-ethyl)-amino]-5-{4-[4-[(4-methyl-1-piperazinyl)-carbonyl]-1-piperidinyl]-sulfonyl}-benzoyl}-morpholine (NVP-SAA164, Br. J. Pharmacol.144 (2005) 889-899); Kᵢ 8 nM; IC₅₀: 33 nM;
2) (R)-N-[2,3-dihydro-2-oxo-5-(2-phenyl-ethyl)-1-propyl-1H-1,4-benzodiazepin-3-yl]-N'-{4-[4-(4-pyridinyl)-1-piperazinyl]-phenyl}-urea (J. Med. Chem. 46 (2003) 1803-1806); Kᵢ 0.59 nM; IC₅₀ 1.9 nM;
3) N-[4-(1,4'-bipiperidin)-1'-ylphenyl]-N'-[(3R)-2,3-dihydro-5-(4-methyl-phenyl)-2-oxo-1-propyl-1H-1,4-benzodiazepin-3-yl]-urea (J. Med. Chem. 46 (2003) 1803-1806); Kᵢ 13.4 nM; IC₅₀ 64.5 nM

The Kᵢ and IC₅₀ data measured by us for the reference compounds are in good agreement with the data given in the literature.

In Table 1 the most effective compounds of this invention measured in functional assay are listed.

**Table 1**

| **Number of example** | **B1 func.** | **Number of example** | **B1 func.** |
|---|---|---|---|
| **1** | ++++ | **64** | +++ |
| **2** | ++++ | **66** | ++++ |
| **3** | ++++ | **76** | +++ |
| **4** | ++++ | **87** | ++ |
| **5** | ++++ | **99** | +++ |
| **6** | ++++ | **101** | +++ |
| **7** | ++++ | **102** | ++++ |
| **8** | +++ | **105** | ++++ |
| **9** | +++ | **111** | ++++ |
| **10** | +++ | **114** | ++++ |
| **11** | +++ | **116** | ++++ |
| **20** | +++ | **121** | +++ |
| **22** | ++++ | **127** | ++++ |
| **23** | ++ | **129** | +++ |
| **24** | ++++ | **132** | +++ |
| **28** | ++++ | **135** | +++ |
| **29** | +++ | **137** | +++ |
| **30** | +++ | **147** | +++ |
| **33** | +++ | **152** | +++ |
| **36** | +++ | **157** | ++++ |
| **37** | +++ | **159** | +++ |
| **54** | +++ | **166** | +++ |
| **56** | +++ | **167** | +++ |
| **58** | ++++ | **169** | ++++ |
| **62** | ++++ | **170** | ++++ |

| | | | |
|---|---|---|---|
| + IC₅₀ > 0.5 µM +++ IC₅₀ is between 20 and 100 nM ++ IC₅₀ is between 0.1 and 0.5 µM ++++ IC₅₀ < 20 nM | | | |

### Receptor binding assays

### 1. Human recombinant bradykinin B1 receptor binding

Binding assays were carried out on human recombinant bradykinin1 receptors (expressed in CHO cells) according to the Euroscreen Technical Data Sheet (Cat.No.:ES-091). 20µg protein/tube was incubated with [3,4-prolyl-3,4-³H(N)]-[Des-Arg¹⁰] Kallidin as radioligand. Non specific binding was determined in the presence of 10 µM Lys-des-Arg⁹-Bradykinin. The final incubation volume was 250 µl. Samples were incubated for 15 min. at 25 °C then were rapidly vacuum filtered through GF/B filters presoaked for at least 1 h in 0.5 % PEI. Radioactivity was determined by liquid scintillation spectroscopy.

In Table 2 the most effective compounds of this invention measured in binding assay are listed.

**Table 2**

| **Number of example** | **B1 binding** | **Number of example** | **B1 binding** |
|---|---|---|---|
| **1** | ++++ | **64** | +++ |
| **2** | ++++ | **66** | ++++ |
| **3** | ++++ | **76** | +++ |
| **4** | ++++ | **87** | ++ |
| **5** | ++++ | **99** | +++ |
| **6** | ++++ | **101** | +++ |
| **7** | ++++ | **102** | ++++ |
| **8** | ++++ | **105** | ++++ |
| **9** | ++++ | **111** | ++++ |
| **10** | ++++ | **114** | ++++ |
| **11** | +++ | **116** | ++++ |
| **20** | +++ | **121** | +++ |
| **22** | ++++ | **127** | +++ |
| **23** | +++ | **129** | +++ |
| **24** | ++++ | **132** | ++++ |
| **28** | ++++ | **135** | +++ |
| **29** | +++ | **137** | +++ |
| **30** | +++ | **147** | +++ |
| **33** | +++ | **152** | +++ |
| **36** | ++++ | **157** | ++++ |
| **37** | +++ | **159** | +++ |
| **54** | +++ | **166** | +++ |
| **56** | ++++ | **167** | +++ |
| **58** | ++++ | **169** | ++++ |
| **62** | ++++ | **170** | ++++ |

| | | | |
|---|---|---|---|
| + Kᵢ > 0.5 µM +++ Kᵢ is between 20 and 100 nM ++ Kᵢ is between 0.1 and 0.5 µM ++++ Kᵢ < 20 nM | | | |

### 2. Human recombinant bradykinin B2 receptor binding

Binding assays were carried out on human recombinant bradykinin2 receptors (expressed in CHO cells) according to the Receptor Biology Technical Data Sheet (Cat.No.:RBHB2M) with minor modifications. 8.4 µg protein/tube was incubated with [2,3,-prolyl-3,4-³H(N)]-Bradykinin as radioligand. Non specific binding was determined in the presence of 5 µM bradykinin. The final incubation volume was 200 µl. Samples were incubated for 90 min. at +4 °C then were rapidly vacuum filtered through GF/B filters presoaked for at least 1 h in 0.5 % PEI. Radioactivity was determined by liquid scintillation spectroscopy.

The compounds exhibited high affinity and selectivity (>50 fold) for the human B1 receptor over the human B2 receptor according to both functional and binding assays.

The synthesis of compounds and pharmaceutical compositions according to the invention is illustrated by the following not limiting Examples.

### Reference Example 1

### 2-(4-Pyridin-4-yl-piperazin-1-yl)-ethylamine

### a) 2-[2-(4-Pyridin-4-yl-piperazin-1-yl)-ethyl]-isoindole-1,3-dione

A mixture of 1-pyridin-4-yl-piperazine [Org. Lett. 4 (2002) 737-740] (1.0 g, 6.12 mmol), *N*-(2-bromoethyl)-phthalimide (1.71 g, 6.74 mmol), potassium carbonate (0.85 g, 6.12 mmol), potassium iodide (1.02 g, 6.12 mmol) and dimethylformamide (10 mL) was stirred at 70 °C for 24 h, then concentrated. The residue was dissolved in water, extracted with dichloromethane, the organic layer was dried over sodium sulfate, filtered and concentrated. The crude product was purified by column chromatography using Kieselgel 60 (0.040-0.063 mm) (Merck) as adsorbent, and chloroform:methanol: ammonium hydroxide = 10:1:0.1 as eluent to yield 1.52 g (74 %) of the title compound, as a white solid.

### b) 2-(4-Pyridin-4-yl-piridin-4-yl-piperazin-1-yl)-ethylamin

A stirred mixture of 2-[2-(4-pyridin-4-yl-piperazin-1-yl)-ethyl]-isoindole-1,3-dione (1.52 g, 4.52 mmol), ethanol (47.5 mL), water (2.5 mL) and hydrazine hydrate (98 %, 0.438 mL, 9.04 mmol) was refluxed for 3 h, then cooled and diluted with diethyl ether (100 mL). The precipitated crystals were filtered off, washed with diethyl ether and filtrate was concentrated. The residue was dissolved N sodium hydroxide (25 mL), extracted with dichloromethane (4x25 mL), the combined organic layers were washed with brine (25 mL), dried over sodium sulfate, filtered and concentrated to yield 0.58 g (62 %) of the title compound as a colorless oil.

### Reference Example 2

### 2-[4-(4,5-Dihydro-1H-imidazol-2-yl)-phenyl]-ethylamine dihydrochloride

### a) 4-[2-(1,3-Dioxo-1,3-dihydro-isoindol-2-yl)-ethyl]-benzonitrile

Under argon, a solution of 4-(2-hydroxy-ethyl)-benzonitrile [Helv. Chim. Acta 64 (1981) 1688-1703] (4.49 g, 30.5 mmol), phthalimide (4.94 g, 33.55 mmol), triphenylphosphine (8.8 g, 33.55 mmol) and dimethylformamide (100 mL) was stirred at 0 °C for 20 minutes, then diethyl azodicarboxylate (7.59 mL, 48.8 mmol) was added dropwise at 0 °C. The so obtained reaction mixture was stirred at room temperature overnight, then poured into ice-water (740 mL). The precipitated product was filtered off, washed with water and dried. The crude product was recrystallized from 2-propanol to yield 7.83 g (93 %) of the title compound as a yellow solid.

### b) 2-{2-[4-(4,5-Dihydro-1H-imidazol-2-yl)-phenyl]-ethyl}-isoindole-1,3-dione

Dry hydrogen chloride gas was bubbled through an ice cold solution of 4-[2-(1,3-dioxo-1,3-dihydro-isoindol-2-yl)-ethyl]-benzonitrile (7.83 g, 28.3 mmol) in ethanol (400 mL) for 3 h, then the so obtained mixture was kept at 8 °C overnight. The reaction mixture was concentrated *in vacuo*, the residue was dissolved in dry ethanol (400 mL), ethylenediamine (2.0 mL, 29.7 mmol) was added and the reaction mixture was stirred at room temperature overnight. The mixture was concentrated *in vacuo*, the residue was partitioned between dichloromethane (400 mL) and concentrated ammonium hydroxide (400 mL), the phases were separated and the water phase was extracted with dichloromethane (2x200 mL). The combined organic layers were dried over sodium sulfate, filtered and concentrated. The crude product was recrystallized from 2-propanol to yield 5.58 g (62 %) of the title compound as a white solid.

### c) 2-[4-(4,5-Dihydro-1H-imidazol-2-yl)-phenyl]-ethylamine dihydrochloride

A mixture of 2-{2-[4-(4,5-dihydro-1*H*-imidazol-2-yl)-phenyl]-ethyl}-isoindole-1,3-dione (5.58 g, 17.47 mmol), ethanol (140 mL) and hydrazine hydrate (98 %, 6.57 mL, 135.4 mmol) was stirred at room temperature for 2 h, then concentrated *in vacuo.* The residue was partitioned between dichloromethane (250 mL) and N sodium hydroxide (250 mL), the phases were separated and the water phase was extracted with dichloromethane (6x250 mL). The combined organic layers were dried over sodium sulfate, filtered and concentrated. The crude product was dissolved in methanol (15 mL), the pH of the solution was adjusted to 5 by addition of methanolic solution of hydrogen chloride, then the mixture was stirred at room temperature for 1 h. After addition of diethyl ether (200 mL) the suspension was stirred at 0 °C for 2 h, the precipitated crystals were filtered off, washed with diethyl ether and dried to yield 4.11 g (90 %) of the title compound as a white solid.

### Reference Example 3

### (3-[1,4']Bipiperidinyl-1'-yl)-propylamine trihydrochloride

### a) (3-[1,4']Bipiperidinyl-1'-yl-propyl-carbamic acid tert-butyl ester

A mixture of 4-piperidinopiperidine (Aldrich) (2.0 g, 11.88 mmol), (3-bromo-propyl)-carbamic acid *tert*-butyl ester [Eur. J. Med. Chem. Chim. Ther. 37 (2002) 573-584] (3.96 g, 16.63 mmol), dimethylformamide (130 mL) and potassium carbonate (1.64 g, 11.88 mmol) was stirred at room temperature overnight, then concentrated *in vacou.* The residue was dissolved in water (150 mL), extracted with dichloromethane (3x150 mL), the combined organic layers were washed with brine (150 mL), dried over sodium sulfate, filtered and concentrated. The crude product was submitted to column chromatography using Kieselgel 60 (0.040-0.063 mm) (Merck) as adsorbent, and chloroform:methanol:ammonium hydroxide = 10:1:0.1 as eluent to yield 2.27 g (59 %) of the title compound as an oil.

### b) 3-[1,4']Bipiperidinyl-1'-yl-propylamine trihydrochloride

A mixture of (3-[1,4']bipiperidinyl-1'-yl-propyl)-carbamic acid *tert*-butyl ester (2.15 g, 6.6 mmol), dry dioxane (40 mL) and 6.5 N hydrogen chloride in dioxane (22 mL) was stirred at room temperature overnight, then diluted with diethyl ether and stirred at 0 °C for 1 h. The precipitated crystals were filtered off, washed with diethyl ether and dried to yield 2.03 g (92 %) of the title compound as a beige solid.

### Reference Example 4

### Trans-4-(2-pyrrolidin-1-yl-ethyl)-cyclohexylamine dihydrochloride

### a) Trans-2-{1-[4-(N-tert-butoxycarbonyl)-amino]-cyclohexyl}-ethanol

A solution of trans-2-{1-[4-(*N*-*tert*-butoxycarbonyl)-amino]-cyclohexyl}-acetic acid methyl ester [J. Med. Chem. 43 (2000) 1878-1885] (28.5 g, 105.2 mmol) in dry tetrahydrofuran (500 mL) was cooled to -2 °C, lithium aluminum hydride (5.4 g, 142 mmol) was added portionwise and the mixture was stirred at -2 °C for 60 minutes. The reaction mixture was cooled to -10 °C and quenched with ethyl acetate (15 mL), then brine (43 ml) was slowly added to the mixture at 0 °C. The precipitated salts were filtered, and washed with ethyl acetate. The filtrate was concentrated *in vacuo.* The residue was recrystallized from diisopropyl ether (100 ml) to yield 23.7 g (93 %) of the title compound as a white powder.

### b) Methanesulfonic acid trans-2-(4-tert-butoxycarbonylamino-cyclohexyl)-ethyl ester

To a stirred solution of trans-2-{1-[4-(*N-tert*-butoxycarbonyl)-amino]-cyclohexyl}-ethanol (15 g, 62 mmol), and triethylamine (10.5 mL, 75 mmol) in dry dichloromethane (150 mL) methanesulfonyl chloride (5.7 mL, 73.4 mmol) in dichloromethane (25 mL) was added dropwise at 0 °C. After stirring 30 minutes at 0 °C, the solution was extracted three times with water. The organic solution was dried over sodium sulfate and concentrated *in vacuo* to yield 13.0 g (65 %) of the title compound.

### c) Trans-[4-(2-pyrrolidin-1-yl-ethyl)-cyclohexyl]-carbamic acid tert-butyl ester

A mixture of methanesulfonic acid trans-2-(4-*tert*-butoxycarbonylamino-cyclohexyl)-ethyl ester (3.2 g, 10 mmol), potassium carbonate (1.4 g, 10 mmol) and pyrrolidine (1.25 mL, 15 mmol) in acetonitrile (40 mL) was stirred at 60 °C for 2 hours. The mixture was cooled to room temperature and poured into water (200 mL). The precipitated white crystals were filtered off and washed with water to yield 1.9 g (64 %) of the title compound.

### d) Trans-4-(2-pyrrolidin-1-yl-ethyl)-cyclohexylamine dihydrochloride

The title compound was prepared from trans-[4-(2-pyrrolidin-1-yl-ethyl)-cyclohexyl]-carbamic acid *tert*-butyl ester according to the method described in Reference Example 3/b.

### Reference Example 5

### (4-Methyl-piperazin-1-yl)-piperidin-4-yl-methanone hydrochloride

### a) 4-(4-Methyl-piperazine-1-carbonyl)-piperidine-1-carboxylic acid tert-butyl ester

The solution of 1-(*tert*-butoxycarbonyl)-4-piperidinecarboxylic acid (Aldrich) (21.88 g, 95.4 mmol), triethylamine (13.3 mL, 95.4 mmol) and HBTU [O-benzotriazol-1-yl-N,N,N',N'-tetramethyluronium hexafluorophosphate (Advanced Chem. Tech.)] (38.36 g, 101.0 mmol) in dry dimethylformamide (100 mL) was stirred at room temperature for five minutes before *N*-methyl-piperazine (10.6 mL, 95.5 mmol) was added. The pH of the reaction mixture was adjusted to 8 by the addition of triethylamine, the so obtained mixture was stirred at room temperature overnight, then concentrated *in vacuo.* The residue was treated with saturated sodium hydrogencarbonate solution (350 mL), extracted with ethyl acetate (3x250 mL), the combined organic layers were washed with saturated sodium hydrogencarbonate solution, water and brine, dried over sodium sulfate, filtered and concentrated. The residue was submitted to column chromatography using Kieselgel 60 (0.040-0.063 mm) (Merck) as adsorbent, and chloroform:methanol = 9:1 as eluent to yield 25.8 g (87 %) of the title compound as an oil.

### b) (4-Methyl-piperazin-1-yl)-piperidin-4-yl-methanone hydrochloride

The title compound was prepared from 4-(4-methyl-piperazine-1-carbonyl)-piperidine-1-carboxylic acid *tert*-butyl ester according to the method described in Reference Example 3/b.

### Reference Example 6

### 2-(4-Pyridin-2-yl-piperazin-1-yl)-ethylamine tetrahydrochloride

### a) 2-(4-Pyridin-2-yl-piperazin-1-yl)-ethanol trihydrochloride

A stirred mixture of 1-(2-pyridyl)-piperazine (Aldrich) (4.6 mL, 30 mmol), 2-bromoethanol (2.5 mL, 35 mmol), potassium carbonate (4.8 g, 35 mmol) and 1-butanol (60 mL) was refluxed overnight, then further amount of 2-bromoethanol (2.5 mL, 35 mmol) was added and the mixture was refluxed for 24 h. After cooling to room temperature the precipitated salts were filtered off, washed with ethyl acetate and the filtrate was concentrated. The residue was dissolved in ethyl acetate (150 mL) and extracted with water (150 mL). The organic layer was dried over sodium sulfate, filtered and concentrated. The residue was dissolved in diethyl ether (100 mL), the pH of the solution was adjusted to 5 by addition of a solution of hydrogen chloride in ethyl acetate, then the mixture was stirred at room temperature for 1 h. After addition of diethyl ether (150 mL) the suspension was stirred at 0 °C for 2 h, the precipitated crystals were filtered off, washed with diethyl ether and dried to yield 4.8 g (50 %) of the title compound.

### b) 2-[2-(4-Pyridin-2-yl-piperazin-1-yl)-ethyl]-isoindole-1,3-dione

The title compound was prepared from 2-(4-pyridin-2-yl-piperazin-1-yl)-ethanol (liberated from trihydrochloride salt with 10 % sodium hydroxide solution and extracted with dichloromethane) according to the method described in Reference Example 2/a.

### c) 2-(4-Pyridin-2-yl-piperazin-1-yl)-ethylamine tetrahydrochloride

The title compound was prepared from 2-[2-(4-pyridin-2-yl-piperazin-1-yl)-ethyl]-isoindole-1,3-dione according to the method described in Reference Example 2/c.

### Reference Example 7

### N-(4-Aminomethyl-benzyl)-guanidine dihydrochloride

### a) (4-(N,N'-Ditert-butoxycarbonyl-guanidinomethyl-benzyl))-carbamic acid tert-butyl ester

A mixture of (4-aminomethyl-benzyl)-carbamic acid tert-butyl ester (Aldrich) (0.47 g, 2 mmol), 1-methyl-di*tert*-butoxy-thiourea [J. Org. Chem. 52 (1987) 1700-1703] (0.6 g, 2 mmol), HgCl₂ (0.56 g, 2 mmol) and dimethylformamide (10 mL) was stirred at room temperature for 48 hours. The precipitated salts were filtered off and the filtrate was concentrated *in vacuo.* The remaining oil was dissolved in chloroform (70 mL), washed with water (3x40 mL), dried over sodium sulfate and concentrated to yield 0.65 g (68 %) of the title compound.

### b) N-(4-Aminomethyl-benzyl)-guanidine dihydrochloride

The title compound was prepared from (4-(*N,N*'-di*tert*-butoxycarbonyl-guanidinomethyl-benzyl))-carbamic acid tert-butyl ester according to the method described in Reference Example 3/b.

### Reference Example 8

### 4-[4-(4,5-Dihydro-1H-imidazol-2-yl)-benzyl]-piperidine

### a) (4-Cyano-benzyl)-phosphonic acid diethyl ester

A mixture of 4-cyano-benzyl bromide (41.8 g, 0.213 mol) and triethyl phosphite (42 mL, 0.244 mol) was stirred in a flask equipped with a Dean-Stark trap at 150 °C for 6 h, then the reaction mixture was submitted to distillation *in vacuo* to yield 52.2 g (97%) of the title compound.

### b) 4-(1-Benzyl-piperidin-4-ylidenemethyl)-benzonitrile

Under argon, to a stirred mixture of *N*-benzyl-4-piperidone (Aldrich) (26.0 g, 0.137 mol) and (4-cyano-benzyl)-phosphonic acid diethyl ester (36.6 g, 0.1445 mol) in dimethylformamide (260 mL) sodium hydride (60 %, 7.8 g, 0.195 mol) was added at 0 °C. The reaction mixture was stirred at room temperature overnight, then ethanol (10 mL) was added dropwise, the so obtained mixture was poured into water (300 mL), and extracted with diethyl ether (3x300 mL). The organic layer was dried over sodium sulfate and concentrated. The residue was purified by column chromatography using Kieselgel 60 (0.040-0.063 mm) (Merck) as adsorbent, and n-hexane:ethyl acetate = 2:1 as eluent to yield 34.65 g (87 %) of the title compound as an oil.

### c) 4-(1-Benzyl-piperidin-4-ylidenemethyl)-benzimidic acid ethyl ester

A mixture of 4-(1-benzyl-piperidin-4-ylidenemethyl)-benzonitrile (14 g, 48.6 mmol), chloroform (10 mL) and 6 M hydrogen chloride in ethanol (300 mL) was stirred at room temperature for 48 hours. The solution was concentrated *in vacuo*, the remaining solid was repeatedly dissolved in ethanol (400 mL) and concentrated *in vacuo* to yield 16.4 g (92.4 %) of the title compound, which was used in the next steps without further purification.

### d) -1-Benzyl-4-[4-(4,5-dihydro-1H-imidazol-2-yl-benzylidene]-piperidine

To a solution of 4-(1-benzyl-piperidin-4-ylidenemethyl)-benzimidic acid ethyl ester (6.8 g, 18.3 mmol) in ethanol (250 mL) ethylenediamine (2.45 mL, 36.6 mmol) was added and the mixture was stirred at room temperature overnight. The precipitated solid was filtered off, and the filtrate was concentrated *in vacuo.* The residue was repeatedly dissolved in ethanol (100 mL) and concentrated *in vacuo.* The crude product was purified by flash chromatography, using chloroform:methanol:ammonium hydroxide =9:2:0.1 as eluent to yield 2.7 g (44.6%) of the title compound.

### e) 4-[4-(4,5-Dihydro-1H-imidazol-2-yl)-benzyl]-piperidine

To a stirred solution of 1-benzyl-4-[4-(4,5-dihydro-1*H*-imidazol-2-yl)-benzylidene]-piperidine (0.1 g, 0.3 mmol) in ethanol (20 mL), ammoniumformate (0.19 g, 3 mmol) and 10 % Pd/C (20 mg) were added ad the mixture was refluxed for 8 hours. The catalyst was filtered off, and the filtrate was concentrated *in vacuo.* The remaining crude material was purified by column chromatography using basic aluminum oxide (150 mesh, Aldrich) as adsorbent and 10 % methanol in chloroform as eluent to yield 68 mg (92%) of the title compound.

### Reference Example 9

### 4-Piperidin-4-ylmethyl-benzamidine

### a) 4-(1-Benzyl-piperidin-4-ylidenemethyl)-benzamidine

The title compound was prepared from 4-(1-benzyl-piperidin-4-ylidenemethyl)-benzimidic acid ethyl ester and methanolic ammonia according to the method described in Reference Example 8/d.

### b) 4-Piperidin-4-ylmethyl-benzamidine

The title compound was prepared from 4-(1-benzyl-piperidin-4-ylidenemethyl)-benzamidine according to the method described in Reference Example 8/e.

### Reference Example 10

### 2-(4-Piperidin-4-ylmethyl-phenyl)-1,4,5,6-tetrahydro-pyrimidine

### a) 2-[4-(1-Benzyl-piperidin-4-ylidenemethyl)-phenyl]-1,4,5,6-tetrahydro-pyrimidine

The title compound was prepared from 4-(1-benzyl-piperidin-4-ylidenemethyl)-benzimidic acid ethyl ester and 1,2-diamino-propane according to the method described in Reference Example 8/d.

### b) 2-(4-Piperidin-4-ylmethyl-phenyl)-1,4,5,6-tetrahydro-pyrimidine

The title compound was prepared from 2-[4-(1-benzyl-piperidin-4-ylidenemethyl)-phenyl]-1,4,5,6-tetrahydro-pyrimidine according to the method described in Reference Example 8/e.

### Reference Example 11

### 2-(4-Pyridin-4-yl-piperazin-1-yl)-propylamine

### a) 2-[2-(4-Pyridin-4-yl-piperazin-1-yl)-propyl]-isoindole-1,3-dione

The title compound was prepared from 1-pyridin-4-yl-piperazine [Org. Lett. 4 (2002) 737-740] and *N*-(2-bromopropyl)-phthalimide according to the method described in Reference Example 1/a.

### b) 2-(4-Pyridin-4-yl-piperazin-1-yl)-propylamine

The title compound was prepared from 2-[2-(4-pyridin-4-yl-piperazin-1-yl)-propyl]-isoindole-1,3-dione according to the method described in Reference Example 1/b.

### Reference Example 12

### 4-(4-Amino-butyl)-piperidine-1-carboxylic acid tert-butyl ester

### a) 4-(3-Methanesulfonyloxy-propyl)-piperidine-1-carboxylic acid tert-butyl ester

To a solution of 4-(3-hydroxypropyl)-piperidine-1-carboxylic acid *tert*-butyl ester (2.12 g, 8.7 mmol) in dichloromethane (10 mL) triethylamine (1.33 mL, 9.57 mmol) and methanesulfonyl chloride (0.74 mL, 9.57 mmol) were added at 0 °C and the reaction mixture was stirred at this temperature for 1 h. After quenching the reaction by the addition of methanol (1 mL), the mixture was washed with water, saturated sodium hydrogencarbonate solution and water, dried over sodium sulfate, filtered and concentrated to yield 2.73 g (97%) of the title compound.

### b) 4-(3-Cyano-propyl)-piperidine-1-carboxylic acid tert-butyl ester

To a solution of 4-(3-methanesulfonyloxy-propyl)-piperidine-1-carboxylic acid *tert-*butyl ester (1.35 g, 4.2 mmol) in dimethylformamide (30 mL) potassium cyanide (0.33 g, 5.1 mmol) was added and the reaction mixture was stirred at 80 °C for 20 h. After concentration *in vacuo* the residue was treated with water and extracted with ethyl acetate (3x50mL). The combined organic layers were washed with water and brine, dried over sodium sulfate, filtered and concentrated to yield 0.919 g (87%) of the title compound.

### c) 4-(4-Amino-butyl)-piperidine-1-carboxylic acid tert-butyl ester

To a stirred solution of 4-(3-cyano-propyl)-piperidine-1-carboxylic acid tert-butyl ester (0.896 g, 3.55 mmol) and lithium hydroxide hydrate (0.447 g, 10.65 mmol) in a mixture of dioxane (56 mL) and water (14 mL) 10% Pd/C (90 mg) and Raney Ni (0.42 g) were added. The reaction mixture was hydrogenated at 50 °C for 3 h, then the catalysts were filtered off and the filtrate was concentrated. The residue was purified by column chromatography using Kieselgel 60 (0.015-0.040 mm) (Merck) as adsorbent, and methanol:ammonium hydroxide = 10:1 as eluent to yield 0.493 g (54 %) of the title compound. MS (EI) 257.2 (MH⁺).

### Reference Example 13

### 4-(3-Amino-propyl)-piperidine-1-carboxylic acid tert-butyl ester

### a) 4-(3-Azido-propyl)-piperidine-1-carboxylic acid tert-butyl ester

To a solution of 4-(3-methanesulfonyloxy-propyl)-piperidine-1-carboxylic acid *tert-*butyl ester (Reference Example 13/a) (1.35 g, 4.2 mmol) in dimethylformamide (30 mL) sodium azide (0.33 g, 5.1 mmol) was added and the reaction mixture was stirred at 80 °C for 20 h. After concentration *in vacuo* the residue was treated with water and extracted with ethyl acetate (3x50mL). The combined organic layers were washed with water and brine, dried over sodium sulfate, filtered and concentrated to yield 1.08 g (96 %) of the title. MS (EI) 291.3 (M+Na⁺).

### b) 4-(3-Amino-propyl)-piperidine-1-carboxylic acid tert-butyl ester

To a solution of 4-(3-azido-propyl)-piperidine-1-carboxylic acid tert-butyl ester (1.738 g, 6.48 mmol) in dry tetrahydrofuran (50 mL) water (0.49 mL) and triphenyl phosphine (3.4 g, 12.96 mmol) were added. The reaction mixture was stirred at room temperature overnight, then concentrated. The residue was purified by column chromatography using Kieselgel 60 (0.015-0.040 mm) (Merck) as adsorbent, and methanol:ammonium hydroxide =10:1 as eluent to yield 1.498 g (95 %) of the title compound. MS (EI) 243.2 (MH⁺).

### Reference Example 14

### 2-(1'-Methyl-[1,4']bipiperidinyl-4-yl)-ethylamine tri-trifluoroacetate

### a) [2-(1'-Methyl-[1,4']bipiperidinyl-4-yl)-ethyl]-carbamic acid tert-butyl ester

To a solution of triethylamine (44 mg, 0.44 mmol) in dry ethanol (5 mL) (2-piperidin-4-yl-ethyl)-carbamic acid tert-butyl ester [Bioorg. Med. Chem. Lett.; 11 (2001) 2325-2330] (300 mg, 0.44 mmol), titanium(IV)isoproproxide (125 mg, 0.44 mmol), and *N*-methyl-piperidone (50 mg, 0.44 mmol) were added. The reaction mixture was stirred at 25 °C for 20 h, then sodium borohydride (17 mg, 0.44 mmol) was added and the resulted mixture was further stirred at 25 °C for 20 h. The reaction was then quenched by pouring the mixture into 2 N aqueous ammonia (0.66 mL), the obtained inorganic precipitate was filtered off and washed with dichloromethane, and the aqueous filtrate was extracted with dichloromethane. The combined extracts were dried over sodium sulfate, filtered and concentrated *in vacuo.* The residue was purified by column chromatography using Kieselgel 60 (0.040-0.063 mm) (Merck) as adsorbent, and chloroform:methanol:ammonium hydroxide = 10:1:0.1 as eluent to yield 77 mg (54 %) of the title compound as yellowish oil. MS (EI) 326.3 (MH⁺).

### b) 2-(1'-Methyl-[1,4']bipiperidinyl-4-yl)-ethylamine tri-trifluoroacetate

To an ice cold solution of [2-(1'-methyl-[1,4']bipiperidinyl-4-yl)-ethyl]-carbamic acid *tert*-butyl ester (77 mg, 0.236 mmol) in dichloromethane (5 mL) trifluoroacetic acid (0.15 mL) was added and the reaction mixture was stirred at room temperature overnight, then concentrated in vacuo to yield 127 mg (95 %) of the title compound. MS (EI) 226.4 (MH⁺).

### Reference (Example 15

### 2-(4-Pyridin-2-ylmethyl-piperazin-1-yl)-ethylamine

### a) (4-Pyridin-2-ylmethyl-piperazin-1-yl)-acetonitrile

To a stirred solution of 1-(2-pyridylmethyl)piperazine (EMKA-Chemie) (309 mg, 1.69 mmol) in ethanol (10 mL) chloro-acetonitrile (306 mg, 4.06 mmol) and sodium carbonate (718 mg, 6.77 mmol) were added and the mixture was refluxed for 4 h. The precipitated inorganic salts were then filtered off, washed with ethanol and the filtrate was concentrated *in vacuo* to yield 340 mg (93 %) of the title compound as a yellowish oil. MS (EI) 217.2 (MH⁺).

### b) 2-(4-Pyridin-2-ylmethyl-piperazin-1-yl-ethylamine

Under argon atmosphere to a suspension of lithium aluminum hydride (125 mg, 3.37 mmol) in dry tetrahydrofuran (15 mL) a solution of (4-pyridin-2-ylmethyl-piperazin-1-yl)-acetonitrile (340 mg, 1.57 mmol) in tetrahydrofuran (15 mL) was added and the reaction mixture was stirred at room temperature for 3 h. The reaction was then quenched by addition of 10 % sodium hydroxide solution (0.2 mL) and water (0.75 mL) and the mixture was stirred at room temperature overnight. The insoluble material was filtered off, washed with tetrahydrofuran and the filtrate was concentrated *in vacuo* to yield 338 mg (98%) of the title compound as a yellowish solid. MS (EI) 221.2 (MH⁺).

### Reference Example 16

### 2-(2,3,5,6-Tetrahydro-[1,2']bipyrazinyl-4-yl)-ethylamine

### a) (2,3,5,6-Tetrahydro-[1,2']bipyrazinyl-4-yl)-acetonitrile

The title compound was prepared from 1-(2-pyrazinylmethyl)piperazine (EMKA-Chemie) according to the method described in Reference Example 17/a. MS (EI) 204.2 (MH⁺).

### b) 2-(2,3,5,6-Tetrahydro-[1,2']bipyrazinyl-4-yl)-ethylamine

The title compound was prepared from (2,3,5,6-tetrahydro-[1,2']bipyrazinyl-4-yl)-acetonitrile according to the method described in Reference Example 17/b. MS (EI) 208.2 (MH⁺).

### Reference Example 17

### 4-(4-Aminomethyl-benzyl)-piperidine-1-carboxylic acid tert-butyl ester

### a) 4-Piperidin-4-ylmethyl-benzonitrile

To a stirred solution of 4-(1-benzyl-piperidin-4-ylidenemethyl)-benzonitrile (Reference Example 8/b) (17.4 g, 60 mmol) in methanol (100 mL) 10 % Pd/C (1.7 g) was added and the reaction mixture was hydrogenated for 3 days, then the catalyst was filtered off and the filtrate was concentrated. The residue was purified by column chromatography using Kieselgel 60 (0.015-0.040 mm) (Merck) as adsorbent, and chloroform:methanol:ammonium hydroxide = 4:1:0.1 as eluent to yield 3.6 g (30 %) of the title compound.

### b) 4-(4-Cyano-benzyl)-piperidine-1-carboxylic acid tert-butyl ester

To a solution of 4-piperidin-4-ylmethyl-benzonitrile (3.6 g, 18 mmol) in dioxane (105 mL) and water (52 mL) di-tert-butyl dicarbonate (5.44 g, 25 mmol) was added and the reaction mixture was stirred overnight at room temperature. After concentration the residue was partitioned between water and chloroform, the organic layer was dried over sodium sulfate, filtered and concentrated *in vacuo* to yield 4.0 g (74 %) of the title compound as a yellowish oil, which solidifies on standing. MS (EI) 323.1 (M+Na⁺).

### c) 4-(4-Aminomethyl-benzyl)-piperidine-1-carboxylic acid tert-butyl ester

The title compound was prepared from 4-(4-cyano-benzyl)-piperidine-1-carboxylic acid tert-butyl ester according to the method described in Reference Example 13/c. MS (EI) 305.3 (MH⁺).

### Example 1

### 4-[2-(2,4-Dichloro-phenoxy)-phenylsulfamoyl]-N-{2-[4-pyridin-4-yl-piperazin-1-yl)-ethyl]-benzamide dihydrochloride

### a) 2,4-Dichloro-1-(2-nitro-phenoxy)-benzene

A mixture of 1-fluoro-2-nitrobenzene (4.8 mL, 45.42 mmol), potassium carbonate (13.8 g, 0.1 mol) and 2,4-dichloro-phenol (8.16 g, 50.06 mmol) in dry dimethylformamide (70 mL) was stirred at 100 °C for 2 h. Solids were filtered off, and the filtrate was concentrated in vacuo. The residue was partitioned between diethyl ether and 1N sodium hydroxide, the organic layer was washed with 1N sodium hydroxide, water and brine, dried over sodium sulfate, filtered and concentrated *in vacuo* to yield 11.69 g (91 %) of the title compound as a yellowish oil, which solidifies on standing. MS (EI) 285.2 (MH⁺). Lit. [Chem. Heterocycl. Compd. (Engl. Transl.) 11 (1975) 1356-1358]

### b) 2-(2,4-Dichloro-phenoxy)-phenylamine [Chem. Abstr. 84 (1976) 164313q]

To a stirred solution of 2,4-dichloro-1-(2-nitro-phenoxy)-benzene (3.5 g, 12.32 mmol) in ethyl acetate (60 mL) stannous chloride dihydrate (13.89 g, 61.6 mmol) was added and the mixture was refluxed for 2 h before it was quenched with saturated sodium hydrogencarbonate solution (192 mL). The organic phase was separated and the aqueous phase was washed several times with ethyl acetate. The combined extracts were dried over sodium sulfate, filtered and concentrated in vacuo to yield 3.1 g (99 %) of the title compound as a yellowish oil: MS (EI) 255.2 (MH⁺).

### c) 4-[2-(2,4-Dichloro-phenoxy)-phenylsulfamoyl]-benzoic acid

Under an atmosphere of argon to an ice cooled solution of 2-(2,4-dichloro-phenoxy)-phenylamine (0.5 g, 1.97 mmol) in dry pyridine (5 mL) 4-chlorosulfonyl benzoic acid (0.45 g, 1.97 mmol) was added portion-wise. The reaction mixture was stirred at room temperature overnight. The mixture was evaporated *in vacuo,* the residue was treated with 1N hydrochloric acid (20 mL), and extracted with ethyl acetate (3x50 mL). The combined organic layers were washed with 1N hydrochloric acid, water and brine, dried over sodium sulfate, filtered and concentrated in vacuo. The residue was submitted to flash column chromatography using Kieselgel 60 (0.015-0.040 mm) as adsorbent (Merck) and chloroform:methanol:acetic acid = 294:6:1 as eluent to yield 0.6 g (70 %) of the title compound as a light pink solid, which was crystallized from diethyl ether-petroleum ether. MS (EI) 439.3 (MH⁺).

### d) 4-[2-(2,4-Dichloro-phenoxy)-phenylsulfamoyl]-N-{2-[4-pyridin-4-yl-piperazin-1-yl)-ethyl]-benzamide dihydrochloride

The solution of 4-[2-(2,4-dichloro-phenoxy)-phenylsulfamoyl]-benzoic acid (0.2 g, 0.45 mmol), triethylamine (0.07 mL, 0.5 mmol) and HBTU [O-benzotriazol-1-yl-N,N,N',N'-tetramethyluronium hexafluorophosphate (Advanced Chem. Tech.)] (0.19 g, 0.5 mmol) in dry dimethylformamide (5 mL) was stirred at room temperature for five minutes before 2-(4-pyridin-4-yl)-piperazin-1-yl)-ethylamine (Reference Example 1) (0.11 g, 0.5 mmol) was added. The pH of the reaction mixture was adjusted to 8 by the addition of triethylamine, the so obtained mixture was stirred at room temperature overnight, then concentrated *in vacuo.* The residue was treated with saturated sodium hydrogencarbonate solution (10 mL), extracted with ethyl acetate (3x25 mL), the combined organic layers were washed with saturated sodium hydrogencarbonate solution, water and brine, dried over sodium sulfate, filtered and concentrated. The residue was submitted to flash column chromatography using Kieselgel 60 (0.015-0.040 mm) as adsorbent (Merck) and chloroform:methanol:ammonium hydroxide = 95:5:1 as eluent, then converted to dihydrochloride salt form to yield 0.078 g (25 %) of the title compound as light yellowish amorphous solid. MS (EI) 627.5 (MH⁺).

### Example 2

### 4-[2-(2,4-Dichloro-phenoxy)-phenylsulfamoyl]-N-{2-[4-(4,5-dihydro-1H-imidazol-2-yl)-phenyl]-ethyl}-benzamide trifluoroacetate

A solution of 4-[2-(2,4-dichloro-phenoxy)-phenylsulfamoyl]-benzoic acid (Example 1/c) (0.35 g, 0.8 mmol), triethylamine (0.36 mL, 2.56 mmol) and HBTU (0.34 g, 0.88 mmol) in dry dimethylformamide (8 mL) was stirred at room temperature for five minutes before 2-[4-(4,5-dihydro-1*H*-imidazol-2-yl)-phenyl]-ethylamine dihydrochloride (Reference Example 2) (0.23 g, 0.88 mmol) was added. The pH of the reaction mixture was adjusted to 8 by the addition of triethylamine, the so obtained mixture was stirred at room temperature overnight, then concentrated *in vacuo.* The residue was submitted to reversed phase HPLC using YMC-Pack ODS-AQ type packings (produced by YMC) and acetonitrile/water/trifluoroacetic acid as eluent. After concentration of the proper fractions, the residue was triturated with ether, filtered and dried to yield 0.33g (57 %) of the title compound as a white amorphous solid. MS (EI) 610.5 (MH⁺).

### Example 3

### N-(3-[1,4']Bipiperidinyl-1'-yl-propyl)-4-[2-(2,4-dichloro-phenoxy)-phenylsulfamoyl]-benzamide

The title compound was prepared from 4-[2-(2,4-dichloro-phenoxy)-phenylsulfamoyl]-benzoic acid (Example 1/c) and 3-[1,4']bipiperidinyl-1'-yl-propylamine trihydrochloride (Reference Example 3) according to Example 1/d. MS (EI) 646.5 (MH⁺).

### Example 4

### 4-[2-(3,4-Dichloro-phenoxy)-phenylsulfamoyl]-N-{2-[4-(4,5-dihydro-1H-imidazol-2-yl)-phenyl]-ethyl}-benzamide

### a) 4-[2-(3,4-Dichloro-phenoxy)-phenylsulfamoyl]-benzoic acid

To a stirred solution of 2-(3,4-dichloro-phenoxy)-phenylamine [Chem. Pharm. Bull. 33 (1985) 4409-4421] (0.2 g, 0.787 mmol) in dry pyridine (5 mL) 4-chlorosulfonyl benzoic acid (0.2 g, 0.9 mmol) was added. The reaction mixture was stirred at room temperature overnight, then poured into ice-water (50 mL). The precipitated crystals were filtered off, washed with water and dried to yield 0.3 g (87%) of the title compound.

### b) 4-[2-(3,4-Dichloro-phenoxy)-phenylsulfamoyl]-N-{2-[4-(4,5-dihydro-1H-imidazol-2-yl)-phenyl]-ethyl}-benzamide

The solution of 4-[2-(3,4-dichloro-phenoxy)-phenylsulfamoyl]-benzoic acid (0.11 g, 0.25 mmol), triethylamine (0.07 mL, 0.5 mmol) and HBTU (0.11 g, 0.29 mmol) in dry dimethylformamide (5 mL) was stirred at room temperature for five minutes before 2-[4-(4,5-dihydro-1*H*-imidazol-2-yl)-phenyl]-ethylamine dihydrochloride (Reference Example 2) (0.11 g, 0.5 mmol) was added. The pH of the reaction mixture was adjusted to 8 by the addition of triethylamine, the so obtained mixture was stirred at room temperature overnight, then concentrated *in vacuo.* The residue was treated with saturated sodium hydrogencarbonate solution (30 mL), the precipitated crystals were filtered off, washed with water and dried. The crude product was purified by column chromatography using Kieselgel 60 (0.040-0.063 mm) (Merck) as adsorbent, and chloroform:methanol:ammonium hydroxide = 9:1:0.1 as eluent. The product was crystallized with diethyl ether to yield 0.65 g (42 %) of the title compound.

### Example 5

### N-(3-[1,4']Bipiperidinyl-1'-yl-propyl)-4-[2-(3,4-dichloro-phenoxy)-phenylsulfamoyl]-benzamide

The title compound was prepared from 4-[2-(3,4-dichloro-phenoxy)-phenylsulfamoyl]-benzoic acid (Example 4/a) and 3-[1,4']bipiperidinyl-1'-yl-propylamine trihydrochloride (Reference Example 3) according to the method described in Example 1/d.

### Example 6

### 4-[2-(4-Chloro-phenoxy)-phenylsulfamoyl]-N-{2-[4-(4,5-dihydro-1H-imidazol-2-yl)-phenyl]-ethyl}-benzamide

### a) 4-[2-(4-Chloro-phenoxy)-phenylsulfamoyl]-benzoic acid

The title compound was prepared from 2-(4-chloro-phenoxy)-phenylamine [Collect. Czech. Chem. Commun. 65 (2000) 862-880] according to the method described in Example 4/a.

### b) 4-[2-(4-Chloro-phenoxy)-phenylsulfamoyl]-N-{2-[4-(4,5-dihydro-1H-imidazol-2-yl)-phenyl]-ethyl}-benzamide

The title compound was prepared from 4-[2-(4-chloro-phenoxy)-phenylsulfamoyl]-benzoic acid and 2-[4-(4,5-dihydro-1*H*-imidazol-2-yl)-phenyl]-ethylamine dihydrochloride (Reference Example 2) according to the method described in Example 4/b.

### Example 7

### 4-[2-(4-Bromo-phenoxy)-phenylsulfamoyl]-N-{2-[4-(4,5-dihydro-1H-imidazol-2-yl)-phenyl]-ethyl}-benzamide

### a) 4-[2-(4-Bromo-phenoxy)-phenylsulfamoyl]-benzoic acid

The title compound was prepared from 2-(4-bromo-phenoxy)-phenylamine [J. Chem.. Soc. (1930) 1202, 1206] according to the method described in Example 4/a.

### b) 4-[2-(4-Bromo-phenoxy)-phenylsulfamoyl]-N-{2-[4-(4,5-dihydro-1H-imidazol-2-yl)-phenyl]-ethyl}-benzamide

The title compound was prepared from 4-[2-(4-bromo-phenoxy)-phenylsulfamoyl]-benzoic acid and 2-[4-(4,5-dihydro-1*H*-imidazol-2-yl)-phenyl]-ethylamine dihydrochloride (Reference Example 2) according to the method described in Example 4/b.

### Example 8

### N-(4-[1,4']Bipiperidinyl-1'-yl-phenyl)-4-[2-(2,4-dichloro-phenoxy)-phenylsulfamoyl]-benzamide

A solution of 4-[2-(2,4-dichloro-phenoxy)-phenylsulfamoyl]-benzoic acid (Example 1/c) (0.3 g, 0.68 mmol), HOBt [1-hydroxybenzotriazol] (0.093 g, 0.68 mmol) and EDC hydrochloride [*N*-(3-dimethylaminopropyl)-*N*'-ethylcarbodiimide] (0.132 g, 0.68 mmol) in dry dimethylformamide (5 mL) was stirred at 0 °C for ten minutes before 4-[1,4']bipiperidinyl-1'-yl-phenylamine [J. Med. Chem. 46 (2003) 1803-1806] (0.178 g, 0.68 mmol) was added and the reaction mixture was stirred at room temperature overnight. The mixture was concentrated *in vacuo*, the residue was treated with saturated sodium hydrogencarbonate solution (10 mL), and extracted with chloroform (3x10 mL). The combined organic layers were dried over sodium sulfate, filtered, and concentrated *in vacuo.* The residue was recrystallized from chloroform to yield 0.115 g (25 %) of the title compound as yellow amorphous solid. MS (EI) 680.6 (MH⁺).

### Example 9

### trans-4-[2-(2,4-Dichloro-phenoxy)-phenylsulfamoyl]-N-[4-(2-pyrrolidin-1-ylethyl)-cyclohexyl]-benzamide

The title compound was prepared from 4-[2-(2,4-dichloro-phenoxy)-phenylsulfamoyl]-benzoic acid (Example 1/c) and trans-4-(2-pyrrolidin-1-ylethyl)-cyclohexylamine dihydrochloride (Reference Example 4) according to the method described in Example 4/b. MS (EI) 617.6 (MH⁺).

### Example 10

### 3-[2-(2,4-Dichloro-phenoxy)-phenylsulfamoyl]-N-[2-(4-pyridin-4-yl-piperazin-1-yl)-ethyl]-benzamide dihydrochloride

### a) 3-[2-(2,4-Dichloro-phenoxy)-phenylsulfamoyl]-benzoic acid

The title compound was prepared from 2-(2,4-dichloro-phenoxy)-aniline (Example 1/b) and 3-chlorosulfonyl benzoic acid according to the method described in Example 1/c. MS (EI) 439.3 (MH⁺).

### b) 3-[2-(2,4-dichlorophenoxy)-phenylsulfamoyl]-N-{2-[4-pyridine-4-yl-pyperazine-1-yl)-ethyl]-benzamide dihydrochloride

The title compound was prepared from 3-[2-(2,4-dichloro-phenoxy)-phenylsulfamoyl]-benzoic acid and 2-(4-pyridin-4-yl)-piperazin-1-yl)-ethylamine (Reference Example 1) according to the method described in Example 1/d. MS (EI) 627.5 (MH⁺).

### Example 11

### 4-[2-(2,4-Dichloro-phenoxy)-phenylsulfamoyl]-N-(2-piperidin-4-ylethyl)-benzamide trifluoroacetate

### a) 4-(2-{4-[2-(2,4-Dichloro-phenoxy)-phenylsulfamoyl]-benzoylamino}-ethyl)-piperidine-1-carboxylic acid tert-butyl ester

The title compound was prepared from 4-[2-(2,4-dichloro-phenoxy)-phenylsulfamoyl]-benzoic acid (Example 1/c) and 4-(2-amino-ethyl)-piperidine-1-carboxylic acid *tert*-butyl ester [Bioorg. Med. Chem. Lett. 13 (2003) 2167-2172] according to the method described in Example 1/d. MS (EI) 672.6 (M+Na⁺).

### b) 4-[2-(2,4-Dichloro-phenoxy)-phenylsulfamoyl]-N-(2-piperidin-4-ylethyl)-benzamide trifluoroacetate

To an ice cold solution of 4-(2-{4-[2-(2,4-dichloro-phenoxy)-phenylsulfamoyl]-benzoylamino}-ethyl)-piperidine-1-carboxylic acid *tert*-butyl ester (0.26 g, 0.4 mmol) in dichloromethane (2 mL) trifluoroacetic acid (0.4 mL) was added and the reaction mixture was stirred at room temperature overnight, then concentrated. The residue was triturated with diethyl ether, filtered and dried to yield 0.22 g (83 %) of the title compound as a white amorphous solid. MS (EI) 549.4 (MH⁺).

### Example 12

### 4-[2-(2,4-Dichloro-phenoxy)-phenylsulfamoyl]-N-(2-dimethylamino-ethyl)-benzamide

The title compound was prepared from 4-[2-(2,4-dichloro-phenoxy)-phenylsulfamoyl]-benzoic acid (Example 1/c) and *N,N*-dimethylethylenediamine (Aldrich) according to the method described in Example 1/d. MS (EI) 509.4 (MH⁺).

### Example 13

### 4-[2-(2,4-Dichloro-phenoxy)-phenylsulfamoyl]-N-[4-(4,5-dihydro-1H-imidazol-2-yl)-benzyl]-N-methyl-benzamide trifluoroacetate

### a) 4-[2-(2,4-Dichloro-phenoxy)-phenylsulfamoyl]-N-(4-cyano-benzyl)-N-methyl-benzamide

The title compound was prepared from 4-[2-(2,4-dichloro-phenoxy)-phenylsulfamoyl]-benzoic acid (Example 1/c) and *N*-methyl-4'-cyano-benzylamine [J. Med. Chem. 26 (1983) 309-312] according to the method described in Example 1/d.

### b) 4-[2-(2,4-Dichloro-phenoxy)-phenylsulfamoyl]-N-[4-(4,5-dihydro-1H-imidazol-2-yl)-benzyl]-N-methyl-benzamide trifluoroacetate

Dry hydrogen chloride gas was bubbled through an ice cold solution of 4-[2-(2,4-dichloro-phenoxy)-phenylsulfamoyl]-*N*-(4-cyano-benzyl)-*N*-methyl-benzamide (0.51 g, 0.9 mmol) in dry ethanol (50 mL) for an hour, then the so obtained mixture was kept at 8 °C overnight. The reaction mixture was concentrated *in vacuo*, the residue was dissolved in dry ethanol (15 mL), ethylenediamine (67 µL, 0.99 mmol) was added and the reaction mixture was stirred at room temperature overnight. The mixture was concentrated *in vacuo*, the residue was subjected to reversed phase HPLC using YMC-Pack ODS-AQ type packings (produced by YMC) and acetonitrile/water/trifluoroacetic acid as eluent. After concentration of the proper fractions, the residue was triturated with ether, filtered and dried to yield 0.347g (53 %) of the title compound as a white solid. MS (EI) 610.5 (MH⁺).

### Example 14

### 4-[2-(2,4-Dichloro-phenoxy)-phenylsulfamoyl]-N-piperidin-4-ylmethyl-benzamide trifluoroacetate

### a) 4-({4-[2-(2,4-Dichloro-phenoxy)-phenylsulfamoyl]-benzoylamino}-methyl)-piperidine-1-carboxylic acid tert-butyl ester

The title compound was prepared from 4-[2-(2,4-dichloro-phenoxy)-phenylsulfamoyl]-benzoic acid (Example 1/c) and 4-aminomethyl-piperidine-1-carboxylic acid *tert*-butyl ester (Fluka) according to the method described in Example 4/b.

### b) 4-[2-(2,4-Dichloro-phenoxy)-phenylsulfamoyl]-N-piperidin-4-ylmethyl-benzamide trifluoroacetate

The title compound was prepared from 4-({4-[2-(2,4-dichloro-phenoxy)-phenylsulfamoyl]-benzoylamino}-methyl)-piperidine-1-carboxylic acid *tert*-butyl ester according to the method described in Example 11/b. MS (EI) 535.5 (MH⁺).

### Example 15

### 4-[2-(2,4-Dichloro-phenoxy)-phenylsulfamoyl]-N-[2-(4,5-dihydro-1H-imidazol-2-yl)-ethyl]-benzamide

### a) 4-[2-(2,4-Dichloro-phenoxy)-phenylsulfamoyl]-N-(propionitrile-3-yl)-benzamide

The title compound was prepared from 4-[2-(2,4-dichloro-phenoxy)-phenylsulfamoyl]-benzoic acid (Example 1/c) and 3-aminopropionitrile (Fluka) according to the method described in Example 1/d. MS 491.4 (EI) (MH⁺).

### b) 4-[2-(2,4-Dichloro-phenoxy)-phenylsulfamoyl]-N-[2-(4,5-dihydro-1H-imidazole-2-yl)-ethyl]-benzamide

The title compound was prepared from 4-[2-(2,4-dichloro-phenoxy)-phenylsulfamoyl]-*N*-(propionitrile-3-yl)-benzamide according to the method described in Example 13/b.

### Example 16

### 4-[2-(2,4-Dichloro-phenoxy)-phenylsulfamoyl]-N-piperidin-4-yl-benzamide trifluoroacetate

### a) 4-{4-[2-(2,4-Dichloro-phenoxy)-phenylsulfamoyl]-benzoylamino}-piperidine-1-carboxylic acid tert-butyl ester

The title compound was prepared from 4-[2-(2,4-dichloro-phenoxy)-phenylsulfamoyl]-benzoic acid (Example 1/c) and 4-amino-piperidine-1-carboxylic acid *tert-*butyl ester hydrochloride (Fluka) according to the method described in Example 4/b. MS (EI) 643.5 (M+Na⁺).

### b) 4-[2-(2,4-Dichloro-phenoxy)-phenylsulfamoyl]-N-piperidin-4-yl-benzamide trifluoroacetate

The title compound was prepared from 4-{4-[2-(2,4-dichloro-phenoxy)-phenylsulfamoyl]-benzoylamino}-piperidine-1-carboxylic acid *tert*-butyl ester according to the method described in Example 11/b.

### Example 17

### 4-[2-(2,4-Dichloro-phenoxy)-phenylsulfamoyl]-N-methyl-benzamide

The title compound was prepared from 4-[2-(2,4-dichloro-phenoxy)-phenylsulfamoyl]-benzoic acid (Example 1/c) and methylamine (1.61 M solution in xylene) according to the method described in Example 1/d. MS 452.3 (EI) (MH⁺).

### Example 18

### N-[2-(2,4-Dichloro-phenoxy)-phenyl]-4-[4-(4-methyl-piperazin-1-carbonyl)-piperidin-1-carbonyl]-benzenesulfonamide

The title compound was prepared from 4-[2-(2,4-dichloro-phenoxy)-phenylsulfamoyl]-benzoic acid (Example 1/c) and (4-methyl-piperazin-1-yl)-piperidin-4-yl-methanone hydrochloride (Reference Example 5) according to the method described in Example 1/d. MS 632.5 (EI) (MH⁺).

### Example 19

### 4-[2-(2,4-Dichloro-phenoxy)-phenylsulfamoyl]-N-(2-dimethylamino-ethyl)-N-methyl-benzamide

The title compound was prepared from 4-[2-(2,4-dichloro-phenoxy)-phenylsulfamoyl]-benzoic acid (Example 1/c) and *N,N,N*'-trimethyl-ethylenediamine (Aldrich) according to the method described in Example 1/d. MS 523.3 (EI) (MH⁺).

### Example 20

### 4-[2-(2,4-Dichloro-phenoxy)-phenylsulfamoyl]-N-[2-(4-pyridin-2-yl-piperazin-1-yl)-ethyl]-benzamide trifluoroacetate

The title compound was prepared from 4-[2-(2,4-dichloro-phenoxy)-phenylsulfamoyl]-benzoic acid (Example 1/c) and 2-(4-pyridin-2-yl-piperazin-1-yl)-ethylamine (Reference Example 6) according to the method described in Example 2.

### Example 21

### 4-([1,4']Bipiperidinyl-1'-carbonyl)-N-[2-(2,4-dichloro-phenoxy)-phenyl]-benzene-sulfonamide

The title compound was prepared from 4-[2-(2,4-dichloro-phenoxy)-phenylsulfamoyl]-benzoic acid (Example 1/c) and 4,4'-bipiperidine according to the method described in Example 8.

### Example 22

### 4-[2-(4-Bromo-2-chloro-phenoxy)-phenylsulfamoyl]-N-[2-[4-(4,5-dihydro-1H-imidazol-2-yl)-phenyl]-ethyl}-benzamide

### a) 4-Bromo-2-chloro-1-(2-nitro-phenoxy)-benzene

The title compound was prepared from 4-bromo-2-chloro-phenol according to the method described in Example 1/a. MS (EI) 329.3 (MH⁺).

### b) 2-(4-Bromo-2-chloro-phenoxy)-phenylamine

The title compound was prepared from 4-bromo-2-chloro-1-(2-nitro-phenoxy)-benzene according to the method described in Example 1/b. MS (EI) 300.2 (MH⁺).

### c) 4-[2-(4-Bromo-2-chloro-phenoxy)-phenylsulfamoyl]-benzoic acid

The title compound was prepared from 2-(4-bromo-2-chloro-phenoxy)-phenylamine according to the method described in Example 1/c. MS (EI) 483.4 (MH⁺).

### d) 4-[2-(4-Bromo-2-chloro-phenoxy)-phenylsulfamoyl]-N-{2-[4-(4,5-dihydro-1H-imidazol-2-yl)-phenyl]-ethyl}-benzamide

The title compound was prepared from 4-[2-(4-bromo-2-chloro-phenoxy)-phenylsulfamoyl]-benzoic acid and 2-[4-(4,5-dihydro-1*H*-imidazol-2-yl)-phenyl]-ethylamine dihydrochloride (Reference Example 2) according to the method described in Example 2. MS 655 (EI) (MH⁺).

### Example 23

### 4-[2-(4-Bromo-phenoxy)-phenylsulfamoyl]-N-(4-guanidinomethyl-benzyl)-benzamide hydrochloride

The title compound was prepared from 4-[2-(4-bromo-phenoxy)-phenylsulfamoyl]-benzoic acid (Example 7/a) and *N*-(4-aminomethyl-benzyl)-guanidine dihydrochloride (Reference Example 7) according to the method described in Example 2.

### Example 24

### 4-[2-(2,4-Dichloro-phenoxy)-phenylsulfamoyl]-N-[2-(1'-methyl-[1,4']bipiperidinyl-4-yl)-ethyl]-benzamide

The title compound was prepared from 4-[2-(2,4-dichloro-phenoxy)-phenylsulfamoyl]-benzoic acid (Example 1/c) and 2-(1'-methyl-[1,4']bipiperidinyl-4-yl)-ethylamine tri-trifluoroacetate (Reference Example 14) according to the method described in Example 1/d. MS (EI) 646.3 (MH⁺).

### Example 25

### N-[2-(4-Bromo-phenoxy)-phenyl]-4-{4-[4-(4,5-dihydro-1H-imidazol-2-yl)-benzyl]-piperidine-1-carbonyl}-benzenesulfonamide

The title compound was prepared from 4-[2-(4-bromo-phenoxy)-phenylsulfamoyl]-benzoic acid (Example 7/a) and 4-[4-(4,5-dihydro-1*H*-imidazol-2-yl)-benzyl]-piperidine (Reference Example 8) according to the method described in Example 2.

### Example 26

### 4-(1-{4-[2-(4-Chloro-phenoxy)-phenylsulfamoyl]-benzoyl}-piperidin-4-ylmethyl)-benzamidine

The title compound was prepared from 4-[2-(4-chloro-phenoxy)-phenylsulfamoyl]-benzoic acid (Example 6/a) and 4-piperidin-4-ylmethyl-benzamidine (Reference Example 9) according to the method described in Example 2.

### Example 27

### N-[2-(4-Chloro-phenoxy)-phenyl]-4-{4-[4-(1,4,5,6-tetrahydro-pyrimidin-2-yl)-benzyl]-piperidine-1-carbonyl}-benzenesulfonamide

The title compound was prepared from 4-[2-(4-chloro-phenoxy)-phenylsulfamoyl]-benzoic acid (Example 6/a) and 2-(4-piperidin-4-ylmethyl-phenyl)-1,4,5,6-tetrahydropyrimidine (Reference Example 10) according to the method described in Example 2.

### Example 28

### 4-[2-(2,4-Dichloro-phenoxy)-phenylsulfamoyl]-N-{2-[4-pyridin-4-yl-piperazin-1-yl)-propyl]-benzamide

The title compound was prepared from 4-[2-(2,4-dichloro-phenoxy)-phenylsulfamoyl]-benzoic acid (Example 1/c) and 2-(4-pyridin-4-yl-piperazin-1-yl)-propylamine (Reference Example 11) according to the method described in Example 2.

### Example 29

### Piperidine-4-carboxylic acid (2-{4-[2-(2,4-dichloro-phenoxy)-phenylsulfamoyl]-benzoylamino}-ethyl)-amide hydrochloride

### a) (2-{4-[2-(2,4-Dichloro-phenoxy)-phenylsulfamoyl]-benzoylamino}-ethyl)-carbamic acid tert-butyl ester

The title compound was prepared from 4-[2-(2,4-dichloro-phenoxy)-phenylsulfamoyl]-benzoic acid (Example 1/c) and (2-amino-ethyl)-carbamic acid *tert*-butyl ester (Aldrich) according to the method described in Example 1/d. MS (EI) 603.2 (M+Na⁺).

### b) N-(2-Amino-ethyl)-4-[2-(2,4-dichloro-phenoxy)-phenylsulfamoyl]-benzamide hydrochloride

To a stirred solution of (2-{4-[2-(2,4-dichloro-phenoxy)-phenylsulfamoyl]-benzoylamino}-ethyl)-carbamic acid *tert*-butyl ester (0.41 g, 0.71 mmol) in dichloromethane (5 mL) 9M hydrogen chloride in ethanol (0.4 mL) was added and the reaction mixture was allowed to stand at room temperature for 2 h. Then the mixture was diluted with diethyl ether (20 mL), the precipitated product was filtered, washed with diethyl ether and dried to yield 0.28 g (77 %) of the title compound as a white solid. MS (EI) 481.2 (MH⁺).

### c) 4-(2-{4-[2-(2,4-Dichloro-phenoxy)-phenylsulfamoyl]-benzoylamino}-ethylcarbamoyl)-piperidine-1-carboxylic acid tert-butyl ester

The title compound was prepared from *N*-(2-amino-ethyl)-4-[2-(2,4-dichloro-phenoxy)-phenylsulfamoyl]-benzamide and piperidine-1,4-dicarboxylic acid mono-*tert*-butyl ester (Aldrich) according to the method described in Example 1/d. MS (EI) 692.1 (MH⁺).

### d) Piperidine-4-carboxylic acid (2-{4-[2-(2,4-dichloro-phenoxy)-phenylsulfamoyl]-benzoylamino}-ethyl)-amide hydrochloride

The title compound was prepared from 4-(2-{4-[2-(2,4-dichloro-phenoxy)-phenylsulfamoyl]-benzoylamino}-ethylcarbamoyl)-piperidine-1-carboxylic acid *tert*-butyl ester according to the method described in Example 29/b. MS (EI) 592.1 (MH⁺).

### Example 30

### 4-[2-(4-Bromo-2-chloro-phenoxy)-phenylsulfamoyl]-N-(3-piperidin-4-yl-propyl)-benzamide hydrochloride

To a stirred solution of 4-[2-(4-bromo-2-chloro-phenoxy)-phenylsulfamoyl]-benzoic acid (Example 22/c) (41 mg, 0.085 mmol) in a mixture of dicloromethane (2 mL) and dimethylformamide (0.2 mL) 4-(3-amino-propyl)-piperidine-1-carboxylic acid tert-butyl ester (Reference Example 13) (24 mg, 0.1 mmol), HBTU (46 mg, 0.12 mmol) and triethylamine (60 µL, 0.4 mmol) were added. The mixture was stirred at room temperature for 24 h, then purified by column chromatography using Kieselgel 60 (0.015-0.040 mm) as adsorbent (Merck) and gradient elution starting with 100% A eluent and processing to 100% B eluent over a period of 20 minutes (eluent A: n-hexane; eluent B: ethyl acetate). The purified compound was dissolved in ethyl acetate (0.5 mL) 2.5 M hydrogen chloride in ethyl acetate (2.0 mL) was added and the mixture was stirred at room temperature for 24 h. The precipitated product was filtered, washed with diethyl ether and dried in vacuum to yield 35 mg (64 %) of the title compound. MS (EI) 608.1 (MH⁺).

### Example 31

### N-(4-[1,4']Bipiperidinyl-1'-yl-phenyl)-4-(2-phenoxy-phenylsulfamoyl)-benzamide

### a) 4-(2-Phenoxy-phenylsulfamoyl)-benzoic acid

The title compound was prepared from 2-phenoxy-phenylamine (Aldrich) according to the method described in Example 4/a. MS (EI) 370.2 (MH⁺).

### b) N-(4-[1,4']Bipiperidinyl-1'-yl-phenyl)-4-(2-phenoxy-phenylsulfamoyl)-benzamide

To a stirred solution of 4-(2-phenoxy-phenylsulfamoyl)-benzoic acid (31 mg, 0.085 mmol) in a mixture of dicloromethane (2 mL) and dimethylformamide (0.2 mL) 4-[1,4']bipiperidinyl-1'-yl-phenylamine [J. Med Chem. 46 (2003) 1803-1806] (26 mg, 0.1 mmol), HBTU (46 mg, 0.12 mmol) and triethylamine (30 µL, 0.2 mmol) were added. The mixture was stirred at room temperature for 24 h, then purified by column chromatography using Kieselgel 60 (0.015-0.040 mm) as adsorbent (Merck) and gradient elution starting with 100% A eluent and processing to a mixture of 70% A and 30% B eluent over a period of 15 minutes (eluent A: chloroform; eluent B: methanol containing 5% of ammonium hydroxide) to yield 24.8 mg (48 %) of the title compound. MS (EI) 611.3 (MH⁺).

### Example 32

### 1-{4-[2-(2,4-Dichloro-phenoxy)-phenylsulfamoyl]-benzoyl}-piperidine-4-carboxylic acid (piperidin-4-ylmethyl)-amide acetate/hydrochloride mixed salt

### a) 1-{4-[2-(2,4-Dichloro-phenoxy)-phenylsulfamoyl]-benzoyl}-piperidine-4-carboxylic acid ethyl ester

The title compound was prepared from 4-[2-(2,4-dichloro-phenoxy)-phenylsulfamoyl]-benzoic acid (Example 1/c) and ethyl isonipecotate (Aldrich) according to the method described in Example 1/d. MS (EI) 578.2 (MH⁺).

### b) 1-{4-[2-(2,4-Dichloro-phenoxy)-phenylsulfamoyl]-benzoyl}-piperidine-4-carboxylic acid

To a stirred solution of 1-{4-[2-(2,4-dichloro-phenoxy)-phenylsulfamoyl]-benzoyl}-piperidine-4-carboxylic acid ethyl ester (0.24 g, 0.42 mmol) in a mixture of tetrahydrofuran (2.5 mL), water (1.25 mL) and methanol (1.25 mL) lithium hydroxide monohydrate (0.044 g, 1.0 mmol) was added and the reaction mixture was stirred at room temperature for 2 h. The mixture was concentrated, the residue was dissolved in water, acidified with 1M hydrochloric acid, the precipitated solid was filtered off, washed with water and dried to yield 0.18 g (79 %) of the title compound. MS (EI) 550.2 NH⁺).

### c) 4-{[(1-{4-(2-4-Dichloro-phenoxy)-phenylsulfamoyl]-benzoyl}-piperidine-4-carbonyl)-amino]-methyl}-peridine-1-carboxylic acid tert-butyl ester

The title compound was prepared from 1-{4-[2-(2,4-dichloro-phenoxy)-phenylsulfamoyl]-benzoyl}-piperidine-4-carboxylic acid and 4-(aminomethyl)-Boc-piperidine (Fluka) according to the method described in Example 2. MS (EI) 746.2 (MH⁺).

### d) 1-{4-[2-(2,4-Dichloro-phenoxy)-phenylsulfamoyl]-benzoyl}-piperidine-4-carboxylic acid (piperidin-4-ylmethyl)amide acetate/hydrochloride mixed salt

The title compound was prepared from 4-{[(1-{4-[2-(2,4-dichloro-phenoxy)-phenylsulfamoyl]-benzoyl}-piperidine-4-carbonyl)-amino]-methyl}-piperidine-1-carboxylic acid *tert-*butyl ester according to the method described in Example 29/b. The crude product was submitted to reversed phase HPLC using YMC-Pack ODS-AQ type packings (produced by YMC) and acetonitrile/water/acetic acid as eluent to yield the title compound. MS (EI] 646.2 (MH⁺).

### Example 33

### 4-[2-(2,4-Dichloro-phenoxy)-phenylsulfamoyl]-N-[2-(piperidin-4-ylcarbamoyl)-ethyl]-benzamide acetate

### a) 3-{4-[2-(2,4-Dichloro-phenoxy)-phenylsulfamoyl]-benzoylamino}-propionic acid ethyl ester

The title compound was prepared from 4-[2-(2,4-dichloro-phenoxy)-phenylsulfamoyl]-benzoic acid (Example 1/c) and *β*-alanine ethyl ester hydrochloride (Aldrich) according to the method described in Example 1/d. MS (EI) 538.2 (MH⁺).

### b)3-{4-[2-(2,4-Dichloro-phenoxy)-phenylsulfamoyl]-benzoylamino}-propionic acid

The title compound was prepared from 3-{4-[2-(2,4-dichloro-phenoxy)-phenylsulfamoyl]-benzoylamino}-propionic acid ethyl ester according to the method described in Example 32/b. MS (EI) 510.1 (MH⁺).

### c) 4-(3-{4-[2-(2,4-Dichloro-phenoxy)-phenylsulfamoyl]-benzoylamino}-propionylamino)-piperidine-1-carboxylic acid tert-butyl ester

The title compound was prepared from 3-{4-(2-(2,4-dichloro-phenoxy)-phenylsulfamoyl]-benzoylamino}-propionic acid and 4-amino-piperidine-1-carboxylic acid *tert*-butyl ester (Fluka) according to the method described in Example 1/d. MS (EI) 692.2 (MH⁺).

### d) 4-[2-(2,4-Dichloro-phenoxy)-phenylsulfamoyl]-N-[2-piperidin-4-ylcarbamoyl)-ethyl]-benzamide acetate

The title compound was prepared from 4-(3-{4-[2-(2,4-dichloro-phenoxy)-phenylsulfamoyl]-benzoylamino}-propionylamino)-piperidine-1-carboxylic acid *tert*-butyl ester according to the method described in Example 29/b. The crude product was submitted to reversed phase HPLC using YMC-Pack ODS-AQ type packings (produced by YMC) and acetonitrile/water/acetic acid as eluent to yield the title compound. MS (EI) 592.2 (MH⁺).

### Example 34

### (S)-1-{4-[2-(2,4-Dichloro-phenoxy)-phenylsulfamoyl]-benzoyl]-pyrrolidine-2-carboxylic acid (piperidin-4-ylmethyl)-amide hydrochloride

### a) (S)-1-{4-[2-(2,4-Dichloro-phenoxy)-phenylsulfamoyl]-benzoyl}-pyrrolidine-2-carboxylic acid benzyl ester

The title compound was prepared from 4-[2-(2,4-dichloro-phenoxy)-phenylsulfamoyl]-benzoic acid (Example 1/c) and L-proline benzyl ester hydrochloride (Aldrich) according to the method described in Example 1/d. MS (EI) 626.3 (MH⁺).

### b) (S)-1-{4-[2-(2,4-Dichloro-phenoxy)-phenylsulfamoyl]-benzoyl}-pyrrolidine-2-carboxylic acid

The title compound was prepared from (*S*)-1-{4-[2-(2,4-dichloro-phenoxy)-phenylsulfamoyl]-benzoyl}-pyrrolidine-2-carboxylic acid benzyl ester according to the method described in Example 32/b. MS (EI) 536.2 (MH⁺).

### c) 4-{[((S)-1-{4-[2-(2,4-Dichloro-phenoxy)-phenylsulfamoyl]-benzoyl}-pyrrolidine-2-carbonyl)-amino]-methyl}-piperidine-1-carboxylic acid tert-butyl ester

The title compound was prepared from (*S*)-1-{4-[2-(2,4-dichloro-phenoxy)-phenylsulfamoyl]-benzoyl}-pyrrolidine-2-carboxylic acid and 4-aminomethyl-piperidine-1-carboxylic acid *tert-*butyl ester (Fluka) according to the method described in Example 1/d. MS (EI) 754.2 (M+Na⁺).

### d) (S)-1-{4-[2-2,4-Dichloro-phenoxy)-phenylsulfamoyl]-benzol}-pyrrolidine-2-carboxylic acid piperidin-4-ylmethyl)-amide hydrochloride

The title compound was prepared from 4-{[((*S*)-1-{4-[2-(2,4-dichloro-phenoxy)-phenylsulfamoyl]-benzoyl}-pyrrolidine-2-carbonyl)-amino]-methyl}-piperidine-1-carboxylic acid *tert*-butyl ester according to the method described in Example 29/b. MS (EI) 632.1 (MH⁺).

### Example 35

### 4-[2-(4-Bromo-phenoxy)-phenylsulfamoyl]-N-{2-[4-(6-methyl-pyridin-2-yl)-piperazin-1-yl]-ethyl}-benzamide

The title compound was prepared from 4-[2-(4-bromo-phenoxy)-phenylsulfamoyl]-benzoic acid (Example 7/a) and 2-[4-(6-methyl-pyridin-2-yl)-piperazin-1-yl]-ethylamine *[*Arzneim. Forsch.; 24 (1974) 1964-1970] according to the method described in Example 31/b. MS (EI) 651.2 (MH⁺).

### Example 36

### 4-[2-(2,4-Dichloro-phenoxy)-phenylsulfamoyl]-N-{2-[4-(6-methyl-pyridin-2-yl)-piperazin-1-yl]-ethyl}-benzamide

The title compound was prepared from 4-[2-(2,4-dichloro-phenoxy)-phenylsulfamoyl]-benzoic acid (Example 1/c) and 2-[4-(6-methyl-pyridin-2-yl)-piperazin-1-yl]-ethylamine *[*Arzneim. Forsch.; 24 (1974) 1964-1970] according to the method described in Example 8. MS (EI) 641.2 (MH⁺).

### Example 37

### 4-[2-(2,4-Dichloro-phenoxy)-phenylsulfamoyl]-N-{2-[(piperidin-4-ylmethyl)-carbamoyl]-ethyl}-benzamide acetate/hydrochloride mixed salt

### a) 4-[(3-{4-[2-(2,4-Dichloro-phenoxy)-phenylsulfamoyl]-benzoylamino}-propionylamino)-methyl]-piperidine-1-carboxylic-acid tert-butyl ester

The title compound was prepared from 3-{4-[2-(2,4-dichloro-phenoxy)-phenylsulfamoyl]-benzoylamino}-propionic acid (Example 33/b) and 4-(aminomethyl)-Boc-piperidine (Fluka) according to the method described in Example 1/d. MS (EI) 706.2 (MH⁺).

### b) 4-[2-(2,4-Dichloro-phenoxy)-phenylsulfamoyl]-N-{2-(piperidin-4-ylmethyl)-carbamoyl]-ethyl}-benzamide acetate/hydrochloride mixed salt

The title compound was prepared from 4-[(3-{4-[2-(2,4-dichloro-phenoxy)-phenylsulfamoyl]-benzoylamino}-propionylamino)-methyl]-piperidine-1-carboxylic acid *tert-*butyl ester according to the method described in Example 29/b. The crude product was submitted to reversed phase HPLC using YMC-Pack ODS-AQ type packings (produced by YMC) and acetonitrile/water/acetic acid as eluent to yield the title compound. MS (EI) 606.2 (MH⁺).

### Example 38

### N-[2-(2,4-Dichloro-phenoxy)-phenyl]-4-[4-(3-piperidin-1-yl-propyl)-piperazine-1-carbonyl]-benzenesulfonamide

The title compound was prepared from 4-[2-(2,4-dichloro-phenoxy)-phenylsulfamoyl]-benzoic acid (Example 1/c) and 1-(3-piperidin-1-yl-propyl)-piperazine (EMKA-Chemie) according to the method described in Example 31/b. MS (EI) 632.2 (MH⁺).

### Example 39

### N-[2-(2,4-Dichloro-phenoxy)-phenyl]-4-[4-(3-pyrrolidin-1-yl-pronyl)-piperazine-1-carbonyl]-benzenesulfonamide

The title compound was prepared from 4-[2-(2,4-dichloro-phenoxy)-phenylsulfamoyl]-benzoic acid (Example 1/c) and 1-(3-pyrrolidin-1-yl-propyl)-piperazine (EMKA-Chemie) according to the method described in Example 31/b. MS (EI) 618.2 (MH⁺).

### Example 40

### N-[2-(2,4-Dichloro-phenoxy)-phenyl]-4-[4-(3-dimethylamino-propyl)-piperazine-1-carbonyl]-benzenesulfonamide

The title compound was prepared from 4-[2-(2,4-dichloro-phenoxy)-phenylsulfamoyl]-benzoic acid (Example 1/c) and dimethyl(3-piperazin-1-yl-propyl)-amine (EMKA-Chemie) according to the method described in Example 31/b. MS (EI) 592.2 (MH⁺).

### Example 41

### N-[2-(2,4-Dichloro-phenoxy)-phenyl]-4-[4-(1-methyl-piperidin-3-ylmethyl)-piperazine-1-carbonyl]-benzenesulfonamide

The title compound was prepared from 4-[2-(2,4-dichloro-phenoxy)-phenylsulfamoyl]-benzoic acid (Example 1/c) and 1-(1-methyl-piperidin-3-ylmethyl)-piperazine (EMKA-Chemie) according to the method described in Example 31/b. MS (EI) 618.2 (MH⁺).

### Example 42

### N-[2-(2,4-Dichloro-phenoxyl-phenyl]-4-[4-(2-pyrrolidin-1-yl-ethyl)-piperidine-1-carbonyl]-benzenesulfonamide

The title compound was prepared from 4-[2-(2,4-dichloro-phenoxy)-phenylsulfamoyl]-benzoic acid (Example 1/c) and 4-(2-pyrrolidine-1-yl-ethyl)-piperidine (EMKA-Chemie) according to the method described in Example 31/b. MS (EI) 603.2 (MH⁺).

### Example 43

### N-[2-(2,4-Dichloro-phenoxy)-phenyl]-4-[4-(2-pyridin-2-yl-ethyl)-piperazine-1-carbonyl]-benzenesulfonamide

The title compound was prepared from 4-[2-(2,4-dichloro-phenoxy)-phenylsulfamoyl]-benzoic acid (Example 1/c) and 1-(2-pyridine-2-yl-ethyl)-piperazine (EMKA-Chemie) according to the method described in Example 31/b. MS (EI) 612.2 (MH⁺).

### Example 44

### N-[2-(2,4-Dichloro-phenoxy)-phenyl]-4-[4-(3-morpholin-4-yl-propyl)-[1,4]diazepane-1-carbonyl]-benzenesulfonamide

The title compound was prepared from 4-[2-(2,4-dichloro-phenoxy)-phenylsulfamoyl]-benzoic acid (Example 1/c) and 1-(3-morpholin-4-yl-propyl)-homopiperazine (EMKA-Chemie) according to the method described in Example 31/b. MS (EI) 648.2 (MH⁺).

### Example 45

### N-[2-(2,4-Dichloro-phenoxy)-phenyl]-4-[4-(2-dimethylamino-ethyl)-piperazine-1-carbonyl]-benzenesulfonamide

The title compound was prepared from 4-[2-(2,4-dichloro-phenoxy)-phenylsulfamoyl]-benzoic acid (Example 1/c) and dimethyl-(2-piperazin-1-yl-ethyl)-amine (EMKA-Chemie) according to the method described in Example 31/b. MS (EI) 578.2 (MH⁺).

### Example 46

### N-[2-(2,4-Dichloro-phenoxyl-phenyl]-4-[4-(2-oxo-2-piperidin-1-yl-ethyl)-piperazine-1-carbonyl]-benzenesulfonamide

The title compound was prepared from 4-[2-(2,4-dichloro-phenoxy)-phenylsulfamoyl]-benzoic acid (Example 1/c) and 2-piperazin-1-yl-piperidin-1-yl-ethanone (EMKA-Chemie) according to the method described in Example 31/b. MS (EI) 632.0 (MH⁺).

### Example 47

### N-[2-(2,4-Dichloro-phenoxy)-phenyl]4-(4-pyridin-2-ylmethyl-piperazine-1-carbonyl)-benzenesulfonamide

The title compound was prepared from 4-[2-(2,4-dichloro-phenoxy)-phenylsulfamoyl]-benzoic acid (Example 1/c) and 1-pyridin-2-ylmethyl-piperazine (EMKA-Chemie) according to the method described in Example 31/b. MS (EI) 598.2 (MH⁺).

### Example 48

### N-[2-(2,4-Dichloro-phenoxy)-phenyl]-4-(4-pyridin-3-ylmethyl-piperazine-1-carbonyl)-benzenesulfonamide

The title compound was prepared from 4-[2-(2,4-dichloro-phenoxy)-phenylsulfamoyl]-benzoic acid (Example 1/c) and 1-pyridin-3-ylmethyl-piperazine (EMKA-Chemie) according to the method described in Example 31/b. MS (EI) 598.2 (MH⁺).

### Example 49

### N-[2-(2,4-Dichloro-phenoxy)-phenyl-4-[4-(2-diethylamino-ethyl)-piperazine-1-carbonyl]-benzenesulfonamide

The title compound was prepared from 4-[2-(2,4-dichloro-phenoxy)-phenylsulfamoyl]-benzoic acid (Example 1/c) and diethyl-(2-piperazin-1-yl-ethyl)-amine (EMKA-Chemie) according to the method described in Example 31/b. MS (EI) 606.2 (MH⁺).

### Example 50

### N-[2-(2,4-Dichloro-phenoxy)-phenyl]-4-[4-(2-pyridin-4-yl-ethyl)-piperazine-1-carbonyl]-benzenesulfonamide

The title compound was prepared from 4-[2-(2,4-dichloro-phenoxy)-phenylsulfamoyl]-benzoic acid (Example 1/c) and 1-(2-pyridin-4-yl-ethyl)-piperazine (EMKA-Chemie) according to the method described in Example 31/b. MS (EI) 612.2 (MH⁺).

### Example 51

### N-[2-(2,4-Dichloro-phenoxy)-phenyl]-4-(4-pyridin-4-ylmethyl-piperazine-1-carbonyl)-benzenesulfonamide

The title compound was prepared from 4-[2-(2,4-dichloro-phenoxy)-phenylsulfamoyl]-benzoic acid (Example 1/c) and 1-pyridin-4-ylmethyl-piperazine (EMKA-Chemie) according to the method described in Example 31/b. MS (EI) 598.2 (MH⁺).

### Example 52

### 2-(4-{4-[2-(2,4-Dichloro-phenoxy)-phenylsulfamoyl]-benzoyl}-piperazin-1-yl)-N-methyl-N-phenyl-acetamide

The title compound was prepared from 4-[2-(2,4-dichloro-phenoxy)-phenylsulfamoyl]-benzoic acid (Example 1/c) and *N-*methyl-*N-*phenyl-2-piperazin-1-yl-acetamide (EMKA-Chemie) according to the method described in Example 31/b. MS (EI) 654.2 (MH⁺).

### Example 53

### 2-(4-{4-[2-(2,4-Dichloro-phenoxy)-phenylsulfamoyl]-benzoyl}-piperazin-1-yl)-N-pyridin-3-yl-acetamide

The title compound was prepared from 4-[2-(2,4-dichloro-phenoxy)-phenylsulfamoyl]-benzoic acid (Example 1/c) and 2-piperazin-1-yl-*N*-pyridin-2-yl-acetamide (EMKA-Chemie) according to the method described in Example 31/b. MS (EI) 641.2 (MH⁺).

### Example 54

### 4-[2-(2,4-Dichloro-phenoxy)-phenylsulfamoyl]-N-{2-[4-(4-methyl-pyridin-2-yl)-piperazin-1-yl]-ethyl}-benzamide

The title compound was prepared from 4-[2-(2,4-dichloro-phenoxy)-phenylsulfamoyl]-benzoic acid (Example 1/c) and 2-[4-(4-methyl-pyridin-2-yl)-piperazin-1-yl]-ethylamine *[*Arzneim. Forsch.; 24 (1974) 1964-1970] according to the method described in Example 8. MS (EI) 641.2 (MH⁺).

### Example 55

### N-[2-(2,4-Dichloro-phenoxy)-phenyl]-4-[4-(2-oxo-2-pyrrolidin-1-yl-ethyl)piperazine-1-carbonyl]-benzenesulfonamide

The title compound was prepared from 4-[2-(2,4-dichloro-phenoxy)-phenylsulfamoyl]-benzoic acid (Example 1/c) and 2-piperazin-1-yl-pyrrolidin-1-yl-ethanone (EMKA-Chemie) according to the method described in Example 31/b. MS (EI) 618.2 (MH⁺).

### Example 56

### 4-[2-(2,4-Dichloro-phenoxy)-phenylsulfamoyl]-N-[2-(2-piperidin-4-yl-ethylcarbamoyl)-ethyl]-benzamide hydrochloride

### a) 4-[2-(3-{4-[2-(2,4-Dichloro-phenoxy)-phenylsulfamoyl]-benzoylamino]-propionylamino)-ethyl]-piperidine-1-carboxylic acid tert-butyl ester

The title compound was prepared from 3-{4-[2-(2,4-dichloro-phenoxy)-phenylsulfamoyl]-benzoylamino}-propionic acid (Example 33/b) and 4-(2-amino-ethyl)-piperidine-1-carboxylic acid *tert-*butyl ester [Bioorg. Med. Chem. Lett.; 13 (2003) 2167-2172.] according to the method described in Example 1/d. MS (EI) 742.1 (M+Na⁺).

### b) 4-[2-(2,4-Dichloro-phenoxy)-phenylsulfamoyl]-N-[2-(2-piperidin-4-yl-ethylcarbamoyl)-ethyl]benzamide hydrochloride

The title compound was prepared from 4-[2-(3-{4-[2-(2,4-dichloro-phenoxy)-phenylsulfamoyl]-benzoylamino}-propionylamino)-ethyl]-piperidine-1-carboxylic acid *tert-*butyl ester according to the method described in Example 29/b. MS (EI) 620.1 (MH⁺).

### Example 57

### 4-[2-(4-Bromo-phenoxy)-phenylsulfamoyl]-N-(3-piperidin-4-yl-propyl)-benzamide trifluoracetate

### a) 4-(3-{4-[2-(4-Bromo-phenoxy)-phenylsulfamoyl]-benzoylamino}-propyl)-piperidine-1-carboxylic acid tert-butyl ester

The title compound was prepared from 4-[2-(4-bromo-phenoxy)-phenylsulfamoyl]-benzoic acid (Example 7/a) and 4-(3-amino-propyl)-piperidine-1-carboxylic acid *tert*-butyl ester (Reference Example 14) according to the method described in Example 1/d. MS (EI) 696.1 (M+Na⁺).

### b) 4-[2-(4-Bromo-phenoxy)-phenylsulfamoyl]-N-(3-piperidin-4-yl-propyl)-benzamide trifluoracetate

The title compound was prepared from 4-(3-{4-[2-(4-bromo-phenoxy)-phenylsulfamoyl]-benzoylamino}-propyl)-piperidine-1-carboxylic acid *tert*-butyl ester according to the method described in Example 11/b. MS (EI) 573.2 (MH⁺).

### Example 58

### N-(4-[1,4']Bipiperidinyl-1'-yl-phenyl)-4-[2-(4-bromo-phenoxy)-5-fluoro-phenylsulfamoyl]-benzamide

### a) 4-[2-(4-Bromo-phenoxy)-5-fluoro-phenylsulfamoyl]-benzoic acid

Under an atmosphere of argon to an ice cooled solution of 2-(4-bromo-phenoxy)-5-fluoro-phenylamine [Yakugaku Zasshi; 87 (1967) 591, 594; Chem.Abstr.; 67 (1967) 73282] (0.43 g, 1.52 mmol) in dry pyridine (10 mL) 4-chlorosulfonyl benzoic acid (0.34 g, 1.52 mmol) was added portion-wise. The reaction mixture was stirred at room temperature overnight. The mixture was concentrated *in vacuo,* the residue was treated with 1N hydrochloric acid (15 mL), and extracted with ethyl acetate (3x20 mL). The combined organic layers were washed with 1N hydrochloric acid, water and brine, dried over sodium sulfate, filtered and concentrated *in vacuo.* The residue was triturated with diethyl ether, filtered, washed with diethyl ether and dried to yield 0.31 g (43 %) of the title compound as a light pink solid. MS (EI) 467.9 (MH⁺).

### b) N-(4-[1,4']Bipiperidinyl-1'-yl-phenyl)-4-[2-(4-bromo-phenoxy)-5-fluoro-phenylsulfamoyl]-benzamide

The title compound was prepared from 4-[2-(4-bromo-phenoxy)-5-fluoro-phenylsulfamoyl]-benzoic acid and 4-[1,4']bipiperidinyl-1'-yl-phenylamine [J. Med. Chem. 46 (2003) 1803-1806] according to the method described in Example 31/b. MS (EI) 707.7 (MH⁺).

### Example 59

### N-[2-(3,4-Dichloro-phenoxy)-phenyl]-4-(4-pyridin-3-ylmethyl-piperazine-1-carbonyl)-benzenesulfonamide

The title compound was prepared from 4-[2-(3,4-dichloro-phenoxy)-phenylsulfamoyl]-benzoic acid (Example 4/a) and 1-pyridin-3-ylmethyl-piperazine (EMKA-Chemie) according to the method described in Example 31/b. MS (EI) 598.1 (MH⁺).

### Example 60

### N-[2-(3,4-Dichloro-phenoxy)-phenyl]-4-[4-(2-pyridin-4-yl-ethyl)-piperazine-1-carbonyl]-benzenesulfonamide

The title compound was prepared from 4-[2-(3,4-dichloro-phenoxy)-phenylsulfamoyl]-benzoic acid (Example 4/a) and 1-(2-pyridin-4-yl-ethyl)-piperazine (EMKA-Chemie) according to the method described in Example 31/b. MS (EI) 612.0 (MH⁺).

### Example 61

### N-[2-(3,4-Dichloro-phenoxyl-phenyl]-4-[4-(2-pyrrolidin-1-yl-ethyl)-piperazine-1-carbonyl]-benzenesulfonamide

The title compound was prepared from 4-[2-(3,4-dichloro-phenoxy)-phenylsulfamoyl]-benzoic acid (Example 4/a) and 1-(2-pyrrolidin-1-yl-ethyl)-piperazine (EMKA-Chemie) according to the method described in Example 31/b. MS (EI) 604.2 (MH⁺).

### Example 62

### N-(3-[1,4']Bipiperidinyl-1'-yl-3-oxo-propyl)-4-[2-(2,4-dichloro-phenoxyl)-phenylsulfamoyl]-benzamide trifluoroacetate

The title compound was prepared from 3-{4-[2-(2,4-dichloro-phenoxy)-phenylsulfamoyl]-benzoylamino}-propionic acid (Example 33/b) according to the method described in Example 8. The crude product was submitted to reversed phase HPLC using YMC-Pack ODS-AQ type packings (produced by YMC) and acetonitrile/water/trifluoroacetic acid as eluent to yield the title compound.

### Example 63

### N-(4-[1,4']Bipiperidinyl-1'-yl-phenyl)-4-[2-(3,4-dichloro-phenoxy)-phenylsulfamoyl]-benzamide

The title compound was prepared from 4-[2-(3,4-dichloro-phenoxy)-phenylsulfamoyl]-benzoic acid (Example 4/a) and 4-[1,4']bipiperidinyl-1'-yl-phenylamine [J. Med. Chem. 46 (2003) 1803-1806] according to the method described in Example 31/b. MS (EI) 680.2 (MH⁺).

### Example 64

### trans-4-[2-(3,4-Dichloro-phenoxy)-phenylsulfamoyl]-N-[4-(2-pyrrolidin-1-yl-ethyl)-cyclohexyl]-benzamide

The title compound was prepared from 4-[2-(3,4-dichloro-phenoxy)-phenylsulfamoyl]-benzoic acid (Example 4/a) and trans-4-(2-pyrrolidin-1-yl-ethyl)-cyclohexylamine dihydrochloride (Reference Example 4) according to the method described in Example 31/b. MS (EI) 617.2 (MH⁺).

### Example 65

### 4-[2-(3,4-Dichloro-phenoxy)-phenulsulfamoyl]-N-[2-(4-pyridin-2-yl-piperazin-1-yl)-ethyl]-benzamide

The title compound was prepared from 4-[2-(3,4-dichloro-phenoxy)-phenylsulfamoyl]-benzoic acid (Example 4/a) and 2-(4-pyridin-2-yl-piperazin-1-yl)-ethylamine tetrahydrochloride (Reference Example 6) according to the method described in Example 31/b. MS (EI) 627.2 (MH⁺).

### Example 66

### 4-[2-(3,4-Dichloro-phenoxy)-phenylsulfamoyl]-N-[3-(4-pyridin-4-yl-piperazin-1-yl)-propyl]-benzamide

The title compound was prepared from 4-[2-(3,4-dichloro-phenoxy)-phenylsulfamoyl]-benzoic acid (Example 4/a) and 2-(4-pyridin-4-yl-piperazin-1-yl)-propylamine (Reference Example 11) according to the method described in Example 31/b. MS (EI) 641.2 (MH⁺).

### Example 67

### 2-(4-{4-[2-(3,4-Dichloro-phenoxy)-phenylsulfamoyl]-benzoyl}-piperazin-1-yl)-N-pyridin-2-yl-acetamide

The title compound was prepared from 4-[2-(3,4-dichloro-phenoxy)-phenylsulfamoyl]-benzoic acid (Example 4/a) and 2-piperazin-1-yl-*N*-pyridin-2-yl-acetamide (EMKA-Chemie) according to the method described in Example 31/b. MS (EI) 641.2 (MH⁺).

### Example 68

### 2-(4-{4-[2-(3,4-Dichloro-phenoxy)-phenylsulfamoyl]-benzoyl}-piperazin-1-yl)-N-pyridin-3-yl-acetamide

The title compound was prepared from 4-[2-(3,4-dichloro-phenoxy)-phenylsulfamoyl]-benzoic acid (Example 4/a) and 2-piperazin-1-yl-*N*-pyridin-3-yl-acetamide (EMKA-Chemie) according to the method described in Example 31/b. MS (EI) 641.2 (MH⁺).

### Example 69

### N-[2-(3,4-Dichloro-phenoxy)-phenyl]-4-(4-pyridin-4-ylmethyl-piperazine-1-carbonyl)-benzenesulfonamide

The title compound was prepared from 4-[2-(3,4-dichloro-phenoxy)-phenylsulfamoyl]-benzoic acid (Example 4/a) and 1-pyridin-4-ylmethyl-piperazine (EMKA-Chemie) according to the method described in Example 31/b. MS (EI) 598.2 (MH⁺).

### Example 70

### 4-[2-(3,4-Dichloro-phenoxy)-phenylsulfamoyl]-N-{2-[4-(6-methyl-pyridin-2-yl)-piperazin-1-yl]-ethyl}-benzamide

The title compound was prepared from 4-[2-(3,4-dichloro-phenoxy)-phenylsulfamoyl]-benzoic acid (Example 4/a) and 2-[4-(6-methyl-pyridin-2-yl)-piperazin-1-yl]-ethylamine [Arzneim. Forsch.; 24 (1974) 1964-1970] according to the method described in Example 31/b. MS (EI) 641.2 (MH⁺).

### Example 71

### 4-[2-(3,4-Dichloro-phenoxy)-phenylsulfamoyl]-N-{2-[4-(4-methyl-pyridin-2-yl)-piperazin-1-yl]-ethyl}-benzamide

The title compound was prepared from 4-[2-(3,4-dichloro-phenoxy)-phenylsulfamoyl]-benzoic acid (Example 4/a) and 2-[4-(4-methyl-pyridin-2-yl)piperazin-1-yl]-ethylamine *[*Arzneim. Forsch.; 24 (1974) 1964-1970] according to the method described in Example 31/b. MS (EI) 641.2 (MH⁺).

### Example 72

### 4-[2-(3,4-Dichloro-phenoxy)-phenylsulfamoyl]-N-[2-(4-pyrimidin-2-yl-piperazin-1-yl)-ethyl]-benzamide

The title compound was prepared from 4-[2-(3,4-dichloro-phenoxy)-phenylsulfamoyl]-benzoic acid (Example 4/a) and 2-(4-pyrimidin-2-yl-piperazin-1-yl)-ethylamine [Bioorg. Med. Chem.; 12 (2004) 3965-3970] according to the method described in Example 31/b. MS (EI) 628.2 (MH⁺).

### Example 73

### N-[2-(3,4-Dichloro-phenoxy)-phenyl]-4-[4-(3-morpholin-4-yl-propyl)-[1,4]diazepane-1-carbonyl]-benzenesulfonamide

The title compound was prepared from 4-[2-(3,4-dichloro-phenoxy)-phenylsulfamoyl]-benzoic acid (Example 4/a) and 1-(3-morpholin-4-yl-propyl)-homopiperazine (EMKA-Chemie) according to the method described in Example 31/b. MS (EI) 648.2 (MH⁺).

### Example 74

### N-[2-(3,4-Dichloro-phenoxy)-phenyl]-4-[4-(3-morpholin-4-yl-propyl)-piperazine-1-carbonyl]-benzenesulfonamide

The title compound was prepared from 4-[2-(3,4-dichloro-phenoxy)-phenylsulfamoyl]-benzoic acid (Example 4/a) and 4-(3-piperazin-1-yl-propyl)-morpholine (EMKA-Chemie) according to the method described in Example 31/b. MS (EI) 634.2 (MH⁺).

### Example 75

### N-[2-(3,4-Dichloro-phenoxy)-phenyl]-4-[4-(2-dimethylamino-ethyl)-piperazine-1-carbonyl]-benzenesulfonamide

The title compound was prepared from 4-[2-(3,4-dichloro-phenoxy)-phenylsulfamoyl]-benzoic acid (Example 4/a) and dimethyl-(2-piperazin-1-yl-ethyl)-amine (EMKA-Chemie) according to the method described in Example 31/b. MS (EI) 578.2 (MH⁺).

### Example 76

### 4-[2-(2,4-Dichloro-phenoxy)-phenylsulfamoyl]-N-(3-piperidin-1-yl-propyl-benzamide

The title compound was prepared from 4-[2-(2,4-dichloro-phenoxy)-phenylsulfamoyl]-benzoic acid (Example 1/c) and 3-piperidin-1-yl-propylamine (EMKA-Chemie) according to the method described in Example 31/b. MS (EI) 563.1 (MH⁺).

### Example 77

### N-[2-(2,4-Dichloro-phenoxy)-phenyl]-4-[4-(2-pyrrolidin-1-yl-ethyl)-piperazine-1-carbonyl]-benzenesulfonamide

The title compound was prepared from 4-[2-(2,4-dichloro-phenoxy)-phenylsulfamoyl]-benzoic acid (Example 1/c) and 1-(2-pyrrolidin-1-yl-ethyl)-piperazine (EMKA-Chemie) according to the method described in Example 31/b. MS (EI) 604.2 (MH⁺).

### Example 78

### N-[2-(2,4-Dichloro-phenoxy)-phenyl]-4-[4-(3-morpholin-4-yl-propyl)-piperazine-1-carbonyl]-benzenesulfonamide

The title compound was prepared from 4-[2-(2,4-dichloro-phenoxy)-phenylsulfamoyl]-benzoic acid (Example 1/c) and 4-(3-piperazin-1-yl-propyl)-morpholine (EMKA-Chemie) according to the method described in Example 31/b. MS (EI) 633.3 (NM⁺).

### Example 79

### N-[2-(2,4-Dichloro-phenoxy)-phenyl]-4-[4-(2-piperidin-1-yl-ethyl)-piperazine-1-carbonyl]-benzenesulfonamide

The title compound was prepared from 4-[2-(2,4-dichloro-phenoxy)-phenylsulfamoyl]-benzoic acid (Example 1/c) and 1-(2-piperidin-1-yl-ethyl)-piperazine (EMKA-Chemie) according to the method described in Example 31/b. MS (EI) 618.2 (MH⁺).

### Example 80

### N-[2-(2,4-Dichloro-phenoxy)-phenyl]-4-[4-(2-morpholin-4-yl-ethyl)-piperazine-1-carbonyl]-benzenesulfonamide

The title compound was prepared from 4-[2-(2,4-dichloro-phenoxy)-phenylsulfamoyl]-benzoic acid (Example 1/c) and 4-(2-piperazin-1-yl-ethyl)-morpholine (EMKA-Chemie) according to the method described in Example 31/b. MS (EI) 620.2 (MH⁺).

### Example 81

### N-[2-(2,4-Dichloro-phenoxy)-phenyl]-4-[4-(3-pyrrolidin-1-yl-propyl)-[1,4]diazepane-1-carbonyl]-benzenesulfonamide

The title compound was prepared from 4-[2-(2,4-dichloro-phenoxy)-phenylsulfamoyl]-benzoic acid (Example 1/c) and 1-(3-pyrrolidin-1-yl-propyl)-homopiperazine (EMKA-Chemie) according to the method described in Example 31/b. MS (EI) 632.1 (MH⁺).

### Example 82

### N-[2-(2,4-Dichloro-phenoxy)-phenyl]-4-[4-(2-morpholin-4-yl-2-oxo-ethyl)-piperazine-1-carbonyl]-benzenesulfonamide

The title compound was prepared from 4-[2-(2,4-dichloro-phenoxy)-phenylsulfamoyl]-benzoic acid (Example 1/c) and 1-morpholin-4-yl-2-piperazin-1-yl-ethanone (EMKA-Chemie) according to the method described in Example 31/b. MS (EI) 634.1 (MH⁺).

### Example 83

### 4-[2-(3,4-Dichloro-phenoxy)-phenylsulfamoyl]-N-(3-piperidin-1-yl-1-propyl)-benzamide

The title compound was prepared from 4-[2-(3,4-dichloro-phenoxy)-phenylsulfamoyl]-benzoic acid (Example 4/a) and 3-piperidin-1-yl-propylamine (EMKA-Chemie) according to the method described in Example 31/b. MS (EI) 563.3 (MH⁺).

### Example 84

### N-[2-(3,4-Dichloro-phenoxy)-phenyl]-4-[4-(2-pyridin-2-yl-ethyl)-piperazine-1-carbonyl]-benzenesulfonamide

The title compound was prepared from 4-[2-(3,4-dichloro-phenoxy)-phenylsulfamoyl]-benzoic acid (Example 4/a) and 1-(2-pyridin-2-yl-ethyl)-piperazine (EMKA-Chemie) according to the method described in Example 31/b. MS (EI) 612.2 (MH⁺).

### Example 85

### N-[2-(3,4-Dichloro-phenoxy)-phenyl]4-(4-pyridin-2-ylmethyl-piperazine-1-carbonyl)-benzenesulfonamide

The title compound was prepared from 4-[2-(3,4-dichloro-phenoxy)-phenylsulfamoyl]-benzoic acid (Example 4/a) and 1-pyridin-2-ylmethyl-piperazine (EMKA-Chemie) according to the method described in Example 31/b. MS (EI) 598.1 (MH⁺).

### Example 86

### N-[2-(3,4-Dichloro-phenoxy)-phenyl]-4-[4-(2-diethylamino-ethyl)-piperazine-1-carbonyl]-benzenesulfonamide

The title compound was prepared from 4-[2-(3,4-dichloro-phenoxy)-phenylsulfamoyl]-benzoic acid (Example 4/a) and diethyl-(2-piperazin-1-yl-ethyl)-amine (EMKA-Chemie) according to the method described in Example 31/b. MS (EI) 606.2 (MH⁺).

### Example 87

### N-[2-(3,4-Dichloro-phenoxy)-phenyl]-4-[4-(3-piperidin-1-yl-propyl)-piperazine-1-carbonyl]-benzenesulfonamide.

The title compound was prepared from 4-[2-(3,4-dichloro-phenoxy)-phenylsulfamoyl]-benzoic acid (Example 4/a) and 1-(3-piperidin-1-yl-propyl)-piperazine (EMKA-Chemie) according to the method described in Example 31/b. MS (EI) 633.2 (MH⁺).

### Example 88

### N-[2-(3,4-Dichloro-phenoxy)-phenyl]-4-[4-(3-pyrrolidin-1-yl-propyl)-piperazine-1-carbonyl]-benzenesulfonamide

The title compound was prepared from 4-[2-(3,4-dichloro-phenoxy)-phenylsulfamoyl]-benzoic acid (Example 4/a) and 1-(3-pyrrolidin-1-yl-propyl)-piperazine (EMKA-Chemie) according to the method described in Example 31/b. MS (EI) 619.1 (MH⁺).

### Example 89

### N-[2-(3,4-Dichloro-phenoxy)-phenyl]-4-[4-(3-dimethylamino-propyl)-piperazine-1-carbonyl]-benzenesulfonamide

The title compound was prepared from 4-[2-(3,4-dichloro-phenoxy)-phenylsulfamoyl]-benzoic acid (Example 4/a) and dimethyl-(3-piperazin-1-yl-propyl)-amine (EMKA-Chemie) according to the method described in Example 31/b. MS (EI) 593.1 (MH⁺).

### Example 90

### N-[2-(3,4-Dichloro-phenoxy)-phenyl]-4-[4-(1-methyl-piperidin-3-ylmethyl)-piperazine-1-carbonyl]-benzenesulfonamide

The title compound was prepared from 4-[2-(3,4-dichloro-phenoxy)-phenylsulfamoyl]-benzoic acid (Example 4/a) and 1-(1-methyl-piperidin-3-ylmethyl)-piperazine (EMKA-Chemie) according to the method described in Example 31/b. MS (EI) 618.0 (MH⁺).

### Example 91

### N-[2-(3,4-Dichloro-phenoxy)-phenyl]-4-[4-(2-pyrrolidin-1-yl-ethyl)-piperidine-1-carbonyl]-benzenesulfonamide

The title compound was prepared from 4-[2-(3,4-dichloro-phenoxy)-phenylsulfamoyl]-benzoic acid (Example 4/a) and 4-(2-pyrrolidin-1-yl-ethyl)-piperidine (EMKA-Chemie) according to the method described in Example 31/b. MS (EI) 603.0 (MH⁺).

### Example 92

### N-[2-(3,4-Dichloro-phenoxy)-phenyl]-4-[4-(2-piperidin-1-yl-ethyl)piperazine-1-carbonyl]-benzenesulfonamide

The title compound was prepared from 4-[2-(3,4-dichloro-phenoxy)-phenylsulfamoyl]-benzoic acid (Example 4/a) and 1-(2-piperidin-1-yl-ethyl)-piperazine (EMKA-Chemie) according to the method described in Example 31/b. MS (EI) 618.0 (MH⁺).

### Example 93

### N-[2-(3,4-Dichloro-phenoxy)-phenyl]-4-[4-(2-oxo-2-pyrrolidin-1-yl-ethyl)-piperazine-1-carbonyl]-benzenesulfonamide

The title compound was prepared from 4-[2-(3,4-dichloro-phenoxy)-phenylsulfamoyl]-benzoic acid (Example 4/a) and 2-piperazin-1-yl-pyrrolidin-1-yl-ethanone (EMKA-Chemie) according to the method described in Example 31/b. MS (EI) 618.0 (MH⁺).

### Example 94

### N-[2-(3,4-Dichloro-phenoxy)-phenyl]-4-[4-(2-morpholin-4-yl-ethyl)-piperazine-1-carbonyl]-benzenesulfonamide

The title compound was prepared from 4-[2-(3,4-dichloro-phenoxy)-phenylsulfamoyl]-benzoic acid (Example 4/a) and 4-(2-piperazin-1-yl-ethyl)-morpholine (EMKA-Chemie) according to the method described in Example 31/b. MS (EI) 620.0 (MH⁺).

### Example 95

### N-[2-(3,4-Dichloro-phenoxy)-phenyl]-4-[4-(2-oxo-2-piperidin-1-yl-ethyl)-piperazine-1-carbonyl-benzenesulfonamide

The title compound was prepared from 4-[2-(3,4-dichloro-phenoxy)-phenylsulfamoyl]-benzoic acid (Example 4/a) and 2-piperazin-1-yl-1-piperidin-1-yl-ethanone (EMKA-Chemie) according to the method described in Example 31/b. MS (EI) 632.0 (MH⁺).

### Example 96

### 2-(4-{4-[2-(3,4-Dichloro-phenoxy)-phenylsulfamoyl]-benzoyl}-piperazin-1-yl)-N-methyl-N-phenyl-acetamide

The title compound was prepared from 4-[2-(3,4-dichloro-phenoxy)-phenylsulfamoyl]-benzoic acid (Example 4/a) and *N*-methyl-*N-*phenyl-2-piperazin-1-yl-acetamide (EMKA-Chemie) according to the method described in Example 31/b. MS (EI) 654.0 (MH⁺).

### Example 97

### N-[2-(3,4-Dichloro-phenoxy)-phenyl]-4-[4-(2-morpholin-4-yl-2-oxo-ethyl)-piperazine-1-carbonyl]-benzenesulfonamide

The title compound was prepared from 4-[2-(3,4-dichloro-phenoxy)-phenylsulfamoyl]-benzoic acid (Example 4/a) and 1-morpholin-4-yl-2-piperazin-1-yl-ethanone (EMKA-Chemie) according to the method described in Example 31/b. MS (EI) 634.0 (MH⁺).

### Example 98

### 4-[2-(4-Bromo-phenoxy)-phenylsulfamoyl]-N-{2-[4-(4-methyl-pyridin-2-yl)-piperazin-1-yl]-ethyl}-benzamide

The title compound was prepared from 4-[2-(4-bromo-phenoxy)-phenylsulfamoyl]-benzoic acid (Example 7/a) and 2-[4-(4-methyl-pyridin-2-yl)-piperazin-1-yl]-ethylamine [Arzneim. Forsch.; 24 (1974) 1964-1970] according to the method described in Example 31/b. MS (EI) 651.2 (MH⁺).

### Example 99

### 4-[2-(4-Bromo-phenoxy)-phenylsulfamoyl]-N-[2-(4-pyridin-2-ylmethyl-piperazin-1-yl)-ethyl]-benzamide

The title compound was prepared from 4-[2-(4-bromo-phenoxy)-phenylsulfamoyl]-benzoic acid (Example 7/a) and 2-(4-pyridin-2-ylmethyl-piperazin-1-yl)-ethylamine (Reference Example 15) according to the method described in Example 31/b. MS (EI) 651.2 (MH⁺).

### Example 100

### 4-[2-(4-Bromo-phenoxy)-phenylsulfamoyl]-N-(2-(4-pyrimidin-2-yl-piperazin-1-yl)-ethyl]-benzamide

The title compound was prepared from 4-[2-(4-bromo-phenoxy)-phenylsulfamoyl]-benzoic acid (Example 7/a) and 2-(4-pyrimidin-2-yl-piperazin-1-yl)-ethylamine [Bioorg. Med. Chem.; 12 (2004) 3965-3970] according to the method described in Example 31/b. MS (EI) 638.2 (MH⁺).

### Example 101

### 4-[2-(4-Bromo-phenoxy)-phenylsulfamoyl]-N-(4-piperidin-4-yl-butyl-benzamide hydrochloride

The title compound was prepared from 4-[2-(4-bromo-phenoxy)-phenylsulfamoyl]-benzoic acid (Example 7/a) and 4-(4-amino-butyl)-piperidine-1-carboxylic acid tert-butyl ester (Reference Example 12) according to the method described in Example 30. MS (EI) 587.2 (MH⁺).

### Example 102

### N-(3-[1,4']Bipiperidinyl-1'-yl-propyl)-4-[2-(4-bromo-phenoxy)-5-fluoro-phenylsulfamoyl]-benzamide

The title compound was prepared from 4-[2-(4-bromo-phenoxy)-5-fluoro-phenylsulfamoyl]-benzoic acid (Example 58/a) and 3-[1,4']bipiperidinyl-1'-yl-propylamine trihydrochloride (Reference Example 3) according to the method described in Example 31/b. MS (EI) 674.2 (MH⁺).

### Example 103

### trans-4-[2-(4-Bromo-phenoxy)-5-fluoro-phenylsulfamoyl]-N-[4-(2-pyrrolidin-1-yl-ethyl)-cyclohexyl]-benzamide

The title compound was prepared from 4-[2-(4-bromo-phenoxy)-5-fluoro-phenylsulfamoyl]-benzoic acid (Example 58/a) and trans-4-(2-pyrrolidin-1-yl-ethyl)-cyclohexylamine dihydrochloride (Reference Example 4) according to the method described in Example 31/b. MS (EI) 645.2 (MH⁺).

### Example 104

### 4-[2-(4-Bromo-phenoxy)-5-fluoro-phenylsulfamoyl]-N-[2-(4-pyridin-2-yl-piperazin-1-yl)-ethyl]-benzamide

The title compound was prepared from 4-[2-(4-bromo-phenoxy)-5-fluoro-phenylsulfamoyl]-benzoic acid (Example 58/a) and 2-(4-pyridin-2-yl-piperazin-1-yl)-ethylamine tetrahydrochloride (Reference Example 6) according to the method described in Example 31/b. MS (EI) 654.2 (MH⁺).

### Example 105

### 4-[2-(4-Bromo-phenoxy)-5-fluoro-phenylsulfamoyl]-N-[3-(4-pyridin-4-yl-piperazin-1-yl)-pronyl]-benzamide

The title compound was prepared from 4-[2-(4-bromo-phenoxy)-5-fluoro-phenylsulfamoyl]-benzoic acid (Example 58/a) and 2-(4-pyridin-4-yl-piperazin-1-yl)-propylamine (Reference Example 11) according to the method described in Example 31/b. MS (EI) 669.2 (MH⁺).

### Example 106

### 4-[2-(4-Bromo-phenoxy)-5-fluoro-phenylsulfamoyl]-N-{2-[4-(6-methyl-pyridin-2-yl)-piperazin-1-yl]-ethyl}-benzamide

The title compound was prepared from 4-[2-(4-bromo-phenoxy)-5-fluoro-phenylsulfamoyl]-benzoic acid (Example 58/a) and 2-[4-(6-methyl-pyridin-2-yl)-piperazin-1-yl]-ethylamine [Arzneim. Forsch.; 24 (1974) 1964-1970] according to the method described in Example 31/b. MS (EI) 669.2 (MH⁺).

### Example 107

### 4-[2-(4-Bromo-phenoxy)-5-fluoro-phenylsulfamoyl]-N-{2-[4-(4-methyl-pyridin-2-yl)-piperazin-1-yl]-ethyl}-benzamide

The title compound was prepared from 4-[2-(4-bromo-phenoxy)-5-fluoro-phenylsulfamoyl]-benzoic acid (Example 58/a) and 2-[4-(4-methyl-pyridin-2-yl)-piperazin-1-yl]-ethylamine [Arzneim. Forsch.; 24 (1974) 1964-1970] according to the method described in Example 31/b. MS (EI) 669.2 (MH⁺).

### Example 108

### 4-[2-(4-Bromo-phenoxy)-5-fluoro-phenylsulfamoyl]-N-[2-(4-pyridin-2-ylmethyl-piperazin-1-yl)-ethyl]-benzamide

The title compound was prepared from 4-[2-(4-bromo-phenoxy)-5-fluoro-phenylsulfamoyl]-benzoic acid (Example 58/a) and 2-(4-pyridin-2-ylmethyl-piperazin-1-yl)-ethylamine (Reference Example 15) according to the method described in Example 31/b. MS (EI) 669.2 (MH⁺).

### Example 109

### 4-[2-(4-Bromo-phenoxy)-5-fluoro-phenylsulfamoyl]-N-[2-(4-pyrimidin-2-yl-piperazin-1-yl)-ethyl]-benzamide

The title compound was prepared from 4-[2-(4-bromo-phenoxy)-5-fluoro-phenylsulfamoyl]-benzoic acid (Example 58/a) and 2-(4-pyrimidin-2-yl-piperazin-1-yl)-ethylamine [Bioorg. Med. Chem.; 12 (2004) 3965-3970] according to the method described in Example 31/b. MS (EI) 656.2 (MH⁺).

### Example 110

### 4-[2-(4-Bromo-phenoxy)-5-fluoro-phenylsulfamoyl]-N-(4-piperidin-4-yl-butyl)-benzamide hydrochloride

The title compound was prepared from 4-[2-(4-bromo-phenoxy)-5-fluoro-phenylsulfamoyl]-benzoic acid (Example 58/a) and 4-(4-amino-butyl)-piperidine-1-carboxylic acid *tert*-butyl ester (Reference Example 12) according to the method described in Example 30. MS (EI) 605.2 (MH⁺).

### Example 111

### N-(3-[1,4]Bipiperidinyl-1'-yl-propyl)-4-[2-(4-bromo-phenoxy)-phenylsulfamoyl]-benzamide

The title compound was prepared from 4-[2-(4-bromo-phenoxy)-phenylsulfamoyl]-benzoic acid (Example 7/a) and 3-[1,4']bipiperidinyl-1'-yl)-propylamine trihydrochloride (Reference Example 3) according to the method described in Example 31/b. MS (EI) 656.2 (MH⁺).

### Example 112

### trans-4-[2-(4-Bromo-phenoxy)-phenylsulfamoyl]-N-[4-(2-pyrrolidin-1-yl-ethyl)-cyclohexyl]-benzamide

The title compound was prepared from 4-[2-(4-bromo-phenoxy)-phenylsulfamoyl]-benzoic acid (Example 7/a) and trans-4-(2-pyrrolidin-1-yl-ethyl)-cyclohexylamine dihydrochloride (Reference Example 4) according to the method described in Example 31/b. MS (EI) 627.2 (MH⁺).

### Example 113

### 4-[2-(4-Bromo-phenoxy)-phenlsulfamoyl]-N-[2-(4-pyridin-2-yl-piperazin-1-yl)-ethyl]-benzamide

The title compound was prepared from 4-[2-(4-bromo-phenoxy)-phenylsulfamoyl]-benzoic acid (Example 7/a) and 2-(4-pyridin-2-yl-piperazin-1-yl)-ethylamine tetrahydrochloride (Reference Example 6) according to the method described in Example 31/b. MS (EI) 637.2 (MH⁺).

### Example 114

### 4-[2-(4-Bromo-phenoxyl-phenylsulfamoyl]-N-[3-(4-pyridin-4-yl-piperazin-1-yl)-propyl]l-benzamide

The title compound was prepared from 4-[2-(4-bromo-phenoxy)-phenylsulfamoyl]-benzoic acid (Example 7/a) and 2-(4-pyridin-4-yl-piperazin-1-yl)-propylamine (Reference Example 11) according to the method described in Example 31/b. MS (EI) 651.2 (MH⁺).

### Example 115

### N-[2-(3,4-Dichloro-phenoxy)-phenyl]-4-[4-(3-pyrrolidin-1-yl-propyl)-[1,4]diazepane-1-carbonyl]-benzenesulfonamide

The title compound was prepared from 4-[2-(3,4-dichloro-phenoxy)-phenylsulfamoyl]-benzoic acid (Example 4/a) and 1-(3-pyrrolidin-1-yl-propyl)-homopiperazine (EMKA-Chemie) according to the method described in Example 31/b. MS (EI) 633.1 (MH⁺).

### Example 116

### N-(3-[1,4']Bipiperidinyl-1'-yl-propyl)4-[2-(2-chloro-4-fluoro-phenoxy)-phenylsulfamoyl]-benzamide

### a) 2-Chloro-4-fluoro-1-(2-nitro-phenoxy)-benzene

The title compound was prepared from 2-chloro-4-fluoro-phenol according to the method described in Example 1/a.

### b) 2-(2-Chloro-4-fluoro-phenoxy)-phenylamine

The title compound was prepared from 2-chloro-4-fluoro-1-(2-nitro-phenoxy)-benzene according to the method described in Example 1/b.

### c) 4-[2-(2-Chloro-4-fluoro-phenoxy)-phenylsulfamoyl]-benzoic acid

The title compound was prepared from 2-(2-chloro-4-fluoro-phenoxy)-phenylamine according to the method described in Example 4/a. MS (EI) 422.1 (MH⁺).

### d) N-(3-[1,4']Bipiperidinyl-1'-yl-propyl)-4-[2-(2-chloro-4-fluoro-phenoxy)-phenylsulfamoyl]-benzamide

The title compound was prepared from 4-[2-(2-chloro-4-fluoro-phenoxy)-phenylsulfamoyl]-benzoic acid and 3-[1,4']bipiperidinyl-1'-yl)-propylamine trihydrochloride (Reference Example 3) according to the method described in Example 31/b. MS (EI) 629.2 (MH⁺).

### Example 117

### 4-[2-(2-Chloro-4-fluoro-phenoxy)-phenylsulfamoyl]-N-(3-piperidin-1-yl-propyl)-benzamide

The title compound was prepared from 4-[2-(2-chloro-4-fluoro-phenoxy)-phenylsulfamoyl]-benzoic acid (Example 116/c) and 3-piperidin-1-yl-propylamine (EMKA-Chemie) according to the method described in Example 31/b. MS (EI) 546.1 (MH⁺).

### Example 118

### 4-[2-(2-Chloro-4-fluoro-phenoxy)-phenylsulfamoyl]-N-(2-piperidin-1-yl-ethyl)-benzamide

The title compound was prepared from 4-[2-(2-chloro-4-fluoro-phenoxy)-phenylsulfamoyl]-benzoic acid (Example 116/c) and 2-piperidin-1-yl-ethylamine (EMKA-Chemie) according to the method described in Example 31/b. MS (EI) 532.1 (MH⁺).

### Example 119

### 4-[2-(2-Chloro-4-fluoro-phenoxy)-phenylsulfamoyl]-N-[2-(4-pyrimidin-2-yl-piperazin-1-yl)-ethyl]-benzamide

The title compound was prepared from 4-[2-(2-chloro-4-fluoro-phenoxy)-phenylsulfamoyl]-benzoic acid (Example 116/c) and 2-(4-pyrimidin-2-yl-piperazin-1-yl)-ethylamine [Bioorg. Med. Chem.; 12 (2004) 3965-3970] according to the method described in Example 31/b. MS (EI) 611.1 (ME⁺).

### Example 120

### N-(4-[1,4']Bipiperidinyl-1'-yl-phenyl)-4-[2-(2-chloro-4-fluoro-phenoxy)-phenylsulfamoyl]-benzamide

The title compound was prepared from 4-[2-(2-chloro-4-fluoro-phenoxy)-phenylsulfamoyl]-benzoic acid (Example 116/c) and 4-[1,4']bipiperidinyl-1'-yl-phenylamine [J. Med. Chem. 46 (2003) 1803-1806] according to the method described in Example 31/b. MS (EI) 663.2 (MH⁺).

### Example 121

### trans-4-12-(2-Chloro-4-fluoro-phenoxy)-phenylsulfamoyl]-N-[4-(2-pyrrolidin-1-yl-ethyl)-cyclohexyl]-benzamide

The title compound was prepared from 4-[2-(2-chloro-4-fluoro-phenoxy)-phenylsulfamoyl]-benzoic acid (Example 116/c) and trans-4-(2-pyrrolidin-1-yl-ethyl)-cyclohexylamine dihydrochloride (Reference Example 4) according to the method described in Example 31/b. MS (EI) 600.2 (MH⁺).

### Example 122

### 4-[2-(4-Chloro-2-methoxy-phenoxy)-phenylsulfamoyl]-N-(2-piperidin-1-yl-ethyl)-benzamide

### a) 2-(4-Chloro-2-methoxy-phenoxy)-phenylamine

The title compound was prepared from 4-chloro-2-methoxy-1-(2-nitro-phenoxy)-benzene [J. Chem. Soc. (1934) 867] according to the method described in Example 1/b.

### b) 4-[2-(4-Chloro-2-methoxy-phenoxy)-phenylsulfamoyl]-benzoic acid

The title compound was prepared from 2-(4-chloro-2-methoxy-phenoxy)-phenylamine according to the method described in Example 1/c. MS (EI) 434.2 (NM⁺).

### c) 4-[2-(4-Chloro-2-methoxy-phenoxy)-phenylsulfamoyl]-N-(2-piperidin-1-yl-ethyl)-benzamide

The title compound was prepared from 4-[2-(4-chloro-2-methoxy-phenoxy)-phenylsulfamoyl]-benzoic acid and 2-piperidin-1-yl-ethylamine (EMKA-Chemie) according to the method described in Example 31/b. MS (EI) 544.2 (MH⁺).

### Example 123

### 4-[2-(4-Chloro-2-methoxy-phenoxy)-phenylsulfamoyl]-N-(3-piperidin-1-yl-propyl)-benzamide

The title compound was prepared from 4-[2-(4-chloro-2-methoxy-phenoxy)-phenylsulfamoyl]-benzoic acid (Example 122/b) and 3-piperidin-1-yl-propylamine (EMKA-Chemie) according to the method described in Example 31/b. MS (EI) 558.2 (MH⁺).

### Example 124

### N-(4-[1,4']Bipiperidinyl-1'-yl-phenyl)4-[2-(4-chloro-2-methoxy-phenoxy)-phenylsulfamoyl]-benzamide

The title compound was prepared from 4-[2-(4-chloro-2-methoxy-phenoxy)-phenylsulfamoyl]-benzoic acid (Example 122/b) and 4-[1,4']bipiperidinyl-1'-yl-phenylamine [J. Med. Chem. 46 (2003) 1803-1806] according to the method described in Example 31/b. MS (EI) 675.2 (MH⁺).

### Example 125

### 4-[2-(4-Chloro-2-methoxy-phenoxy)-phenylsuyfamoyl]-N-[2-(4-pyrimidin-2-yl-piperazin-1-yl)-ethyl]-benzamide

The title compound was prepared from 4-[2-(4-chloro-2-methoxy-phenoxy)-phenylsulfamoyl]-benzoic acid (Example 122/b) and 2-(4-pyrimidin-2-yl-piperazin-1-yl)-ethylamine *[*Bioorg. Med. Chem.; 12 (2004) 3965-3970] according to the method described in Example 31/b. MS (EI) 623.2 (MH⁺).

### Example 126

### 4-[2-(4-Chloro-2-methoxy-phenoxy)-phenylsulfamoyl]-N-[2-(2,3,5,6-tetrahydro-[1,2']bipyrazinyl-4-yl)-ethyl]-benzamide

The title compound was prepared from 4-[2-(4-chloro-2-methoxy-phenoxy)-phenylsulfamoyl]-benzoic acid (Example 122/b) and 2-(2,3,5,6-tetrahydro-[1,2']bipyrazinyl-4-yl)-ethylamine (Reference Example 16) according to the method described in Example 31/b. MS (EI) 623.2 (MH⁺).

### Example 127

### N-(4-[1,4']Bipiperidinyl-1'-yl-phenyl)-4-[2-(4-trifluoromethyl-phenoxy)-phenylsulfamoyl]-benzamide

### a) 4-[2-(4-Trifluormethyl-phenoxy)-phenylsulfamoyl]-benzoic acid

The title compound was prepared from 2-(4-trifluoromethyl-phenoxy)-phenylamine [J. Chem. Soc. Perkin Trans. 1. (1976) 1279-1285] according to the method described in Example 4/a. MS (EI) 438.0 (MH⁺).

### b) N-(4-[1,4']Bipiperidinyl-1'-yl-phenyl)-4-[2-(4-trifluoromethyl-phenoxy)-phenylsulfamoyl]-benzamide

The title compound was prepared from 4-[2-(4-trifluormethyl-phenoxy)-phenylsulfamoyl]-benzoic acid and 4-[1,4']bipiperidinyl-1'-yl-phenylamine [J. Med. Chem. 46 (2003) 1803-1806] according to the method described in Example 31/b. MS (EI) 680.2 (MH⁺).

### Example 128

### N-(3-Pineridin-1-yl-propyl)-4-[2-(4-trifluoromethyl-phenoxy)-phenylsulfamoyl]-benzamide

The title compound was prepared from 4-[2-(4-trifluormethyl-phenoxy)-phenylsulfamoyl]-benzoic acid (Example 127/a) and 3-piperidin-1-yl-propylamine (EMKA-Chemie) according to the method described in Example 31/b. MS (EI] 562.2 (MH⁺).

### Example 129

### N-(3-[1,4']Bipiperidinyl-1'-yl-propyl)-4-[2-(4-trifluoromethyl-phenoxy)-phenylsulfamoyl]-benzamide

The title compound was prepared from 4-[2-(4-trifluormethyl-phenoxy)-phenylsulfamoyl]-benzoic acid (Example 127/a) and 3-[1,4']bipiperidinyl-1'-yl)-propylamine trihydrochloride (Reference Example 3) according to the method described in Example 31/b. MS (EI) 645.2 (MH⁺).

### Example 130

### trans-N-[4-(2-Pyrrolidin-1-yl-ethyl)-cyclohexyl]-4-[2-(4-trifluoromethyl-phenoxy)-phenylsulfamoyl]-benzamide

The title compound was prepared from 4-[2-(4-trifluormethyl-phenoxy)-phenylsulfamoyl]-benzoic acid (Example 127/a) and trans-4-(2-pyrrolidin-1-yl-ethyl)-cyclohexylamine dihydrochloride (Reference Example 4) according to the method described in Example 31/b. MS (EI) 616.2 (MH⁺).

### Example 131

### N-[2-(4-Pyrimidin-2-yl-piperazin-1-yl)-ethyl]-4-[2-(4-trifluoromethyl-phenoxy)-phenylsulfamoyl]-benzamide

The title compound was prepared from 4-[2-(4-trifluormethyl-phenoxy)-phenylsulfamoyl]-benzoic acid (Example 127/a) and 2-(4-pyrimidin-2-yl-piperazin-1-yl)-ethylamine [Bioorg. Med. Chem.; 12 (2004) 3965-3970] according to the method described in Example 31/b. MS (EI) 627.2 (MH⁺).

### Example 132

### N-(3-[1,4']Bipineridinyl-1'-yl-propyl)-4-[2-(4-chloro-2-methoxy-phenoxy)-phenylsulfamoyl]-benzamide

The title compound was prepared from 4-[2-(4-chloro-2-methoxy-phenoxy)-phenylsulfamoyl]-benzoic acid (Example 122/b) and 3-[1,4']bipipeidinyl-1'-yl)-propylamine trihydrochloride (Reference Example 3) according to the method described in Example 31/b. MS (EI) 641.2 (MH⁺).

### Example 133

### trans-4-[2-(4-Chloro-2-methoxy-phenoxy)-phenylsulfamoyl]-N-[4-(2-pyrrolidin-1-yl-ethyl)-cyclohexyl]-benzamide

The title compound was prepared from 4-[2-(4-chloro-2-methoxy-phenoxy)-phenylsulfamoyl]-benzoic acid (Example 122/b) and trans-4-(2-pyrrolidin-1-yl-ethyl)-cyclohexylamine dihydrochloride (Reference Example 4) according to the method described in Example 31/b. MS (EI) 612.2 (MH⁺).

### Example 134

### 4-[2-(4-Chloro-2-methoxy-phenoxy)-phenylsulfamoyl]-N-(4-piperidin-4-yl-butyl)-benzamide hydrochloride

The title compound was prepared from 4-[2-(4-chloro-2-methoxy-phenoxy)-phenylsulfamoyl]-benzoic acid (Example 122/b) and 4-(4-amino-butyl)-piperidine-1-carboxylic acid tert-butyl ester (Reference Example 12) according to the method described in Example 30. MS (EI) 572.2 (MH⁺).

### Example 135

### 4-[2-(2-Chloro-4-fluoro-phenoxy)-phenylsulfamoyl]-N-(4-piperidin-4-yl-butyl)-benzamide hydrochloride

The title compound was prepared from 4-[2-(2-chloro-4-fluoro-phenoxy)-phenylsulfamoyl]-benzoic acid (Example 116/c) and 4-(4-amino-butyl)-piperidine-1-carboxylic acid tert-butyl ester (Reference Example 12) according to the method described in Example 30. MS (EI) 560.2 (MH⁺).

### Example 136

### N-(4-Piperidin-4-yl-butyl)-4-[2-(4-trifluoromethyl-phenoxy)-phenylsulfamoyl]-benzamide hydrochloride

The title compound was prepared from 4-[2-(4-trifluormethyl-phenoxy)-phenylsulfamoyl]-benzoic acid (Example 127/a) and 4-(4-amino-butyl)-piperidine-1-carboxylic acid tert-butyl ester (Reference Example 12) according to the method described in Example 30. MS (EI) 576.1 (MH⁺).

### Example 137

### 4-[2-(2,4-Dichloro-phenoxy)-phenylsulfamoyl]-N-(3-piperidin-4-yl-propyl-benzamide trifluoracetate

### a) 4-(3-{4-[2-(2,4-Dichloro-phenoxy)-phenylsulfamoyl]-benzoylamino}propyl)-piperidine-1-carboxylic acid tert-butyl ester

The title compound was prepared from 4-[2-(2,4-dichloro-phenoxy)-phenylsulfamoyl]-benzoic acid (Example 1/c) and 4-(3-amino-propyl)-piperidine-1-carboxylic acid tert-butyl ester (Reference Example 13) according to the method described in Example 1/d. MS (EI) 685.2 (M+Na⁺).

### b) 4-[2-(2,4-Dichloro-phenoxyl)-phenylsulfamoyl]-N-(3-piperidin-4-yl-propyl)-benzamide trifluoracetate

The title compound was prepared from 4-(3-{4-[2-(2,4-dichloro-phenoxy)-phenylsulfamoyl]-benzoylamino}-propyl)-piperidine-1-carboxylic acid tert-butyl ester according to the method described in Example 11/b. MS (EI) 563.2 (MH⁺).

### Example 138

### N-(3-[1,4']Bipiperidinyl-1'-yl-propyl)-4-[2-(4-tritluoromethoxy-phenoxy)-phenylsulfamoyl]-benzamide

### a) 4-[2-(4-Trifluormethoxy-phenox)-phenylsulfamoyl]-benzoic acid

The title compound was prepared from 2-(4-trifluoromethoxy-phenoxy)-phenylamine [J. Med. Chem. 13 (1970) 295-297] according to method described in Example 4. MS (EI) 454.1 (MH⁺).

### b) N-(3-1,4']Bipiperidinyl-1'-yl-propyl)-4-[2-(4-trifluoromethoxyphenoxy)-phenylsulfamoyl]-benzamide

The title compound was prepared from 4-[2-(4-trifluormethoxy-phenoxy)-phenylsulfamoyl]-benzoic acid and 3-[1,4']bipiperidinyl-1'-yl-propylamine trihydrochloride (Reference Example 3) according to the method described in Example 31/b. MS (EI] 661.2 (MH⁺).

### Example 139

### N-(4-[1,4']Biniperidinyl-1'-yl-phenyl)-4-[2-(4-trifluoromethoxy-phenoxy)-phenylsulfamoyl]-benzamide

The title compound was prepared from 4-[2-(4-triffuormethoxy-phenoxy)-phenylsulfamoyl]-benzoic acid (Example 138/a) and 4-[1,4']bipiperidinyl-1'-yl-phenylamine [J. Med. Chem. 46 (2003) 1803-1806] according to the method described in Example 31/b. MS (EI) 695.2 (MH⁺).

### Example 140

### N-[2-(4-Pyrimidin-2-yl-piperazin-1-yl)-ethyl]-4-[2-(4-trifluoromethoxy-phenoxy)-phenylsulfamoyl]-benzamide

The title compound was prepared from 4-[2-(4-trifluormethoxy-phenoxy)-phenylsulfamoyl]-benzoic acid (Example 138/a) and 2-(4-pyrimidin-2-yl-piperazin-1-yl)-ethylamine [Bioorg. Med. Chem.; 12 (2004) 3965-3970] according to the method described in Example 31/b. MS (EI) 643.2 (MH⁺).

### Example 141

### N-[2-(2,3,5,6-Tetrahydro-[1,2']bipyrazinyl-4-yl)-ethyl]4-[2-(4-trifluoromethoxy-phenoxy)-phenylsulfamoyl]-benzamide

The title compound was prepared from 4-[2-(4-trifluormethoxy-phenoxy)-phenylsulfamoyl]-benzoic acid (Example 138/a) and 2-(2,3,5,6-tetrahydro-[1,2']bipyrazinyl-4-yl)-ethylamine (Reference Example 16) according to the method described in Example 31/b. MS (EI) 643.3 (MH⁺).

### Example 142

### 4-[2-(2-Chloro-4-methoxy-phenoxy)-phenylsulfamoyl]-N-(3-piperidin-1-yl-propyl)-benzamide

### a) 2-Chloro-4-methoxy-1-(2-nitro-phenoxy)-benzene

The title compound was prepared from 2-chloro-4-methoxy-phenol according to the method described in Example 1/a.

### b) 2-(2-Chloro-4-methoxy-phenoxy)-phenylamine

The title compound was prepared from 2-chloro-4-methoxy-1-(2-nitro-phenoxy)-benzene according to the method described in Example 1/b.

### c) 4-[2-(2-Chloro-4-methoxy-phenoxy)-phenylsulfamoyl]-benzoic acid

The title compound was prepared from 2-(2-chloro-4-methoxy-phenoxy)-phenylamine according to the method described in Example 4/a. MS (EI) 434.1 (MH⁺).

### d) 4-[2-(2-Chloro-4-methoxy-phenoxy)-phenylsulfamoyl]-N-(3-piperidin-1-yl-propyl)-benzamide

The title compound was prepared from 4-[2-(2-chloro-4-methoxy-phenoxy)-phenylsulfamoyl]-benzoic acid and 3-piperidin-1-yl-propylamine (EMKA-Chemie) according to the method described in Example 31/b. MS (EI) 558.2 (MH⁺).

### Example 143

### 4-[2-(2-Chloro-4-methoxy-phenoxy)-phenylsulfamoyl]-N-(4-piperidin-4-yl-butyl)-benzamide hydrochloride

The title compound was prepared from 4-[2-(2-chloro-4-methoxy-phenoxy)-phenylsulfamoyl]-benzoic acid (Example 142/c) and 4-(4-amino-butyl)-piperidine-1-carboxylic acid tert-butyl ester (Reference Example 12) according to the method described in Example 30. MS (EI) 572.2 (MH⁺).

### Example 144

### N-(4-Piperidin-4-yl-butyl)-4-[2-(4-trifluoromethoxy-phenoxy)-phenylsulfamoyl]-benzamide hydrochloride

The title compound was prepared from 4-[2-(4-trifluormethoxy-phenoxy)-phenylsulfamoyl]-benzoic acid (Example 138/a) and 4-(4-amino-butyl)-piperidine-1-carboxylic acid *tert*-butyl *e*ster (Reference Example 12) according to the method described in Example 30. MS (EI) 592.2 (MH⁺).

### example 145

### N-(4-[1,4']Bipiperidinyl-1'-yl-phenyl)-4-[2-(2-chloro-4-methoxy-phenoxy)-phenylsulfamoyl]-benzamide

The title compound was prepared from 4-[2-(2-chloro-4-methoxy-phenoxy)-phenylsulfamoyl]-benzoic acid (Example 142/c) and 4-[1,4']bipiperidinyl-1'-yl-phenylamine [J. Med. Chem. 46 (2003) 1803-1806] according to the method described in Example 31/b. MS (EI) 675.2 (MH⁺).

### Example 146

### 4-[2-(2-Chloro-4-methoxy-phenoxy)-phenylsulfamoyl]-N-(2-piperidin-1-yl-ethyl)-benzamide

The title compound was prepared from 4-[2-(2-chloro-4-methoxy-phenoxy)-phenylsulfamoyl]-benzoic acid (Example 142/c) and 2-piperidin-1-yl-ethylamine (EMKA-Chemie) according to the method described in Example 31/b. MS (EI) 544.2 (MH⁺).

### Example 147

### N-(3-[1,4']Bipiperidinyl-1'-yl-propyl)-4-[2-(2-chloro-4-methoxy-phenoxy)-phenylsulfamoyl]-benzamide

The title compound was prepared from 4-[2-(2-chloro-4-methoxy-phenoxy)-phenylsulfamoyl]-benzoic acid (Example 142/c) and 3-[1,4']bipiperidinyl-1'-yl)-propylamine trihydrochloride (Reference Example 3) according to the method described in Example 31/b. MS (EI) 641.2 (MH⁺).

### Example 148

### trans-4-[2-(2-Chloro-4-methoxy-phenoxy)-phenylsulfamoyl]-N-[4-(2-pyrrolidin-1-yl-ethyl)-cyclohexyl]-benzamide

The title compound was prepared from 4-[2-(2-chloro-4-methoxy-phenoxy)-phenylsulfamoyl]-benzoic acid (Example 142/c) and trans-4-(2-pyrrolidin-1-yl-ethyl)-cyclohexylamine dihydrochloride (Reference Example 4) according to the method described in Example 31/b. MS (EI) 612.2 (MH⁺).

### Example 149

### 4-[2-(2-Chloro-4-methoxy-phenoxy)-phenylsulfamoyl]-N-[2-(4-pyrimidin-2-yl-piperazin-1-yl)-ethyl]-benzamide

The title compound was prepared from 4-[2-(2-chloro-4-methoxy-phenoxy)-phenylsulfamoyl]-benzoic acid (Example 142/c) and 2-(4-pyrimidin-2-yl-piperazin-1-yl)-ethylamine [Bioorg. Med. Chem.; 12 (2004) 3965-3970] according to the method described in Example 31/b. MS (EI) 623.2 (MH⁺).

### Example 150

### 4-[2-(2-Chloro-4-methoxy-phenoxy)-phenylsulfamoyl]-N-[2-(2,3,5,6-tetrahydro-[1,2']bipyrazinyl-4-yl)-ethyl]-benzamide

The title compound was prepared from 4-[2-(2-chloro-4-methoxy-phenoxy)-phenylsulfamoyl]-benzoic acid (Example 142/c) and 2-(2,3,5,6-tetrahydro-[1,2']bipyrazinyl-4-yl)-ethylamine (Reference Example 16) according to the method described in Example 31/b. MS (EI) 623.3 (MI⁺).

### Example 151

### 4-(2-Phenoxy-phenylsulfamoyl)-N-(2-piperidin-1-yl-ethyl)-benzamide

The title compound was prepared from 4-(2-phenoxy-phenylsulfamoyl)-benzoic acid (Example 31/a) and 2-piperidin-1-yl-ethylamine (EMKA-Chemie) according to the method described in Example 31/b. MS (EI) 480.2 (MH⁺).

### Example 152

### N-(3-[1,4']Bipiperidinyl-1'-yl-propyl)-4-(2-phenoxy-phenylsulfamoyl)-benzamide

The title compound was prepared from 4-(2-phenoxy-phenylsulfamoyl)-benzoic acid (Example 31/a) and 3-[1,4']bipiperidinyl-1'-yl)-propylamine trihydrochloride (Reference Example 3) according to the method described in Example 31/b. MS (EI) 577.2 (MH⁺).

### Example 153

### trans-4-(2-Phenoxy-phenylsulfamoyl)-N-[4-(2-pyrrolidin-1-yl-ethyl)-cyclohexyl]-benzamide

The title compound was prepared from 4-(2-phenoxy-phenylsulfamoyl)-benzoic acid (Example 31/a) and trans-4-(2-pyrrolidin-1-yl-ethyl)-cyclohexylamine dihydrochloride (Reference Example 4) according to the method described in Example 31/b. MS (EI) 548.3 (MH⁺).

### Example 154

### 4-(2-Phenoxy-phenylsulfamoyl)-N-[2-(4-pyrimidin-2-yl-piperazin-1-yl)-ethyl]-benzamide

The title compound was prepared from 4-(2-phenoxy-phenylsulfamoyl)-benzoic acid (Example 31/a) and 2-(4-pyrimidin-2-yl-piperazin-1-yl)-ethylamine [Bioorg. Med. Chem.; 12 (2004) 3965-3970] according to the method described in Example 31/b. MS (EI) 559.2 (MH⁺).

### Example 155

### 4-(2-Phenoxy-phenylsulfamoyl)-N-(4-piperidin-4-yl-butyl)-benzamide hydrochloride

The title compound was prepared from 4-(2-phenoxy-phenylsulfamoyl)-benzoic acid (Example 31/a) and 4-(4-amino-butyl)-piperidine-1-carboxylic acid tert-butyl ester (Reference Example 12) according to the method described in Example 30. MS (EI) 508.2 MH⁺).

### Example 156

### 4-[2-(4-Bromo-2-chloro-phenoxy)-phenylsulfamoyl]-N-(3-piperidin-1-yl-propyl)-benzamide

The title compound was prepared from 4-[2-(4-bromo-2-chloro-phenoxy)-phenylsulfamoyl]-benzoic acid (Example 22/c) and 3-piperidin-1-yl-propylamine (EMKA-Chemie) according to the method described in Example 31/b. MS (EI) 608.1 (MH⁺).

### Example 157

### N-(3-[1,4']Bipiperidinyl-1'-yl-propyl)-4-[2-(4-bromo-2-chloro-phenoxy)-phenylsulfamoyl]-benzamide

### N-(3-[1,4']Bipiperidinyl-1'-yl-propyl)-4-[2-(4-bromo-2-chloro-phenoxy)-phenylsulfamoyl]-benzamide

The title compound was prepared from 4-[2-(4-bromo-2-chloro-phenoxy)-phenylsulfamoyl]-benzoic acid (Example 22/c) and 3-[1,4']bipiperidinyl-1'-yl)-propylamine trihydrochloride (Reference Example 3) according to the method described in Example 31/b. MS (EI) 691.1 (MH⁺).

### Example 158

### 4-[2-(4-Bromo-2-chloro-phenoxy)-phenylsulfamoyl]-N-[2-(4-pyrirmidin-2-yl-piperazin-1-yl)-ethyl]-benzamide

The title compound was prepared from 4-[2-(4-bromo-2-chloro-phenoxy)-phenylsulfamoyl]-benzoic acid (Example 22/c) and 2-(4-pyrimidin-2-yl-piperazin-1-yl)-ethylamine [Bioorg. Med. Chem.; 12 (2004) 3965-3970] according to the method described in Example 31/b. MS (EI) 673.0 (MH⁺).

### Example 159

### 4-[2-(4-Bromo-2-chloro-phenoxy)-phenylsulfamoyl]-N-[2-(2,3,5,6-tetrahydro-[1,2']bipyrazinyl-4-yl)-ethyl]-benzamide

The title compound was prepared from 4-[2-(4-bromo-2-chloro-phenoxy)-phenylsulfamoyl]-benzoic acid (Example 22/c) and 2-(2,3,5,6-tetrahydro-[1,2']bipyrazinyl-4-yl)-ethylamine (Reference Example 16) according to the method described in Example 31/b. MS (EI) 673.0 (MH⁺).

### Example 160

### 4-[2-(3-Diethylamino-phenoxy)-phenylsulfamoyl]-N-(3-piperidin-1-yl-propyl)-benzamide

### a) 3-Diethylamino-1-(2-nitro-phenoxyl-benzene

The title compound was prepared from 3-diethylamino-phenol according to the method described in Example 1/a. MS (EI) 287.1 (MH⁺).

### b) 2-(3-Diethylamino-phenoxy)-phenylamine

The title compound was prepared from 3-diethylamino-1-(2-nitro-phenoxy)-benzene according to the method described in Example 1/b.

### c) [2-(3-Diethylamino-phenoxy)-phenylsulfamoyl]-benzoic acid

The title compound was prepared from 2-(3-diethylamino-phenoxy)-phenylamine according to the method described in Example 1/c. MS (EI) 441.1 (MH⁺).

### d) 4-[2-(3-Diethylamino-phenoxy)-phenylsulfamoyl]-N-(3-piperidin-1-yl-propyl)-benzamide

The title compound was prepared from 4-[2-(3-diethylamino-phenoxy)-phenylsulfamoyl]-benzoic acid and 3-piperidin-1-yl-propylamine (EMKA-Chemie) according to the method described in Example 31/b. MS (EI) 565.3 (MH⁺).

### Example 161

### N-(3-[1,4']Bipiperidinyl-1'-yl-propyl)-4-[2-(3-diethylamino-phenoxy)-phenylsulfamoyl]-benzamide

The title compound was prepared from 4-[2-(3-diethylamino-phenoxy)-phenylsulfamoyl]-benzoic acid (Example 160/c) and 3-[1,4']bipiperidinyl-1'-yl)-propylamine trihydrochloride (Reference Example 3) according to the method described in Example 31/b. MS (EI) 648.3 (MH⁺).

### Example 162

### trans-4-[2-(3-Diethylamino-phenoxy)-phenylsulfamoyl]-N-[4-(2-pyrrolidin-1-yl-ethyl)-cyclohexyl]-benzamide

The title compound was prepared from 4-[2-(3-diethylamino-phenoxy)-phenylsulfamoyl]-benzoic acid (Example 160/c) and trans-4-(2-pyrrolidin-1-yl-ethyl)-cyclohexylamine dihydrochloride (Reference Example 4) according to the method described in Example 31/b. MS (EI) 619.3 (MH⁺).

### Example 163

### 4-[2-(3-Diethylamino-phenoxy)-phenylsulfamoyl]-N-[2-(4-pyrimidin-2-yl-pinerazin-1-yl)-ethyl]-benzamide

The title compound was prepared from 4-[2-(3-diethylamino-phenoxy)-phenylsulfamoyl]-benzoic acid (Example 160/c) and 2-(4-pyrimidin-2-yl-piperazin-1-yl)-ethylamine [Bioorg. Med. Chem.; 12 (2004) 3965-3970] according to the method described in Example 31/b. MS (EI) 630.3 (MH⁺).

### Example 164

### 4-[2-(3-Diethylamino-phenoxy)-phenylsulfamoyl]-N-(3-piperidin-4-yl-propyl)-benzamide hydrochloride

The title compound was prepared from 4-[2-(3-diethylamino-phenoxy)-phenylsulfamoyl]-benzoic acid (Example 160/c) and 4-(3-amino-propyl)-piperidine-1-carboxylic acid tert-butyl ester (Reference Example 13) according to the method described in Example 30. MS (EI) 565.3 (MH⁺).

### Example 165

### 4-[2-(3-Diethylamino-phenoxy)-phenylsulfamoyl]-N-(4-piperidin-4-ylmethyl-benzyl)-benzamide hydrochloride

The title compound was prepared from 4-[2-(3-diethylamino-phenoxy)-phenylsulfamoyl]-benzoic acid (Example 160/c) and 4-(4-aminomethyl-benzyl)-piperidine-1-carboxylic acid tert-butyl ester (Reference Example 17) according to the method described in Example 30. MS (EI] 627.3 (MH⁺).

### Example 166

### 4-[2-(4-Bromo-2-chloro-phenoxy)-phenylsulfamoyl]-N-(4-piperidin-4-ylmethyl-benzyl)-benzamide hydrochloride

The title compound was prepared from 4-[2-(4-bromo-2-chloro-phenoxy)-phenylsulfamoyl]-benzoic acid (Example 160/c) and 4-(4-aminomethyl-benzyl)-piperidine-1-carboxylic acid tert-butyl ester (Reference Example 17) according to the method described in Example 30. MS (EI) 670.2 (MH⁺).

### Example 167

### 4-[2-(4-Bromo-2-chloro-phenoxy)-phenylsulfamoyl]-N-(4-piperidin-4-yl-butyl)-benzamide hydrochloride

The title compound was prepared from 4-[2-(4-bromo-2-chloro-phenoxy)-phenylsulfamoyl]-benzoic acid (Example 22/c) and 4-(4-amino-butyl)-piperidine-1-carboxylic acid tert-butyl ester (Reference Example 12) according to the method described in Example 30. MS (EI) 622.1 (MH⁺).

### Example 168

### N-(4-[1,4']Bipiperidinyl-1'-yl-phenyl)4-[2-(3-diethylamino-phenoxy)-phenylsulfamoyl]-benzamide

The title compound was prepared from 4-[2-(3-diethylamino-phenoxy)-phenylsulfamoyl]-benzoic acid (Example 160/c) and 4-[1,4']bipiperidinyl-1'-yl-phenylamine [J. Med. Chem. 46 (2003) 1803-1806] according to the method described in Example 31/b. MS (EI) 682.3 (MH⁺).

### Example 169

### N-(4-[1,4']Bipiperidinyl-1'-yl-phenyl)-4-[2-(4-bromo-2-chloro-phenoxy)-phenylsnlfamoyl]-benzamide

The title compound was prepared from 4-[2-(4-bromo-2-chloro-phenoxy)-phenylsulfamoyl]-benzoic acid (Example 22/c) and 4-[1,4']bipiperidinyl-1'-yl-phenylamine [J. Med. Chem. 46 (2003) 1803-1806] according to the method described in Example 31/b. MS (EI) 725.1 (MH⁺).

### Example 170

### 4-[2-(2,4-Dichloro-phenoxy)-phenylsulfamoyl]-N-(4-piperidin-4-yl-butyl-benzamide hydrochloride

The title compound was prepared from 4-[2-(2,4-dichloro-phenoxy)-phenylsulfamoyl]-benzoic acid (Example 1/c) and 4-(4-amino-butyl)-piperidirie-1-carboxylic acid tert-butyl ester (Reference Example 12) according to the method described in Example 30. MS (EI) 576.2 (MH⁺).

### Example 171

### N-(2-Benzoyl-phenyl)-4-[4-(3-piperidin-1-yl-propyl)-piperazin-1-carbonyl]-benzenesulfonamide

### a) 4-(2-Benzoyl-phenylsulfamoyl)-benzoic acid

The title compound was prepared from 2-amino-benzophenone according to the method described in Example 1/c. MS (EI) 382.2 (MH⁺).

### b) N-(2-Benzoyl-phenyl)-4-(3-piperidin-1-yl-propyl)-piperazin-1-carbonyl]-benzenesulfonamide

The title compound was prepared from 4-(2-benzoyl-phenylsulfamoyl)-benzoic acid and 1-(3-piperidin-1-yl-propyl)-piperazine (EMKA-Chemie) according to the method described in Example 1/d. MS (EI) 575.2 (MH⁺).

### Example 172

### N-[2-(2,4-Dichloro-phenoxy)-phenyl]-4-[4-(3-piperidin-4-yl-propyl)-piperidine-1-carbonyl]-benzenesulfonamide

The title compound was prepared from 4-[2-(2,4-dichloro-phenoxy)-phenylsulfamoyl]-benzoic acid (Example 1/c) and 4,4'-trimethylenedipiperidine (Aldrich) according to the method described in Example 31/b. MS (EI) 631.2 (MH⁺).

### Example 173

### N-(2-Benzoyl-phenyl)-4-[4-(3-pyrrolidin-1-yl-propyl)-piperazine-1-carbonyl]-benzenesulfonamide

The title compound was prepared from 4-(2-benzoyl-phenylsulfamoyl)-benzoic acid (Example 171/a) and 1-(3-pyrrolidin-1-yl-propyl)-piperazine (EMKA-Chemie) according to the method described in Example 31/b. MS (EI) 575.2 (MH⁺).

### Example 174

### N-[2-(2,4-Dichloro-benzoyl)-phenyl]-4-[4-(3-pyrrolidin-1-yl-propyl)-piperazine-1-carbonyl]-benzenesulfonamide

### a) 4-[2-(2,4-Dichloro-benzoyl)-phenylsulfamoyl]-benzoic acid

The title compound was prepared from (2-amino-phenyl)-(2,4-dichloro-phenyl)-methanone [Synthesis, (1980) 677-688] and 4-chlorosulfonyl-benzoic acid according to the method described in Example 1/c. MS (EI) 451 (MH⁺).

### b) N-[2-(2,4-Dichloro-benzoyl)-phenyl]-4-[4-(3-pyrrolidin-1-yl-propyl)-piperazine-1-carbonyl]-benzenesulfonamide

The title compound was prepared from 4-[2-(2,4-dichloro-benzoyl)-phenylsulfamoyl]-benzoic acid and 1-(3-pyrrolidin-1-yl-propyl)-piperazine (EMKA-Chemie) according to the method described in Example 31/b. MS (EI) 630.4 (MH⁺).

### Example 175

### N-[2-(2,4-Dichloro-benzoyl)-phenyl]-4-[4-(3-piperidin-1-yl-propyl)-piperazine-1-carbonyl]-benzenesulfonamide

The title compound was prepared from 4-[2-(2,4-dichloro-benzoyl)-phenylsulfamoyl]-benzoic acid (Example 174/a) and 1-(3-pyrrolidin-1-yl-propyl)-piperazine (EMKA-Chemie) according to the method described in Example 31/b. MS (EI) 644.3 (MH⁺).

### Example 176

### trans-4-[2-(2,4-Dichloro-benzoyl)-phenylsulfamoyl]-N-[4-(2-pyrrolidin-1-yl-ethyl)-cyclohexyl]-benzamide

The title compound was prepared from 4-[2-(2,4-dichloro-benzoyl)-phenylsulfamoyl]-benzoic acid (Example 174/a) and trans-4-(2-pyrrolidin-1-ylethyl)-cyclohexylamine dihydrochloride (Reference Example 4) according to the method described in Example 31/b. MS (EI) 629.2 (MH⁺).

### Example 177

### 4-[2-(2,4-Dichloro-benzoyl)-phenylsulfamoyl]-N [3-(4-pyridin-4-yl-piperazin-1-yl)-propyl]-benzamide

The title compound was prepared from 4-[2-(2,4-dichloro-benzoyl)-phenylsulfamoyl]-benzoic acid (Example 174/a) and 2-(4-pyridin-4-yl-piperazin-1-yl)-propylamine (Reference Example 11) according to the method described in Example 31/b. MS (EI) 653.2 (MH⁺).

### Example 178

### N-[2-(2,4-Dichloro-benzoyl)-phenyl]-4-[4-(3-piperidin-4-yl-propyl)-piperidine-1-carbonyl]-benzenesulfonamide

The title compound was prepared from 4-[2-(2,4-dichloro-benzoyl)-phenylsulfamoyl]-benzoic acid (Example 174/a) and 4,4'-trimethylenedipiperidine (Aldrich) according to the method described in Example 31/b. MS (EI) 643.2 (MH⁺).

### Example 179

### trans-4-(2-Benzoyl-phenylsulfamoyl)-N-[4-(2-pyrrolidin-1-yl-ethyl)-cyclohexyl]-benzamide

The title compound was prepared from 4-(2-benzoyl-phenylsulfamoyl)-benzoic acid (Example 171/a) and trans-4-(2-pyrrolidin-1-ylethyl)-cyclohexylamine dihydrochloride (Reference Example 4) according to the method described in Example 31/b. MS (EI) 560.5 (MH⁺).

### Example 180

### 4-(2-Benzoyl-phenylsulfamoyl)-N-[3-(4-pyridin-4-yl-pinerazin-1-yl)-propyl]-benzamide

The title compound was prepared from 4-(2-benzoyl-phenylsulfamoyl)-benzoic acid (Example 171/a) and 2-(4-pyridin-4-yl-piperazin-1-yl)-propylamine (Reference Example 11) according to the method described in Example 31/b. MS (EI) 584.4 (MH).

### Example 181

### 4-[2-(2,4-Dichloro-benzoyl)-phenylsulfamoyl]-N-(3-piperidin-4-yl-propyl)-benzamide

### hydrochloride

The title compound was prepared from 4-[2-(2,4-dichloro-benzoyl)-phenylsulfamoyl]-benzoic acid (Example 174/a) and 4-(3-amino-propyl)-piperidine-1-carboxylic acid tert-butyl ester (Reference Example 13) according to the method described in Example 30. MS (EI) 575.3 (MH⁺).

### Example 182

### 4-(2-Benzoyl-phenylsulfamoyl)-N-(4-piperidin-4-yl-butyl)-benzamide hydrochloride

The title compound was prepared from 4-[2-(2,4-dichloro-benzoyl)-phenylsulfamoyl]-benzoic acid (Example 171/a) and 4-(4-amino-butyl)-piperidine-1-carboxylic acid tert-butyl ester (Reference Example 12) according to the method described in Example 30. MS (EI) 520.4 (MH⁺).

### Example 183

### N-(2-Benzoyl-phenyl)4-[4-(3-piperidin-4-yl-propyl)-piperidine-1-carbonyl]-benzenesulfonamide

The title compound was prepared from 4-(2-benzoyl-phenylsulfamoyl)-benzoic acid (Example 171/a) and and 4,4'-trimethylenedipiperidine (Aldrich) according to the method described in Example 31/b. MS (EI) 574.2 (MH⁺).

### Example 184

### Preparation of pharmaceutical compositions:

### a) Tablets:

0.01-50 % of active ingredient of formula (I), 15-50 % of lactose, 15-50 % of potato starch, 5-15 % of polyvinyl pyrrolidone, 1-5 % of talc, 0.01-3 % of magnesium stearate, 1-3 % of colloid silicon dioxide and 2-7 % of ultraamylopectin were mixed, then granulated by wet granulation and pressed to tablets.

### b) Dragées, filmcoated tablets:

The tablets made according to the method described above were coated by a layer consisting of entero- or gastrosolvent film, or of sugar and talc. The dragées were polished by a mixture of beeswax and carnuba wax.

### c) Capsules:

0.01-50 % of active ingredient of formula (I), 1-5 % of sodium lauryl sulfate, 15-50 % of starch, 15-50 % of lactose, 1-3 % of colloid silicon dioxide and 0.01-3 % of magnesium stearate were thoroughly mixed, the mixture was passed through a sieve and filled in hard gelatin capsules.

### d) Suspensions:

Ingredients: 0.01-15 % of active ingredient of formula (I), 0.1-2 % of sodium hydroxide, 0.1-3 % of citric acid, 0.05-0.2 % of nipagin (sodium methyl 4-hydroxybenzoate), 0.005-0.02 % of nipasol, 0.01-0.5 % of carbopol (polyacrilic acid), 0.1-5 % of 96 % ethanol, 0.1-1 % of flavoring agent, 20-70 % of sorbitol (70 % aqueous solution) and 30-50 % of distilled water.

To solution of nipagin and citric acid in 20 ml of distilled water, carbopol was added in small portions under vigorous stirring, and the solution was left to stand for 10-12 h. Then the sodium hydroxide in 1 ml of distilled water, the aqueous solution of sorbitol and finally the ethanolic raspberry flavor were added with stirring. To this carrier the active ingredient was added in small portions and suspended with an immersing homogenizator. Finally the suspension was filled up to the desired final volume with distilled water and the suspension syrup was passed through a colloid milling equipment.

### e) Suppositories:

For each suppository 0.01-15% of active ingredient of formula (I) and 1-20% of lactose were thoroughly mixed, then 50-95% of adeps pro suppository (for example Witepsol 4) was melted, cooled to 35 °C and the mixture of active ingredient and lactose was mixed in it with homogenizator. The obtained mixture was mould in cooled forms.

### f) Lyophilized powder ampoule compositions:

A 5 % solution of mannitol or lactose was made with bidistilled water for injection use, and the solution was filtered so as to have sterile solution. A 0.01-5 % solution of the active ingredient of formula (I) was also made with bidistilled water for injection use, and this solution was filtered so as to have sterile solution. These two solutions were mixed under aseptic conditions, filled in 1 ml portions into ampoules, the content of the ampoules was lyophilized, and the ampoules were sealed under nitrogen. The contents of the ampoules where dissolved in sterile water or 0.9 % (physiological) sterile aqueous sodium chloride solution before administration.

## Claims

1. Bradykinin B1 receptor antagonist sulfonamide derivatives of formula (I) 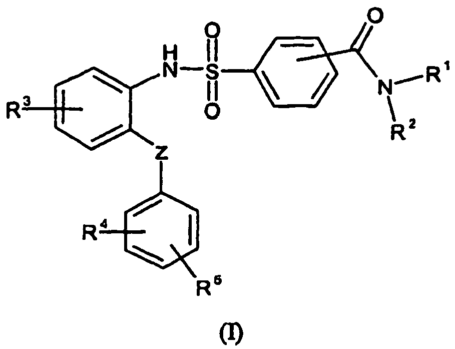 wherein
R¹ is hydrogen atom or C₁-C₄ alkyl group;
R² is selected from (1) hydrogen atom; with the proviso that R¹ and R² can not be simultaneously hydrogen atom; (2) -(CH₂)ₙ-NR^{a}R^{b}, (3) -(CH₂)ₘ X-Q, or
R¹ and R² together with the nitrogen atom to which they are attached form a 4-7 membered heterocyclic ring containing 0-3 heteroatom (in addition to the nitrogen atom to which R¹ and R² attached) selected from O, S and N; wherein said ring is optionally substituted with C₁-C₄ alkyl, -(CH₂)_{q}-Y-P, oxo, 4-(4,5-dihydro-1*H*-imidazol-2-yl)-benzyl, 4-(1,4,5,6-tetrahydro-pyrimidin-2-yl)-benzyl or 4-ylmethyl-benzamidine group;
R³, R⁴ and R⁵ are independently of each other hydrogen atom, halogen atom, cyano, amino, or amino substituted with one or more C₁-C₄ alkyl group, C₁-C₄ alkoxy, trifluoromethyl, trifluoromethoxy, C₁-C₄ alkyl, hydroxy, C₁-C₄ alkoxycarbonyl or -C(=O)-NH₂ group;
Z is selected from (1) single bond; (2) oxygen atom; (3) CH₂ group; (4) CO group; (5) NR^{c} group; (6) S atom; (7) SO₂ group ;
n is an integer from 1 to 4;
m is 0 or an integer from 2 to 6;
q is an integer from 0 to 4;
R^{a} and R^{b} are (1) hydrogen atom, (2) straight or branched C₁-C₆ alkyl group;
R^{c} is hydrogen atom or C₁-C₄ alkyl group;
X is a single bond, -CO- or -CO-NH- or -NH-CO- group;
Y is a single bond, -CO- or -CONR^{a} group;
P is (1) -N(C₁-C₄ alkyl group; (2) -NH-(CH₂)ₙ-Het group; (3) a saturated, partially unsaturated or aromatic 4-7 membered ring containing 1-3 heteroatom selected from O, S and N; wherein said ring is optionally substituted with oxo or C₁-C₄ alkyl group;
Q is (1) a saturated, partially unsaturated or aromatic 4-7 membered ring containing 1-3 heteroatom selected from O, S and N; wherein said ring is optionally substituted with oxo, -(CH₂)ₙ-Het, 1-piperidinyl, 1-(C₁-C₄-alkyl)-4-piperidinyl, 4-piperidinyl, 2-pyrimidinyl, 2-pyrazinyl, 2-pyridyl, 4-methyl-2-pyridyl, 6-methyl-2-pyridyl or 4-pyridyl group; (2) phenyl group, optionally substituted with -CH₂)ₙ-Het, -(CH₂)ₙ-NH-C(=NH)-NH₂, 4,5-dihydro-1*H*-imidazol-2-yl or [1,4']bipiperidinyl-1'-yl group; (3) C₅-C₇ cycloalkyl group, optionally substituted with -(CH₂)ₙ-Het group; (4) benzyl group, optionally substituted with -CH₂)ₙ-Het, -(CH₂)ₙ-NH-C(=NH)-NH₂ or 4,5-dihydro-1*H* imidazol-2-yl group; (5) -CH₂)ₙ-Het group;
Het is a 4-7 membered heterocyclic ring containing 1-3 heteroatom selected from O, S, SO₂ and N, optionally substituted with (1) oxo;. (2) one or more C₁-C₄ alkyl group;
and optical antipodes or racemates and/or salts and/or hydrates and/or solvates thereof, wherein 3-[([1,1'-biphenyl]-2-ylamino sulphonyl]-N-[2-(2-pyridinyl)-ethyl]benzamide is excluded.

2. A compound of Claim 1 selected from the group of 4-[2-(2,4-dichloro-phenoxy)-phenylsulfamoyl]-*N*-{2-[4-pyridin-4-yl-piperazin-1-yl)-ethyl]-benzamide dihydrochloride, 4-[2-(2,4-dichloro-phenoxy)-phenylsulfynoyl]-*N*-{2-[4-(4,5-dihydro-1*H*-imidazol-2-yl)-phenyl]-ethyl}-benzamide trifluoroacetate, *N*-(3-[1,4']bipiperidinyl-1'-yl-propyl)-4-[2-(2,4-dichloro-phenoxy)-phenylsulfamoyl]-benzamide, 4-[2-(3,4-dichloro-phmoxy)-phenylsulfamoyl]-*N*-{2-[4-(4,5-dihydro-1*H*-inlidazol-2-yl)-phenyl]-ethyl}-benzamide, *N*-(3-[1,4']bipiperidinyl-1'-yl-propyl)-4-[2-(3,4-dichloro-phenoxy)-phenylsulfamoyl]-benzamide, 4-[2-(4-chloro-phenoxy)-phenylsulfamoyl]-*N*-{2-{4-(4,5-dihydro-1*H*-imidazol-2-yl)-phenyl]-ethyl}-benzamide, 4-[2-(4-bromo-phenoxy)-phenylsulfamoyl]-*N*-(2-[4-(4,5-dihydro-1H-imidazol-2-yl)-phenyl]-ethyl)-benzamide, *N*-(4-[1,4']bipiperidinyl-1'-yl-phenyl)-4-[2-(2,4-dichloro-phenoxy)-phenylsulfamoyl]-benzamide, trans-4-[2-(2,4-dichloro-phenoxy)-phenylsulfamoyl]-*N*-[4-(2-pyrrolidin-1-yl-ethyl)-cyclohexyl]-benzamide, 3-[2-(2,4-dichloro-phenoxy)-phenylsulfarnoyl]-*N*-[2-(4-pyridin-4-yl-piperazin-1-yl)-ethyl]-benzamide dihydrochloride, 4-[2-(2,4-dichloro-phenoxy)-phenylsulfamoyl]-*N*-(2-piperidin-4-yl-ethyl)-benzamide trifluoroacetate. 4-[2-(2,4-dichloro-phenoxy)-phenylsulfamoyl]-*N*-[2-(4-pyridin-2-yl-piperazin-1-yl)-ethyl]-benzamide trifluoroacetate, 4-[2-(4-bromo-2-chloro-phenoxy)-phenylsulfamoyl]-*N*-{2-[4-(4,5-dihydro-1*H*-imidazol-2-yl)-phenyl]-ethyl}-benzamide, 4-[2-(4-bromo-phenoxy)-phenylsulfamoyl]-*N*-(4-guanidinomethyl-benzyl)-benzamide hydrochloride, 4-[2-(2,4-dichloro-phenoxy)-phenylsulfalmoyl]-*N*-[2-(1'-methyl-[1,4']bipiperidinyl-4-yl)-ethyl]-benzamide, 4-[2-(2,4-dichloro-phenoxy)-phenylsulfamoyl]-*N*-{2-[4-pyridin-4-yl-piperazin-1-yl)-propyl]-benzamide, piperidine-4-carboxylic acid (2-{4-[2-(2,4-dichloro-phenoxy)-phenylsulfamoyl]-benzoylamino}-ethyl)-amide, 4-[2-(4-bromo-2-chloro-phenoxy)-phenylsulfamoyl]-*N*-(3-piperidin-4-yl-propyl)-benzamide hydrochloride, *N*-(4-[1,4']bipiperidinyl-1'-yl-phenyl)-4-(2-phenoxy-phenylsulfamoyl)-benzamide, 4-[2-(2,4-dichloro-phenoxy)-phenylsulfamoyl]-*N*-[2-(piperidin-4-ylcarbamoyl)-ethyl]-benzamide acetate, 4-[2-(2,4-dichloro-phenoxy)-phenylsulfamoyl]-*N*-{2-[4-(6-methyl-pyridin-2-yl)-piperazin-1-yl]-ethyl}-benzamide, 4-[2-(2,4-dichloro-phenoxy)-phenylsulfamoyl]-*N*-{2-[(piperidin-4-ylmethyl)-carbamoyl]-ethyl}-benzamide hydrochloride, 4-[2-(2,4-dichloro-phenoxy)-phenylsulfamoyl]-*N*-{2-[4-(4-methyl-pyridin-2-yl)-piperazin-1-yl]-ethyl}-benzamide, 4-[2-(2,4-dichloro-phenoxy)-phenylsulfamoyl]-*N*-[2-(2-piperidin-4-yl-ethylcarbamoyl)-ethyl]-benzamide hydrochloride, *N*-(4-[1,4']bipiperidinyl-1'-yl-phenyl)-4-[2-(4-bromo-phenoxy)-5-fluoro-phenylsulfamoyl]-benzamide, *N*-(3-[1,4']bipiperidinyl-1'-yl-3-oxo-propyl)-4-[2-(2,4-dichloro-phenoxy)-phenylsulfamoyl]-benzamide trifluoroacetate, 4-[2-(3,4-dichloro-phenoxy)-phenylsulfamoyl]-*N*-[4-(2-pyrrolidin-1-yl-ethyl)-cyclohexyl]-benzamide, 4-[2-(3,4-dichloro-phenoxy)-phenylsulfamoyl]-*N*-[2-(4-pyridin-2-yl-piperazin-1-yl)-ethyl]-benzamide, 4-[2-(3,4-dichloro-phenoxy)-phenylsulfamoyl]-*N*-[3-(4-pyridin-4-yl-piperazin-1-yl)-propyl]-benzamide, 4-[2-(3,4-dichloro-phenoxy)-phenylsulfamoyl]-*N-*{2-[4-(6-methyl-pyridin-2-yl)-piperazin-1-yl]-ethyl}-benzamide, 4-[2-(2,4-dichloro-phenoxy)-phenylsulfamoyl]-*N*-(3-piperidin-1-yl-propyl)-benzamide, *N*-[2-(3,4-dichloro-phenoxy)-phenyl]-4-[4-(3-piperidin-1-yl-propyl)-piperazine-1-carbonyl]-benzenesulfonamide, 4-[2-(4-bromo-phenoxy)-phenylsulfamoyl]-*N*-[2-(4-pyridin-2-ylmethyl-piperazin-1-yl)-ethyl]-benzamide, 4-[2-(4-bromo-phenoxy)-phenylsulfamoyl]-*N*-(4-piperidin-4-yl-butyl)-benzamide hydrochloride, *N*-(3-[1,4']bipiperidinyl-1'-yl-propyl)-4-[2-(4-bromo-phenoxy)-5-fluoro-phenylsulfamoyl]-benzamide, 4-[2-(4-bromo-phenoxy)-5-fluoro-phenylsulfamoyl]-*N*-[4-(2-pyrrolidin-1-yl-ethyl)-cyclohexyl]-benzamide, 4-[2-(4-bromo-phenoxy)-5-fluoro-phenylsulfamoyl]-*N*-[3-(4-pyridin-4-yl-piperazin-1-yl)-propyl]-benzamide, 4-[2-(4-bromo-phenoxy)-5-fluoro-phenylsulfamoyl]-*N*-(4-piperidin-4-yl-butyl)-benzamide hydrochloride, *N*-(3-[1,4']bipiperidinyl-1'-yl-propyl)-4-[2-(4-bromo-phenoxy)-phenylsulfamoyl]-benzamide, 4-[2-(4-bromo-phenoxy)-phenylsulfamoyl]-*N*-[3-(4-pyridin-4-yl-piperazin-1-yl)-propyl]-benzamide, *N*-(3-[1,4']bipiperidinyl-1'-yl-propyl)-4-[2-(2-chloro-4-fluoro-phenoxy)-phenylsulfamoyl]-benzamide, 4-[2-(2-chloro-4-fluoro-phenoxy)-phenylsulfamoyl]-*N*-[4-(2-pyrrolidin-1-yl-ethyl)-cyclohexyl]-benzamide, *N*-(3-[1,4']bipiperidinyl-1'-yl-propyl)-4-[2-(4-trifluoromethyl-phenoxy)-phenylsulfamoyl]-benzamide, *N*-(3-[1,4']bipiperidinyl-1'-yl-propyl)-4-[2-(4-chloro-2-methoxy-phenoxy)-phenylsulfamoyl]-benzamide, 4-[2-(2-chloro-4-fluoro-phenoxy)-phenylsulfamoyl]-*N-*(4-piperidin-4-yl-butyl)-benzamide hydrochloride, *N*-(4-[1,4']bipiperidinyl-1'-yl-phenyl)-4-[2-(4-trifluoromethyl-phenoxy)-phenylsulfamoyl]-benzamide, 4-[2-(2,4-dichloro-phenoxy)-phenylsulfamoyl]-*N*-(3-piperidin-4-yl-propyl)-benzamide hydrochloride, *N*-(3-[1,4']bipiperidinyl-1'-yl-propyl)-4-[2-(2-chloro-4-methoxy-phenoxy)-phenylsulfamoyl]-benzamide, *N*-(3-[1,4']bipiperidinyl-1'-yl-propyl)-4-(2-phenoxy-phenylsulfamoyl)-benzamide, *N*-(3-[1,4']bipiperidinyl-1'-yl-propyl)-4-[2-(4-bromo-2-chloro-phenoxy)-phenylsulfamoyl]-benzamide, 4-[2-(4-bromo-2-chloro-phenoxy)-phenylsulfamoyl]-*N*-[2-(2,3,5,6-tetahydro-[1,2']bipyrazinyl-4-yl)-ethyl]-benzamide, 4-[2-(4-bromo-2-chloro-phenoxy)-phenylsulfamoyl]-*N*-(4-piperidin-4-ylmethyl-benzyl)-benzamide hydrochloride, 4-[2-(4-bromo-2-chloro-phenoxy)-phenylsulfamoyl]-*N*-(4-piperidin-4-yl-butyl)-benzamide hydrochloride, *N*-(4-[1,4']bipiperidinyl-1'-yl-phenyl)-4-[2-(4-bromo-2-chloro-phenoxy)-phenylsulfamoyl]-benzamide, 4-[2-(2,4-dichloro-phenoxy)-phenylsulfamoyl]-*N*-(4-piperidin-4-yl-butyl)-benzamide hydrochloride or *N*-(2-benzoyl-phenyl)-4-[4-(3-piperidin-1-yl-propyl)-piperazin-1-carbonyl]-benzenesulfonamide.

3. A process for preparing the compounds of formula (I) as claimed in Claim 1 which comprises reacting an amine derivative of formula (II) 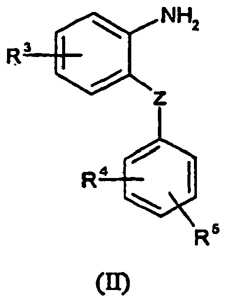
- wherein the meaning of R³, R⁴ and R⁵ are as described above for the formula of (I) - with sulfonyl chloride of formula (III) 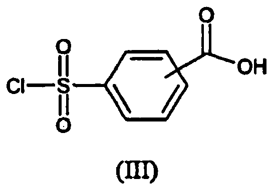 then reacting the so obtained phenylsulfamoyl benzoic acid derivative of formula (IV) 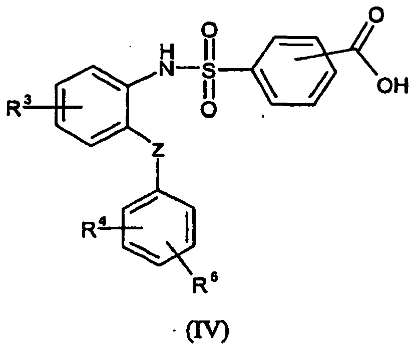
- wherein the meaning of R³, R⁴ and R⁵ are as defined above - with an amine derivative of formula (V) 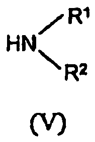
- wherein the meaning of R¹ and R² are as defined above- to obtain phenylsulfamoyl benzamide derivative of formula (I) or optical antipodes or racemates and/or pharmaceutically acceptable salts and/or hydrates and/or solvates thereof.

4. A process for preparing the compounds of formula (I) as claimed in Claim 1 which comprises transforming a compound of formula (I) into an other compound of formula (I) by introducing new substituents and/or modifying or removing the existing ones, and/or salt formation and/or liberating the compound from salts.

5. A compound of formula (V) selected from the group of N-(4-aminomethyl-benzyl)-guanidine dihydrochloride and 4-piperidin-4-ylmethyl-benzamidine.

6. A pharmaceutical composition comprising a therapeutically effective amount of a compound of formula (I) as claimed in Claim 1 or optical antipodes or racemates or pharmaceutically acceptable salt or hydrate or solvate thereof and one or more pharmaceutically acceptable excipients.

7. Use of a compound of formula (I) as claimed in Claim 1 or optical antipodes or racemates or a pharmaceutically acceptable salt or hydrate or solvate thereof for the manufacture of a medicament for prevention and/or treatment of a condition which requires inhibition of a bradykinin receptor.

8. Use according to Claim 7 wherein the bradykinin receptor is bradykinin B1 receptor.

9. The compound of formula (I) as claimed in Claim 1 or optical antipodes or racemates or pharmaceutically acceptable salt or hydrate or solvate thereof for use in a method of treating and/or preventing a condition which requires inhibition of a bradykinin receptor.

10. The compound for use according to Claim 9 wherein the bradykinin receptor is bradykinin B1 receptor.

11. A bradykinin B1 receptor antagonist sulfonamide derivatives of formula (I) for use as a medicament 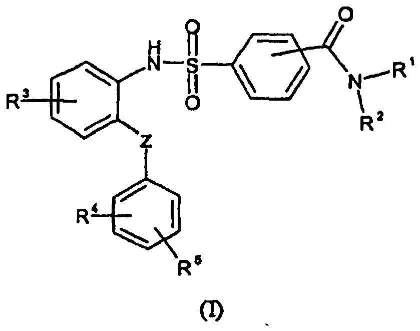 wherein
R¹ is hydrogen atom or C₁-C₄ alkyl group;
R² is selected from (1) hydrogen atom; with the proviso that R¹ and R² can not be simultaneously hydrogen atom; (2) -(CH₂)ₙ-NR^{a}R^{b}, (3) -(CH₂)ₘ-X-Q, or
R¹ and R² together with the nitrogen atom to which they are attached form a 4-7 membered heterocyclic ring containing 0-3 heteroatom (in addition to the nitrogen atom to which R¹ and R² attached) selected from O, S and N; wherein said ring is optionally substituted with C₁-C₄ alkyl, -(CH₂)_{q}-Y-P, oxo, 4-(4,5-dihydro-1*H*-imidazol-2-yl)-benzyl, 4-(1,4,5,6-tetrahydro-pyrimidin-2-yl)-benzyl or 4-ylmethyl-benzamidine group;
R³, R⁴ and R⁵ are independently of each other hydrogen atom, halogen atom, cyano, amino, or amino substituted with one or more C₁-C₄ alkyl group, C₁-C₄ alkoxy, trifluoromethyl, trifluoromethoxy, C₁-C₄ alkyl, hydroxy, C₁-C₄ alkoxycarbonyl or -C(=O)-NH₂ group;
Z is selected from (1) single bond; (2) oxygen atom; (3) CH₂ group; (4) CO group; (5) NR^{c} group; (6) S atom; (7) SO₂ group;
n is an integer from 1 to 4;
m is 0 or an integer from 2 to 6;
q is an integer from 0 to 4;
R^{a} and R^{b} are (1) hydrogen atom, (2) straight or branched C₁-C₆ alkyl group;
R^{c} is hydrogen atom or C₁-C₄ alkyl group;
X is a single bond, -CO- or -CO-NH- or -NH-CO- group;
Y is a single bond, -CO- or -CONR^{a} group;
P is (1) -N(C₁-C₄ alkyl)₂ group; (2) -NH-(CH₂)ₙ-Het group; (3) a saturated, partially unsaturated or aromatic 4-7 membered ring containing 1-3 heteroatom selected from O, S and N; wherein said ring is optionally substituted with oxo or C₁-C₄ alkyl group;
Q is (1) a saturated, partially unsaturated or aromatic 4-7 membered ring containing 1-3 heteroatom selected from O, S and N; wherein said ring is optionally substituted with oxo, -(CH₂)ₙ-Het, 1-piperidinyl, 1-(C₁-C₄-alkyl)-4-piperidinyl, 4-piperidinyl, 2-pyrimidinyl, 2-pyrazinyl, 2-pyridyl, 4-methyl-2-pyridyl, 6-methyl-2-pyridyl or 4-pyridyl group; (2) phenyl group, optionally substituted with -(CH₂)ₙ-Het, -(CH₂)ₙ-NH-C(=NH)-NH₂, 4,5-dihydro-1*H*-imidazol-2-yl or [1,4']bipiperidinyl-1'-yl group; (3) C₅-C₇ cycloalkyl group, optionally substituted with -(CH₂)ₙ-Het group; (4) benzyl group, optionally substituted with -(CH₂)ₙ-Het, -(CH₂)ₙ-NH-C(=NH)-NH₂ or 4,5-dihydro-1*H*-imidazol-2-yl group; (5) -(CH₂)ₙ-Het group;
Het is a 4-7 membered heterocyclic ring containing 1-3 heteroatom selected from O, S, SO₂ and N, optionally substituted with (1) oxo; (2) one or more C₁-C₄ alkyl group;
and optical antipodes or racemates and/or salts and/or hydrates and/or solvates thereof.

## Patentansprüche

1. Bradykinin B1-Rezeptor-Antagonist-Sulfonamidderivat der Formel (I) 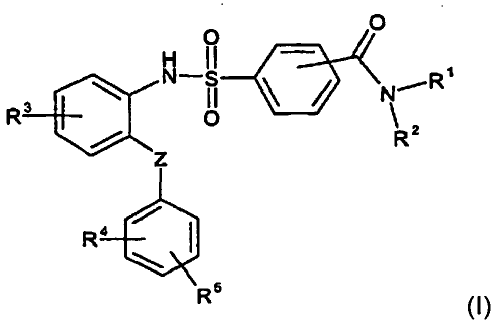 worin:
R¹ ein Wasserstoffatom oder eine C₁₋₄-Alkylgruppe ist;
R² aus (1) einem Wasserstoffatom, mit der Massgabe, dass R¹ und R² nicht gleichzeitig ein Wasserstoffatom sein können; (2) -(CH₂)ₙ-NR^{a}R^{b}; (3) -(CH₂)ₘ-X-Q ausgewählt ist, oder
R¹ und R² zusammen mit dem Stickstoffatom, an das sie gebunden sind, einen 4- bis 7-gliedrigen heterocyclischen Ring, enthaltend 0 bis 3 Heteroatom(e) (zusätzlich zu dem Stickstoffatom, an das R¹ und R² gebunden sind), ausgewählt aus O, S und N, bilden; wobei dieser Ring gegebenenfalls mit einer C₁₋₄-Alkyl-, -(CH₂)_{q}-Y-P-, Oxo-, 4-(4,5-Dihydro-1H-imidazol-2-yl)-benzyl-, 4-(1,4,5,6-Tetrahydro-pyrimidin-2-yl)-benzyl- oder -4-ylmethyl-benzamidin-Gruppe substituiert ist;
R³, R⁴ und R⁵ jeweils unabhängig voneinander ein Wasserstoffatom, ein Halogenatom, Cyano, Amino oder mit einer oder mehreren C₁₋₄-Alkylgruppe(n) substituiertes Amino; C₁₋₄-Alkoxy, Trifluormethyl, Trifluormethoxy, C₁₋₄-Alkyl, Hydroxy, C₁₋₄-Alkoxycarbonyl oder eine -C(=O)-NH₂-Gruppe sind;
Z aus (1) einer Einfachbindung; (2) einem Sauerstoffatom; (3) einer CH₂-Gruppe; (4) einer CO-Gruppe; (5) einer NR^{c}-Gruppe; (6) einem S-Atom; (7) einer SO₂-Gruppe ausgewählt ist;
n eine ganze Zahl von 1 bis 4 ist;
m 0 oder eine ganze Zahl von 2 bis 6 ist;
q eine ganze Zahl von 0 bis 4 ist;
R^{a} und R^{b} (1) ein Wasserstoffatom; (2) eine geradkettige oder verzweigte C₁₋₆-Alkylgruppe sind;
R^{c} ein Wasserstoffatom oder eine C₁₋₄-Alkylgruppe ist;
X eine Einfachbindung, -CO- oder eine -CO-NH- oder -NH-CO-Gruppe ist;
Y eine Einfachbindung, -CO- oder eine -CONR^{a}-Gruppe ist;
P (1) eine -N(C₁₋₄-Alkyl)₂-Gruppe; (2) eine -NH-(CH₂)ₙ-Het-Gruppe; (3) ein gesättigter, teilweise ungesättigter oder aromatischer 4- bis 7-gliedriger Ring, enthaltend 1 bis 3 Heteroatom(e), ausgewählt aus O, S und N, ist; wobei dieser Ring gegebenenfalls mit Oxo oder einer C₁₋₄-Alkylgruppe substituiert ist;
Q (1) ein gesättigter, teilweise ungesättigter oder aromatischer 4- bis 7-gliedriger Ring, enthaltend 1 bis 3 Heteroatom(e), ausgewählt aus O, S und N; wobei dieser Ring gegebenenfalls mit einer Oxo-, -(CH₂)ₙ-Het-, 1-Piperidinyl-, 1-(C₁₋₄-Alkyl)-4-piperidinyl-, 4-Piperidinyl-, 2-Pyrimidinyl-, 2-Pyrazinyl-, 2-Pyridyl-, 4-Methyl-2-pyridyl-, 6-Methyl-2-pyridyl- oder 4-Pyridylgruppe substituiert ist; (2) eine Phenylgruppe, gegebenenfalls substituiert mit einer -(CH₂)ₙ-Het-, -(CH₂)ₙ-NH-C(=NH)-NH₂-, 4,5-Dihydro-1H-imidazol-2-yl-oder [1,4']Bipiperidinyl-1'-yl-Gruppe; (3) eine C₅₋₇-Cycloalkylgruppe, gegebenenfalls substituiert mit einer -(CH₂)ₙ-Het-Gruppe; (4) eine Benzylgruppe, gegebenenfalls substituiert mit einer -(CH₂)ₙ-Het-, -(CH₂)ₙ-NH-C(=NH)-NH₂- oder 4,5-Dihydro-1H-imidazol-2-yl-Gruppe; (5) eine -(CH₂)ₙ-Het-Gruppe ist;
Het ein 4- bis 7-gliedriger heterocyclischer Ring, enthaltend 1 bis 3 Heteroatom(e), ausgewählt aus O, S, SO₂ and N, gegebenenfalls substituiert mit (1) Oxo; (2) einer oder mehreren C₁₋₄-Alkylgruppe(n) ist;
und optische Antipoden oder Racemate und/oder Salze und/oder Hydrate und/oder Solvate davon,
wobei 3-[([1,1'-Biphenyl]-2-ylamino)sulfonyl]-N-[2-(2-pyridinyl)-ethyl]benzamid ausgeschlossen ist.

2. Verbindung gemäss Anspruch 1, ausgewählt aus der Gruppe:
4-[2-(2,4-Dichlor-phenoxy)-phenylsulfamoyl]-N-[2-(4-pyridin-4-yl-piperazin-1-yl)-ethyl]-benzamid-Dihydrochlorid,
4-[2-(2,4-Dichlor-phenoxy)-phenylsulfamoyl]-N-{2-[4-(4,5-dihydro-1H-imidazol-2-yl)-phenyl]-ethyl}-benzamidtrifluoracetat,
N-(3-[1,4']Bipiperidinyl-1'-yl-propyl)-4-[2-(2,4-dichlor-phenoxy)-phenylsulfamoyl]-benzamid,
4-[2-(3,4-Dichlor-phenoxy)-phenylsulfamoyl]-N-{2-[4-(4,5-dihydro-1H-imidazol-2-yl)-phenyl]-ethyl}-benzamid,
N-(3-[1,4']Bipiperidinyl-1'-yl-propyl)-4-[2-(3,4-dichlor-phenoxy)-phenylsulfamoyl]-benzamid,
4-[2-(4-Chlor-phenoxy)-phenylsulfamoyl]-N-{2-[4-(4,5-dihydro-1H-imidazol-2-yl)-phenyl]-ethyl}-benzamid,
4-[2-(4-Brom-phenoxy)-phenylsulfamoyl]-N-{2-[4-(4,5-dihydro-1H-imidazol-2-yl)-phenyl]-ethyl}-benzamid,
N-(4-[1,4']Bipiperidinyl-1'-yl-phenyl)-4-[2-(2,4-dichlor-phenoxy)-phenylsulfamoyl]-benzamid,
trans-4-[2-(2,4-Dichlor-phenoxy)-phenylsulfamoyl]-N-[4-(2-pyrrolidin-1-yl-ethyl)-cyclohexyl]-benzamid,
3-[2-(2,4-Dichlor-phenoxy)-phenylsulfamoyl]-N-[2-(4-pyridin-4-yl-piperazin-1-yl)-ethyl]-benzamid-Dihydrochlorid,
4-[2-(2,4-Dichlor-phenoxy)-phenylsulfamoyl]-N-(2-piperidin-4-yl-ethyl)-benzamidtrifluoracetat,
4-[2-(2,4-Dichlor-phenoxy)-phenylsulfamoyl]-N-[2-(4-pyridin-2-yl-piperazin-1-yl)-ethyl]-benzamidtrifluoracetat,
4-[2-(4-Brom-2-chlor-phenoxy)-phenylsulfamoyl]-N-{2-[4-(4,5-dihydro-1H-imidazol-2-yl)-phenyl]-ethyl}-benzamid,
4-[2-(4-Brom-phenoxy)-phenylsulfamoyl]-N-(4-guanidinomethyl-benzyl)-benzamid-Hydrochlorid,
4-[2-(2,4-Dichlor-phenoxy)-phenylsulfamoyl]-N-[2-(1'-methyl-[1,4']bipiperidinyl-4-yl)-ethyl]-benzamid,
4-[2-(2,4-Dichlor-phenoxy)-phenylsulfamoyl]-N-[2-(4-pyridin-4-yl-piperazin-1-yl)-propyl]-benzamid,
Piperidin-4-carbonsäure-(2-{4-[2-(2,4-dichlor-phenoxy)-phenylsulfamoyl]-benzoylamino}-ethyl)-amid,
4-[2-(4-Brom-2-chlor-phenoxy)-phenylsulfamoyl]-N-(3-piperidin-4-yl-propyl)-benzamid-Hydrochlorid,
N-(4-[1,4']Bipiperidinyl-1'-yl-phenyl)-4-(2-phenoxy-phenylsulfamoyl)-benzamid,
4-[2-(2,4-Dichlor-phenoxy)-phenylsulfamoyl]-N-[2-(piperidin-4-ylcarbamoyl)-ethyl]-benzamidacetat,
4-[2-(2,4-Dichlor-phenoxy)-phenylsulfamoyl]-N-{2-[4-(6-methyl-pyridin-2-yl)-piperazin-1-yl]-ethyl}-benzamid,
4-[2-(2,4-Dichlor-phenoxy)-phenylsulfamoyl]-N-{2-[(piperidin-4-ylmethyl)-carbamoyl]-ethyl}-benzamid-Hydrochlorid,
4-[2-(2,4-Dichlor-phenoxy)-phenylsulfamoyl]-N-{2-[4-(4-methyl-pyridin-2-yl)-piperazin-1-yl]-ethyl}-benzamid,
4-[2-(2,4-Dichlor-phenoxy)-phenylsulfamoyl]-N-[2-(2-piperidin-4-yl-ethylcarbamoyl)-ethyl]-benzamid-Hydrochlorid,
N-(4-[1,4']Bipiperidinyl-1'-yl-phenyl)-4-[2-(4-brom-phenoxy)-5-fluor-phenylsulfamoyl]-benzamid,
N-(3-[1,4']Bipiperidinyl-1'-yl-3-oxo-propyl)-4-[2-(2,4-dichlor-phenoxy)-phenylsulfamoyl]-benzamidtrifluoracetat,
4-[2-(3,4-Dichlor-phenoxy)-phenylsulfamoyl]-N-[4-(2-pyrrolidin-1-yl-ethyl)-cyclohexyl]-benzamid,
4-[2-(3,4-Dichlor-phenoxy)-phenylsulfamoyl]-N-[2-(4-pyridin-2-yl-piperazin-1-yl)-ethyl]-benzamid,
4-[2-(3,4-Dichlor-phenoxy)-phenylsulfamoyl]-N-[3-(4-pyridin-4-yl-piperazin-1-yl)-propyl]-benzamid,
4-[2-(3,4-Dichlor-phenoxy)-phenylsulfamoyl]-N-{2-[4-(6-methyl-pyridin-2-yl)-piperazin-1-yl]-ethyl}-benzamid,
4-[2-(2,4-Dichlor-phenoxy)-phenylsulfamoyl]-N-(3-piperidin-1-yl-propyl)-benzamid,
N-[2-(3,4-Dichlor-phenoxy)-phenyl]-4-[4-(3-piperidin-1-yl-propyl)-piperazin-1-carbonyl]-benzolsulfonamid,
4-[2-(4-Brom-phenoxy)-phenylsulfamoyl]-N-[2-(4-pyridin-2-ylmethyl-piperazin-1-yl)-ethyl]-benzamid,
4-[2-(4-Brom-phenoxy)-phenylsulfamoyl]-N-(4-piperidin-4-yl-butyl)-benzamid-Hydrochlorid,
N-(3-[1,4']Bipiperidinyl-1'-yl-propyl)-4-[2-(4-brom-phenoxy)-5-fluor-phenylsulfamoyl]-benzamid,
4-[2-(4-Brom-phenoxy)-5-fluor-phenylsulfamoyl]-N-[4-(2-pyrrolidin-1-yl-ethyl)-cyclohexyl]-benzamid,
4-[2-(4-Brom-phenoxy)-5-fluor-phenylsulfamoyl]-N-[3-(4-pyridin-4-yl-piperazin-1-yl)-propyl]-benzamid,
4-[2-(4-Brom-phenoxy)-5-fluor-phenylsulfamoyl]-N-(4-piperidin-4-yl-butyl)-benzamid-Hydrochlorid,
N-(3-[1,4']Bipiperidinyl-1'-yl-propyl)-4-[2-(4-brom-phenoxy)-phenylsulfamoyl]-benzamid,
4-[2-(4-Brom-phenoxy)-phenylsulfamoyl]-N-[3-(4-pyridin-4-yl-piperazin-1-yl)-propyl]-benzamid,
N-(3-[1,4']Bipiperidinyl-1'-yl-propyl)-4-[2-(2-chlor-4-fluor-phenoxy)-phenylsulfamoyl]-benzamid,
4-[2-(2-Chlor-4-fluor-phenoxy)-phenylsulfamoyl]-N-[4-(2-pyrrolidin-1-yl-ethyl)-cyclohexyl]-benzamid,
N-(3-[1,4']Bipiperidinyl-1'-yl-propyl)-4-[2-(4-trifluormethyl-phenoxy)-phenylsulfamoyl]-benzamid,
N-(3-[1,4']Bipiperidinyl-1'-yl-propyl)-4-[2-(4-chlor-2-methoxy-phenoxy)-phenylsulfamoyl]-benzamid,
4-[2-(2-Chlor-4-fluor-phenoxy)-phenylsulfamoyl]-N-(4-piperidin-4-yl-butyl)-benzamid-Hydrochlorid,
N-(4-[1,4']Bipiperidinyl-1'-yl-phenyl)-4-[2-(4-trifluormethyl-phenoxy)-phenylsulfamoyl]-benzamid,
4-[2-(2,4-Dichlor-phenoxy)-phenylsulfamoyl]-N-(3-piperidin-4-yl-propyl)-benzamid-Hydrochlorid,
N-(3-[1,4']Bipiperidinyl-1'-yl-propyl)-4-[2-(2-chlor-4-methoxy-phenoxy)-phenylsulfamoyl]-benzamid,
N-(3-[1,4']Bipiperidinyl-1'-yl-propyl)-4-(2-phenoxy-phenylsulfamoyl)-benzamid,
N-(3-[1,4']Bipiperidinyl-1'-yl-propyl)-4-[2-(4-brom-2-chlor-phenoxy)-phenylsulfamoyl]-benzamid,
4-[2-(4-Brom-2-chlor-phenoxy)-phenylsulfamoyl]-N-[2-(2,3,5,6-tetrahydro-[1,2']bipyrazinyl-4-yl)-ethyl]-benzamid,
4-[2-(4-Brom-2-chlor-phenoxy)-phenylsulfamoyl]-N-(4-piperidin-4-ylmethyl-benzyl)-benzamid-Hydrochlorid,
4-[2-(4-Brom-2-chlor-phenoxy)-phenylsulfamoyl]-N-(4-piperidin-4-yl-butyl)-benzamid-Hydrochlorid,
N-(4-[1,4']Bipiperidinyl-1'-yl-phenyl)-4-[2-(4-brom-2-chlor-phenoxy)-phenylsulfamoyl]-benzamid,
4-[2-(2,4-Dichlor-phenoxy)-phenylsulfamoyl]-N-(4-piperidin-4-yl-butyl)-benzamid-Hydrochlorid oder
N-(2-Benzoyl-phenyl)-4-[4-(3-piperidin-1-yl-propyl)-piperazin-1-carbonyl]-benzolsulfonamid.

3. Verfahren zur Herstellung von Verbindungen der Formel (I) gemäss Anspruch 1, umfassend:
Umsetzen eines Aminderivats der Formel (II): 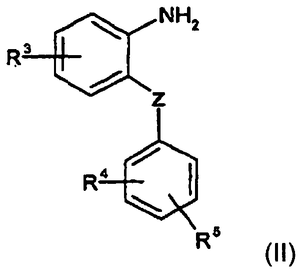
- worin die Bedeutungen von R³, R⁴ und R⁵ wie oben für die Formel (I) beschrieben sind - mit einem Sulfonylchlorid der Formel (III): 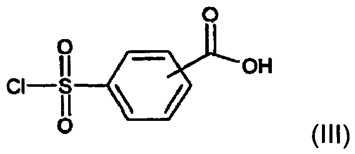 dann Umsetzen des so erhaltenen Phenylsulfamoylbenzoesäurederivats der Formel (IV): 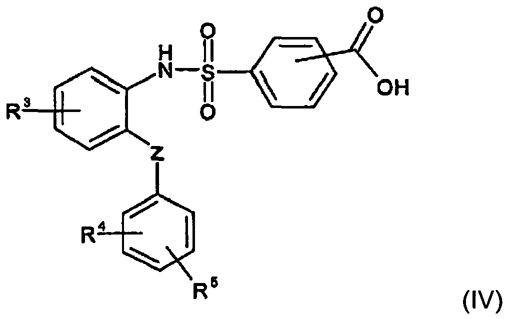
- worin die Bedeutungen von R³, R⁴ und R⁵ wie oben definiert sind - mit einem Aminderivat der Formel (V) : 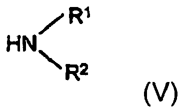
- worin die Bedeutungen von R¹ und R² wie oben definiert sind - um ein Phenylsulfamoylbenzamidderivat der Formel (I) oder optische Antipoden oder Racemate und/oder pharmazeutisch annehmbare Salze und/oder Hydrate und/oder Solvate davon zu erhalten.

4. Verfahren zur Herstellung der Verbindungen der Formel (I) gemäss Anspruch 1, das die Umwandlung einer Verbindung der Formel (I) in eine andere Verbindung der Formel (I) durch Einführen neuer Substituenten und/oder Modifizieren oder Entfernen der existierenden Substituenten und/oder Salzbildung und/oder Freisetzen der Verbindung von dem Salz umfasst.

5. Verbindung der Formel (V), ausgewählt aus der Gruppe N-(4-Aminomethyl-benzyl)-guanidin-Dihydrochlorid und 4-Piperidin-4-ylmethyl-benzamidin.

6. Pharmazeutische Zusammensetzung, umfassend eine therapeutisch wirksame Menge einer Verbindung der Formel (I) gemäss Anspruch 1 oder optische Antipoden oder Racemate oder pharmazeutisch annehmbare Salze oder Hydrate oder Solvate davon und einen oder mehrere pharmazeutisch annehmbare Hilfsstoff(e).

7. Verwendung einer Verbindung der Formel (I) gemäss Anspruch 1 oder optischen Antipoden oder Racematen oder pharmazeutisch annehmbaren Salzen oder Hydraten oder Solvaten davon zur Herstellung eines Medikaments zur Vorbeugung und/oder Behandlung eines Zustands, der die Inhibierung eines Bradykinin-Rezeptors erfordert.

8. Verwendung gemäss Anspruch 7, wobei der Bradykinin-Rezeptor ein Bradykinin B1-Rezeptor ist.

9. Verbindung der Formel (I) gemäss Anspruch 1 oder optische Antipoden oder Racemate oder pharmazeutisch annehmbare Salze oder Hydrate oder Solvate davon zur Verwendung in einem Verfahren zur Behandlung und/oder Vorbeugung eines Zustands, der die Inhibierung eines Bradykinin-Rezeptors erfordert.

10. Verbindung zur Verwendung gemäss Anspruch 9, wobei der Bradykinin-Rezeptor ein Bradykinin B1-Rezeptor ist.

11. Bradykinin B1-Rezeptor-Antagonist-Sulfonamidderivat der Formel (I) zur Verwendung als Medikament: 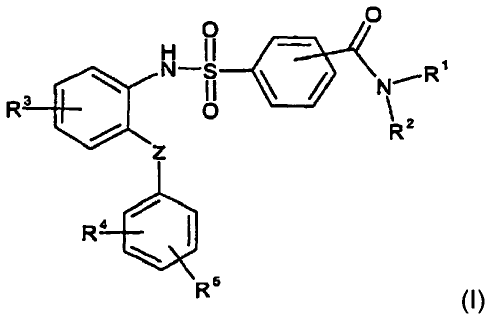 worin:
R¹ ein Wasserstoffatom oder eine C₁₋₄-Alkylgruppe ist;
R² aus (1) einem Wasserstoffatom, mit der Massgabe, dass R¹ und R² nicht gleichzeitig ein Wasserstoffatom sein können; (2) -(CH₂)ₙ-NR^{a}R^{b}; (3) -(CH₂)ₘ-X-Q ausgewählt ist, oder
R¹ und R² zusammen mit dem Stickstoffatom, an das sie gebunden sind, einen 4- bis 7-gliedrigen heterocyclischen Ring, enthaltend 0 bis 3 Heteroatom(e) (zusätzlich zu dem Stickstoffatom, an das R¹ und R² gebunden sind), ausgewählt aus O, S und N, bilden; wobei dieser Ring gegebenenfalls mit einer C₁₋₄-Alkyl-, -(CH₂)_{q}-Y-P-, Oxo-, 4-(4,5-Dihydro-1H-imidazol-2-yl)-benzyl-, 4-(1,4,5,6-Tetrahydro-pyrimidin-2-yl)-benzyl- oder -4-ylmethyl-benzamidin-Gruppe substituiert ist;
R³, R⁴ und R⁵ jeweils unabhängig voneinander ein Wasserstoffatom, ein Halogenatom, Cyano, Amino oder mit einer oder mehreren C₁₋₄-Alkylgruppe(n) substituiertes Amino; C₁₋₄-Alkoxy, Trifluormethyl, Trifluormethoxy, C₁₋₄-Alkyl, Hydroxy, C₁₋₄-Alkoxycarbonyl oder eine -C(=O)-NH₂-Gruppe sind;
Z aus (1) einer Einfachbindung; (2) einem Sauerstoffatom; (3) einer CH₂-Gruppe; (4) einer CO-Gruppe; (5) einer NR^{c}-Gruppe; (6) einem S-Atom; (7) einer SO₂-Gruppe ausgewählt ist;
n eine ganze Zahl von 1 bis 4 ist;
m 0 oder eine ganze Zahl von 2 bis 6 ist;
q eine ganze Zahl von 0 bis 4 ist;
R^{a} und R^{b} (1) ein Wasserstoffatom; (2) eine geradkettige oder verzweigte C₁₋₆-Alkylgruppe sind;
R^{c} ein Wasserstoffatom oder eine C₁₋₄-Alkylgruppe ist;
X eine Einfachbindung, -CO- oder eine -CO-NH- oder -NH-CO-Gruppe ist;
Y eine Einfachbindung, -CO- oder eine -CONR^{a}-Gruppe ist;
P (1) eine -N(C₁₋₄-Alkyl)₂-Gruppe; (2) eine -NH-(CH₂)ₙ-Het-Gruppe; (3) ein gesättigter, teilweise ungesättigter oder aromatischer 4- bis 7-gliedriger Ring, enthaltend 1 bis 3 Heteroatom(e), ausgewählt aus O, S und N, ist; wobei dieser Ring gegebenenfalls mit Oxo oder einer C₁₋₄-Alkylgruppe substituiert ist;
Q (1) ein gesättigter, teilweise ungesättigter oder aromatischer 4- bis 7-gliedriger Ring, enthaltend 1 bis 3 Heteroatom(e), ausgewählt aus O, S und N; wobei dieser Ring gegebenenfalls mit einer Oxo-, -(CH₂)ₙ-Het-, 1-Piperidinyl-, 1-(C₁₋₄-Alkyl)-4-piperidinyl-, 4-Piperidinyl-, 2-Pyrimidinyl-, 2-Pyrazinyl-, 2-Pyridyl-, 4-Methyl-2-pyridyl-, 6-Methyl-2-pyridyl- oder 4-Pyridylgruppe substituiert ist; (2) eine Phenylgruppe, gegebenenfalls substituiert mit einer -(CH₂)ₙ-Het-, -(CH₂)ₙ-NH-C(=NH)-NH₂-, 4,5-Dihydro-1H-imidazol-2-yl-oder [1,4']Bipiperidinyl-1'-yl-Gruppe; (3) eine C₅₋₇-Cycloalkylgruppe, gegebenenfalls substituiert mit einer -(CH₂)ₙ-Het-Gruppe; (4) eine Benzylgruppe, gegebenenfalls substituiert mit einer -(CH₂)ₙ-Het-, -(CH₂)ₙ-NH-C(=NH)-NH₂- oder 4,5-Dihydro-1H-imidazol-2-yl-Gruppe; (5) eine -(CH₂)ₙ-Het-Gruppe ist;
Het ein 4- bis 7-gliedriger heterocyclischer Ring, enthaltend 1 bis 3 Heteroatom(e), ausgewählt aus O, S, SO₂ and N, gegebenenfalls substituiert mit (1) Oxo; (2) einer oder mehreren C₁₋₄-Alkylgruppe(n) ist;
und optische Antipoden oder Racemate und/oder Salze und/oder Hydrate und/oder Solvate davon.

## Revendications

1. Dérivés de sulfonamide antagonistes de récepteur de bradykinine B1 de formule (I) 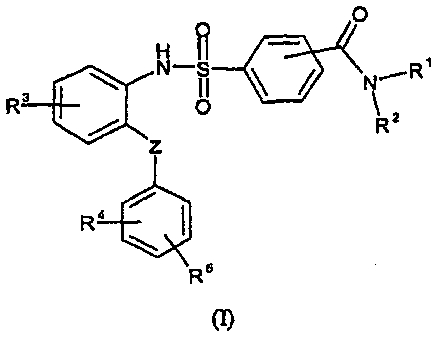 dans laquelle
R¹ est un atome d'hydrogène ou un groupe alkyle en C₁ à C₄ ;
R² est choisi parmi (1) un atome d'hydrogène, à condition que R¹ et R² ne puissent pas être simultanément un atome d'hydrogène ; (2) -(CH₂)ₙ-NR^{a}R^{b}, (3) -(CH₂)ₘ-X-Q, ou
R¹ et R² conjointement avec l'atome de carbone auquel ils sont attachés forment un cycle hétérocyclique à 4 à 7 chaînons contenant 0 à 3 hétéroatomes (en plus de l'atome d'azote auquel R¹ et R² sont attachés) choisis parmi O, S et N ; où ledit cycle est facultativement substitué par un groupe alkyle en C₁ à C₄, -(CH₂)_{q}-Y-P, oxo, 4-(4,5-dihydro-1*H*-imidazol-2-yl)-benzyle, 4-(1,4,5,6-tétrahydro-pyrimidin-2-yl)-benzyle ou 4-ylméthyl-benzamidine ;
R³, R⁴ et R⁵ sont indépendamment les uns des autres un atome d'hydrogène, un atome d'halogène, un groupe cyano, amino ou amino substitué par un ou plusieurs groupes alkyle en C₁ à C₄, un groupe alcoxy en C₁ à C₄, trifluorométhyle, trifluorométhoxy, alkyle en C₁ à C₄, hydroxy, alcoxycarbonyle en C₁ à C₄ ou -C(=O)-NH₂ ;
Z est choisi parmi (1) une simple liaison ; (2) un atome d'oxygène ; (3) un groupe CH₂ ; (4) un groupe CO ; (5) un groupe NR^{c} ; (6) un atome S ; (7) un groupe SO₂ ;
n est un nombre entier de 1 à 4 ;
m vaut 0 ou est un nombre entier de 2 à 6 ;
q est un nombre entier de 0 à 4 ;
R^{a} et R^{b} sont (1) un atome d'hydrogène, (2) un groupe alkyle en C₁ à C₆ linéaire ou ramifié ;
R^{c} est un atome d'hydrogène ou un groupe alkyle en C₁ à C₄ ;
X est une simple liaison ou un groupe -CO- ou -CO-NH- ou -NH-CO- ;
Y est une simple liaison ou un groupe -CO- ou -CONR^{a} ;
P est (1) un groupe -N(alkyle en C₁ à C₄)₂ ; (2) un groupe -NH-(CH₂)ₙ-Het ; (3) un cycle à 4 à 7 chaînons saturé, partiellement insaturé ou aromatique contenant 1 à 3 hétéroatomes choisis parmi O, S et N ; où ledit cycle est facultativement substitué par un groupe oxo ou un groupe alkyle en C₁ à C₄ ;
Q est (1) un cycle à 4 à 7 chaînons saturé, partiellement insaturé ou aromatique contenant 1 à 3 hétéroatomes choisis parmi O, S et N ; où ledit cycle est facultativement substitué par un groupe oxo, -(CH₂)ₙ-Het, 1-pipéridinyle, 1-(alkyle en C₁ à C₄)-4-pipéridinyle, 4-pipéridinyle, 2-pyrimidinyle, 2-pyrazinyle, 2-pyridyle, 4-méthyl-2-pyridyle, 6-méthyl-2-pyridyle ou 4-pyridyle ; (2) un groupe phényle, facultativement substitué par un groupe -(CH₂)ₙ-Het, -(CH₂)ₙ-NH-C(=NH)-NH₂, 4,5-dihydro-1*H-*imidazol-2-yle ou [1,4']bipipéridinyl-1'-yle ; (3) un groupe cycloalkyle en C₅ à C₇, facultativement substitué par un groupe -(CH₂)ₙ-Het ; (4) un groupe benzyle, facultativement substitué par un groupe -(CH₂)ₙ-Het, -(CH₂)ₙ-NH-C(=NH)-NH₂ ou 4,5-dihydro-1*H*-imidazol-2-yle ; (5) un groupe -(CH₂)ₙ-Het ;
Het est un cycle hétérocyclique à 4 à 7 chaînons contenant 1 à 3 hétéroatomes choisis parmi O, S, SO₂ et N, facultativement substitué par (1) un groupe oxo ; (2) un ou plusieurs groupes alkyle en C₁ à C₄ ;
et leurs antipodes ou racémates optiques et/ou sels et/ou hydrates et/ou solvates, dans lesquels le 3-[([1,1'-biphényl]-2-ylaminosulfonyl]-N-[2-(2-pyridinyl)-éthyl]benzamide est exclu.

2. Composé de la revendication 1 choisi dans le groupe constitué par le dichlorhydrate de 4-[2-(2,4-dichloro-phénoxy)-phénylsulfamoyl]-*N*-{2-[4-pyridin-4-yl-pipérazin-1-yl)-éthyl]-benzamide, le trifluoroacétate de 4-[2-(2,4-dichloro-phénoxy)-phénylsulfamoyl]-*N-*{2-[4-(4,5-dihydro-1H-imidazol-2-yl)-phényl]-éthyl}-benzamide, le *N*-(3-[1,4']bipipéridinyl-1'-yl-propyl)-4-[2-(2,4-dichloro-phénoxy)-phénylsulfamoyl]-benzamide, le 4-[2-(3,4-dichloro-phénoxy)-phénylsulfamoyl]-*N*-{2-[4-(4,5-dihydro-1*H-*imidazol-2-yl)-phényl]-éthyl}-benzamide, le *N*-(3-[1,4']bipipéridinyl-1'-yl-propyl)-4-[2-(3,4-dichloro-phénoxy)-phénylsulfamoyl]-benzamide, le 4-[2-(4-chloro-phénoxy)-phénylsulfamoyl]-*N*-{2-[4-(4,5-dihydro-1*H*-imidazol-2-yl)-phényl]-éthyl}-benzamide, le 4-[2-(4-bromo-phénoxy)- phénylsulfamoyl]-*N*-{2-[4-(4,5-dihydro-1*H-*imidazol-2-yl)-phényl]-éthyl}-benzamide, le *N*-(4-[1,4']bipipéridinyl-1'-yl-phényl)-4-[2-(2,4-dichloro-phénoxy)-phénylsulfamoyl]-benzamide, le trans-4-[2-(2,4-dichloro-phénoxy)-phénylsulfamoyl]-*N*-[4-(2-pyrrolidin-1-yl-éthyl)-cyclohexyl]-benzamide, le dichlorhydrate de 3-[2-(2,4-dichloro-phénoxy)-phénylsulfamoyl]-*N*-[2-(4-pyridin-4-yl-pipérazin-1-yl)-éthyl]-benzamide, le trifluoroacétate de 4-[2-(2,4-dichloro-phénoxy)-phénylsulfamoyl]-*N*-(2-pipéridin-4-yl-éthyl)-benzamide, le trifluoroacétate de 4-[2-(2,4-dichloro-phénoxy)-phénylsulfamoyl]-*N*-[2-(4-pyridin-2-yl-pipérazin-1-yl)-éthyl]-benzamide, le 4-[2-(4-bromo-2-chloro-phénoxy)-phénylsulfamoyl]-*N*-{2-[4-(4,5-dihydro-1*H-*imidazol-2-yl)-phényl]-éthyl}-benzamide, le chlorhydrate de 4-[2-(4-bromo-phénoxy)-phénylsulfamoyl]-*N*-(4-guanidinométhyl-benzyl)-benzamide, le 4-[2-(2,4-dichloro-phénoxy)-phénylsulfamoyl]-*N*-[2-(1'-méthyl-[1,4']bipipéridinyl-4-yl)-éthyl]-benzamide, le 4-[2-(2,4-dichloro-phénoxy)-phénylsulfamoyl)-*N*-{2-[4-pyridin-4-yl-pipérazin-1-yl)-propyl]-benzamide, le (2-{4-[2-(2,4-dichloro-phénoxy)-phénylsulfamoyl]-benzoylamino}-éthyl)-amide de l'acide pipéridine-4-carboxylique, le chlorhydrate de 4-[2-(4-bromo-2-chloro-phénoxy)-phénylsulfamoyl]-*N*-(3-pipéridin-4-yl-propyl)-benzamide, le *N*-(4-[1,4']bipipéridinyl-1'-yl-phényl)-4-(2-phénoxy-phénylsulfamoyl)-benzamide, l'acétate de 4-[2-(2,4-dichloro-phénoxy)-phénylsulfamoyl]-*N*-[2-(pipéridin-4-ylcarbamoyl)-éthyl]-benzamide, le 4-[2-(2,4-dichloro-phénoxy)-phénylsulfamoyl]-*N*-{2-[4-(6-méthyl-pyridin-2-yl)-pipéradin-1-yl]-éthyl}-benzamide, le chlorhydrate de 4-[2-(2,4-dichloro-phénoxy)-phénylsulfamoyl]-*N*-{2-[(pipéridin-4-ylméthyl)-carbamoyl]-éthyl}-benzamide, le 4-[2-(2,4-dichloro-phénoxy)-phénylsulfamoyl]-*N*-{2-[4-(4-méthyl-pyridin-2-yl)-pipérazin-1-yl]-éthyl}-benzamide, le chlorhydrate de 4-[2-(2,4-dichloro-phénoxy)-phénylsulfamoyl]-*N*-[2-(2-pipéridin-4-yl-éthylcarbamoyl)-éthyl]-benzamide, le *N*-(4-[1,4']bipipéridinyl-1'-yl-phényl)-4-[2-(4-bromo-phénoxy)-5-fluoro-phénylsulfamoyl]-benzamide, le trifluoroacétate de *N*-(3-[1,4']bipipéridinyl-1'-yl-3-oxo-propyl)-4-[2-(2,4-dichloro-phénoxy)-phénylsulfamoyl]-benzamide, le 4-[2-(3,4-dichloro-phénoxy)-phénylsulfamoyl]-*N*-[4-(2-pyrrolidin-1-yl-éthyl)-cyclohexyl]-benzamide, le 4-[2-(3,4-dichloro-phénoxy)-phénylsulfamoyl]-*N*-[2-(4-pyridin-2-yl-pipérazin-1-yl)-éthyl]-benzamide, le 4-[2-(3,4-dichloro-phénoxy)-phénylsulfamoyl]-*N*-[3-(4-pyridin-4-yl-pipérazin-1-yl)-propyl]-benzamide, le 4-[2-(3,4-dichloro-phénoxy)-phénylsulfamoyl]-*N*-{2-[4-(6-méthyl-pyridin-2-yl)-pipérazin-1-yl]-éthyl}-benzamide, le 4-[2-(2,4-dichloro-phénoxy)-phénylsulfamoyl]-*N-*(3-pipéridin-1-yl-propyl)-benzamide, le *N*-[2-(3,4-dichloro-phénoxy)-phényl]-4-[4-(3-pipéridin-1-yl-propyl)-pipérazine-1- carbonyl]-benzènesulfonamide, le 4-[2-(4-bromo-phénoxy)-phénylsulfamoyl]-*N*-[2-(4-pyridin-2-ylméthyl-pipérazin-1-yl)-éthyl]-benzamide, le chlorhydrate de 4-[2-(4-bromo-phénoxy)-phénylsulfamoyl]-*N*-(4-pipéridin-4-yl-butyl)-benzamide, le *N*-(3-[1,4']bipipéridinyl-1'-yl-propyl)-4-[2-(4-bromo-phénoxy)-5-fluoro-phénylsulfamoyl]-benzamide, le 4-[2-(4-bromo-phénoxy)-5-fluoro-phénylsulfamoyl]-*N*-[4-(2-pyrrolidin-1-yl-éthyl)-cyclohexyl]-benzamide, le 4-[2-(4-bromo-phénoxy)-5-fluoro-phénylsulfamoyl]-*N*-[3-(4-pyridin-4-yl-pipérzin-1-yl)-propyl]-benzamide, le chlorhydrate de 4-[2-(4-bromo-phénoxy)-5-fluoro-phénylsulfamoyl]-*N*-(4-pipéridin-4-yl-butyl)-benzamide, le *N*-(3-[1,4']bipipéridinyl-1'-yl-propyl)-4-[2-(4-bromo-phénoxy)-phénylsulfamoyl]-benzamide, le 4-[2-(4-bromo-phénoxy)-phénylsulfamoyl]-*N*-[3-(4-pyridin-4-yl-pipérazin-1-yl)-propyl]-benzamide, le *N*-(3-[1,4']bipipéridinyl-1'-yl-propyl)-4-[2-(2-chloro-4-fluoro-phénoxy)-phénylsulfamoyl]-benzamide, le 4-[2-(2-chloro-4-fluoro-phénoxy)-phénylsulfamoyl)-*N*-[4-(2-pyrrolidin-1-yl-éthyl)-cyclohexyl]-benzamide, le *N*-(3-[1,4']bipipéridinyl-1'-yl-propyl)-4-[2-(4-trifluorométhyl-phénoxy)-phénylsulfamoyl]-benzamide, le *N*-(3-[1,4']bipipéridinyl-1'-yl-propyl)-4-[2-(4-chloro-2-méthoxy-phénoxy)-phénylsulfamoyl]-benzamide, le chlorhydrate de 4-[2-(2-chloro-4-fluoro-phénoxy)-phénylsulfamoyl]-*N*-(4-pipéridin-4-yl-butyl)-benzamide, le *N*-(4-[1,4']bipipéridinyl-1'-yl-phényl)-4-[2-(4-trifluorométhyl-phénoxy)-phénylsulfamoyl]-benzamide, le chlorhydrate de 4-[2-(2,4-dichloro-phénoxy)-phénylsulfamoyl]-*N*-(3-pipéridin-4-yl-propyl)-benzamide, le *N*-(3-[1,4']bipipéridinyl-1'-yl-propyl)-4-[2-(2-chloro-4-méthoxy-phénoxy)-phénylsulfamoyl]-benzamide, le *N*-(3-[1,4']bipipéridinyl-1'-yl-propyl)-4-(2-phénoxy-phénylsulfamoyl)-benzamide, le *N*-(3-[1,4']bipipéridinyl-1'-yl-propyl)-4-[2-(4-bromo-2-chloro-phénoxy)-phénylsulfamoyl]-benzamide, le 4-[2-(4-bromo-2-chloro-phénoxy)-phénylsulfamoyl]-*N*-[2-(2,3,5,6-tetrahydro-[1,2']bipyridinyl-4-yl)-éthyl]-benzamide, le chlorhydrate de 4-[2-(4-bromo-2-chloro-phénoxy)-phénylsulfamoyl]-*N*-(4-pipéridin-4-ylméthyl-benzyl)benzamide, le chlorhydrate de 4-[2-(4-bromo-2-chloro-phénoxy)-phénylsulfamoyl]-*N*-(4-piperidin-4-yl-butyl)-benzamide, le *N*-(4-[1,4']bipipéridinyl-1'-yl-phényl)-4-[2-(4-bromo-2-chloro-phénoxy)-phénylsulfamoyl]-benzamide, le chlorhydrate de 4-[2-(2,4-dichloro-phénoxy)-phénylsulfamoyl]-*N*-(4-pipéridin-4-yl-butyl)-benzamide ou le *N*-(2-benzoyl-phényl)-4-[4-(3-pipéridin-1-yl-propyl)-pipérazin-1-carbonyl]-benzènesulfonamide.

3. Procédé de préparation des composés de formule (I) tels que revendiqués dans la revendication 1 qui comprend la réaction d'un dérivé amine de formule (II) 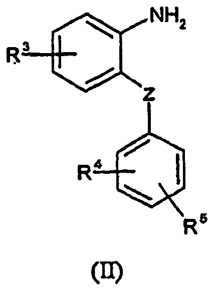
- dans laquelle la signification de R³, R⁴ et R⁵ est comme décrit ci-dessus pour la formule (I) - avec le chlorure de sulfonyle de formule (III) 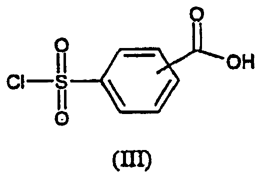 puis la réaction du dérivé d'acide phénylsulfamoyl benzoïque ainsi obtenu de formule (IV) 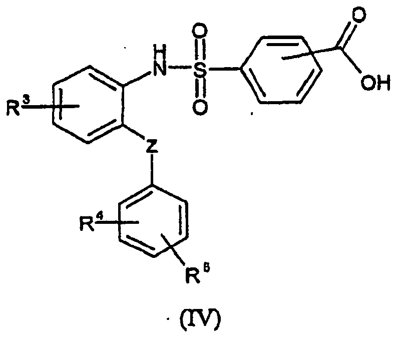
- dans laquelle la signification de R³, R⁴ et R⁵ est comme décrit ci-dessus pour la formule (I) - avec un acide aminé de formule (V) 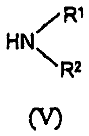
- dans laquelle la signification de R¹ et R² est comme décrit ci-dessus - pour obtenir un dérivé de phénylsulfamoyl benzamide de formule (I) ou ses antipodes ou racémates optiques et/ou sels et/ou hydrates et/ou solvates pharmaceutiquement acceptables.

4. Procédé de préparation des composés de formule (I) selon la revendication 1 qui comprend la transformation d'un composé de formule (I) en un autre composé de formule (I) en introduisant de nouveaux substituants et/ou en modifiant ou éliminant ceux existants, et/ou par formation de sel et/ou par libération du composé des sels.

5. Composé de formule (V) choisi dans le groupe constitué du dichlorhydrate de N-(4-aminométhyl-benzyl)-guanidine et de la 4-pipéridin-4-ylméthyl-benzamidine.

6. Composition pharmaceutique comprenant une quantité thérapeutiquement efficace d'un composé de formule (I) selon la revendication 1 ou de ses antipodes ou racémates optiques ou l'un de ses sels ou hydrates ou solvates pharmaceutiquement acceptables et un ou plusieurs excipients pharmaceutiquement acceptables.

7. Utilisation d'un composé de formule (I) selon la revendication 1 ou de ses antipodes ou racémates optiques ou l'un de ses sels ou hydrates ou solvates pharmaceutiquement acceptables pour la fabrication d'un médicament destiné à la prévention et/ou au traitement d'une affection qui requiert l'inhibition d'un récepteur de bradykinine.

8. Utilisation selon la revendication 7, dans laquelle le récepteur de bradykinine est le récepteur de bradykinine B1.

9. Composé de formule (I) selon la revendication 1 ou ses antipodes ou racémates optiques ou l'un de ses sels ou hydrates ou solvates pharmaceutiquement acceptables pour l'utilisation dans un procédé de traitement et/ou de prévention d'une affection qui requiert l'inhibition d'un récepteur de bradykinine.

10. Composé pour l'utilisation selon la revendication 9 dans lequel le récepteur de bradykinine est un récepteur de bradykinine B1.

11. Dérivés de sulfonamide antagonistes de récepteur de bradykinine B1 de formule (I) pour l'utilisation en tant que médicament 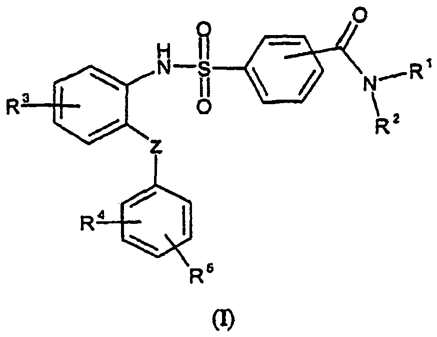 dans laquelle
R¹ est un atome d'hydrogène ou un groupe alkyle en C₁ à C₄ ;
R² est choisi parmi (1) un atome d'hydrogène, à condition que R¹ et R² ne puissent pas être simultanément un atome d'hydrogène ; (2) -(CH₂)ₙ-NR^{a}R^{b}, (3) -(CH₂)ₘ-X-Q, ou
R¹ et R² conjointement avec l'atome de carbone auquel ils sont attachés forment un cycle hétérocyclique à 4 à 7 chaînons contenant 0 à 3 hétéroatomes (en plus de l'atome d'azote auquel R¹ et R² sont attachés) choisis parmi O, S et N ; où ledit cycle est facultativement substitué par un groupe alkyle en C₁ à C₄, -(CH₂)_{q}-Y-P, oxo, 4-(4,5-dihydro-1*H*-imidazol-2-yl)-benzyle, 4-(1,4,5,6-tétrahydro-pyrimidin-2-yl)-benzyle ou 4-ylméthyl-benzamidine ;
R³, R⁴ et R⁵ sont indépendamment les uns des autres un atome d'hydrogène, un atome d'halogène, un groupe cyano, amino ou amino substitué par un ou plusieurs groupes alkyle en C₁ à C₄, un groupe alcoxy en C₁ à C₄, trifluorométhyle, trifluorométhoxy, alkyle en C₁ à C₄, hydroxy, alcoxycarbonyle en C₁ à C₄ ou -C(=O)-NH₂ ;
Z est choisi parmi (1) une simple liaison ; (2) un atome d'oxygène ; (3) un groupe CH₂ ; (4) un groupe CO ; (5) un groupe NR^{c} ; (6) un atome S ; (7) un groupe SO₂ ;
n est un nombre entier de 1 à 4 ;
m vaut 0 ou est un nombre entier de 2 à 6 ;
q est un nombre entier de 0 à 4 ;
R^{a} et R^{b} sont (1) un atome d'hydrogène, (2) un groupe alkyle en C₁ à C₆ linéaire ou ramifié ;
R^{c} est un atome d'hydrogène ou un groupe alkyle en C₁ à C₄ ;
X est une simple liaison ou un groupe -CO- ou -CO-NH- ou NH-CO- ;
Y est une simple liaison ou un groupe -CO- ou -CONR^{a} ;
P est (1) un groupe -N(alkyle en C₁ à C₄)₂ ; (2) un groupe -NH-(CH₂)ₙ-Het ; (3) un cycle à 4 à 7 chaînons saturé, partiellement insaturé ou aromatique contenant 1 à 3 hétéroatomes choisis parmi O, S et N ; où ledit cycle est facultativement substitué par un groupe oxo ou un groupe alkyle en C₁ à C₄ ;
Q est (1) un cycle à 4 à 7 chaînons saturé, partiellement insaturé ou aromatique contenant 1 à 3 hétéroatomes choisis parmi O, S et N ; où ledit cycle est facultativement substitué par un groupe oxo, -(CH₂)ₙ-Het, 1-pipéridinyle, 1-(alkyle en C₁ à C₄)-4-pipéridinyle, 4-pipéridinyle, 2-pyrimidinyle, 2-pyrazinyle, 2-pyridyle, 4-méthyl-2-pyridyle, 6-méthyl-2-pyridyle ou 4-pyridyle ; (2) un groupe phényle, facultativement substitué par un groupe -(CH₂)ₙ-Het, -(CH₂)ₙ-NH-C(=NH)-NH₂, 4,5-dihydro-1*H-*imidazol-2-yle ou [1,4']bipipéridinyl-1'-yle ; (3) un groupe cycloalkyle en C₅ à C₇, facultativement substitué par un groupe -(CH₂)ₙ-Het ; (4) un groupe benzyle, facultativement substitué par un groupe -(CH₂)ₙ-Het, -(CH₂)ₙ-NH-C(=NH)-NH₂ ou 4,5-dihydro-1*H*-imidazol-2-yle ; (5) un groupe -(CH₂)ₙ-Het ;
Het est un cycle hétérocyclique à 4 à 7 chaînons contenant 1 à 3 hétéroatomes choisis parmi O, S, SO₂ et N, facultativement substitué par (1) un groupe oxo ; (2) un ou plusieurs groupes alkyle en C₁ à C₄ ;
et leurs antipodes ou racémates optiques et/ou sels et/ou hydrates et/ou solvates.
